# EUROPEAN PATENT APPLICATION

(11) **EP 2 402 338 A1**
(43) Date of publication of application: **04.01.2012**
(21) Application number: 11171608.0
(22) Date of filing: 28.06.2011
(51) Int. Cl.: C07D 471/04, C07D 487/04, A01N 43/90, A01P 3/00

(54) **Pyrazolopyridine compounds**

(30) Priority: 29.06.2010 EP 10167632
(71) Applicant: BASF SE, 67056 Ludwigshafen (DE)
(72) Inventor: Boudet, Nadege, 69502 Hemsbach (DE); Dietz, Jochen, 76227 Karlsruhe (DE); Glättli, Alice, 60435 Frankfurt (DE); Grammenos, Wassilios, 67071 Ludwigshafen (DE); Müller, Bernd, 67227 Frankenthal (DE); Lohmann, Jan Klaas, 67245 Lambsheim (DE); Pilger, Christian, 67063 Ludwigshafen (DE); Riggs, Richard, 68165 Mannheim (DE); Vrettou-Schultes, Marianna, 68165 Mannheim (DE)

(57) **Abstract**

The use of pyrazolopyridine compounds of the formula I 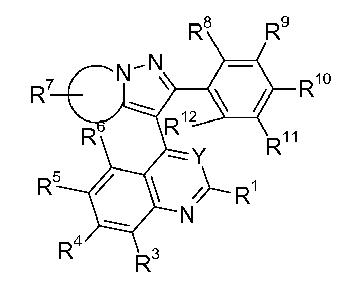 in which the variables have the meanings as defined in the description and the claims, for controlling phytopathogenic fungi.

## Description

The present invention relates to the use of pyrazolopyridine compounds of the formula I

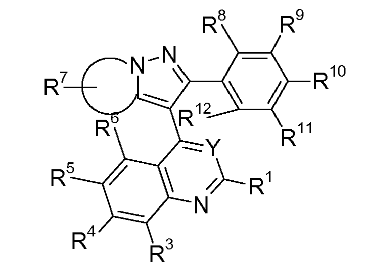

Furthermore the present invention relates to novel pyrazolopyridine compounds.

Furthermore the present invention relates to an agrochemical composition, comprising at least one compound of the formula I and/or a salt thereof and at least one solid or liquid carrier.

Furthermore the present invention relates to a composition furthermore comprising at least one further fungicidally, insecticidally and/or herbicidally active compound.

Furthermore the present invention relates to seed, comprising at least one compound of the formula I and/or an agriculturally acceptable salt thereof in an amount of from 1 to 1000 g per 100 kg.

Furthermore the present invention relates to a method for controlling phytopathogenic harmful fungi, comprising treating the fungi, their habitat or the seed, the soil or the plants to be protected against fungal attack with an effective amount of the compound of the formula I and/or an agriculturally acceptable salt thereof.

Pyrazolopyridine compounds are described, for example, in WO 2002/094833. These compounds are said to be suitable for the treatment of herpes infections. An application in crop protection is not mentioned.

Accordingly, it was an object of the present invention to find a new use of pyrazolopyridine compounds. Moreover, it was an object of the present invention to provide novel pyrazolopyridine compounds.

Surprisingly, it was found that pyrazolopyridine compounds of the general formula I, as defined below, and the agriculturally acceptable salts of the compounds of the formula I have fungicidal activity.

Accordingly, the present invention relates to the use of pyrazolopyridine compounds of the formula I 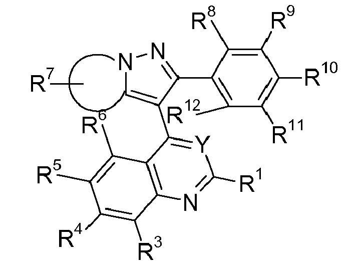 in which 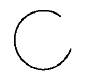 is C₅-C₆-cycloalkyl;
- Y: is N or C-X,
- R¹ to R¹², X: independently of one another are
H, halogen, cyano, nitro, N₃; or
C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalkyl, C₃-C₁₀-cycloalkenyl; or
phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or
NA¹A² where
A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl, wherein
the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or
independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or OA³ where
A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or a radical of the formula -C(=O)A⁴, -OC(=O)A⁴, -NA⁴C(=O)A⁴, -N=OA⁴ where
A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or a radical of the formula -S(O)ₙA⁵, - OS(O)ₙA⁵, -NA⁵S(O)ₙA⁵ where
n is 0, 1, 2
A⁵ is C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy;
and/or of an agriculturally acceptable salt thereof for controlling phytopathogenic fungi.

Some of the compounds of the formula I described above are novel. Accordingly, the present invention also provides novel pyrimidine compounds of the formula I according to the definition above, with the proviso, that if 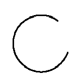 is C₅-cycloalkyl;
- R⁸ to R¹²: can not be simultaneously H; and
- R⁸: is not F, methoxy;
- R⁹: is not Cl, F, C₁-haloalkyl, OA³ where A³ is CH₃; C₁-haloalkoxy;
- R¹⁰: is not Cl, F, C₁-haloalkyl;
- R¹¹: is not Cl, F, C₁-haloalkyl, OA³ where A³ is CH₃; C₁-haloalkoxy; and
- R¹²: is not F, methoxy.

Further, the present invention also provides novel pyrimidine compounds of the formula I according to the definition above, with the proviso, that if 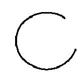 is C₆-cycloalkyl;
- R⁸ to R¹²: can not be simultaneously H; and
- R⁸: is not F;
- R⁹: is not Cl, F, C₁-haloalkyl, C₁-haloalkoxy;
- R¹⁰: is not Cl, F, C₁-haloalkyl;
- R¹¹: is not Cl, F, C₁-haloalkyl, C₁-haloalkoxy; and
- R¹²: is not F.

Further the present invention also provides fungicidal compositions comprising these compounds and/or their agriculturally acceptable salts and suitable carriers.

Further the present invention also provides fungicidal compositions comprising at least one further fungicidally, insecticidally and/or herbicidally active compound.

Further the present invention also provides seed comprising these compounds and/or their agriculturally acceptable salts.

Further the present invention also provides a method for controlling phytopathogenic fungi which use these compounds and/or their agriculturally acceptable salts.

The terms used for organic groups in the definition of the variables are, for example the expression "halogen", collective terms which represent the individual members of these groups of organic units.

The prefi⁶ **Cₓ-C_{y}** denotes the number of possible carbon atoms in the particular case.
**halogen:** fluorine, bromine, chlorine or iodine, especially fluorine, chlorine or bromine;
**alkyl and the alkyl moieties of composite groups such as, for example, alkoxy, alkylamino, alkoxycarbonyl:** saturated straight-chain or branched hydrocarbon radicals having 1 to 10 carbon atoms, for example C₁-C₁₀-akyl, such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl; heptyl, octyl, 2-ethylhexyl and positional isomers thereof; nonyl, decyl and positional isomers thereof;
**haloalkyl:** straight-chain or branched alkyl groups having 1 to 10 carbon atoms (as mentioned above), where some or all of the hydrogen atoms in these groups are replaced by halogen atoms as mentioned above. In one embodiment, the alkyl groups are substituted at least once or completely by a particular halogen atom, preferably fluorine, chlorine or bromine. In a further embodiment, the alkyl groups are partially or fully halogenated by different halogen atoms; in the case of mixed halogen substitutions, the combination of chlorine and fluorine is preferred. Particular preference is given to (C₁-C₃)-haloalkyl, more preferably (C₁-C₂)-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl or 1,1,1-trifluoroprop-2-yl;
**alkenyl and also the alkenyl moieties in composite groups, such as alkenyloxy:** unsaturated straight-chain or branched hydrocarbon radicals having 2 to 10 carbon atoms and one double bond in any position. According to the invention, it may be preferred to use small alkenyl groups, such as (C₂-C₄)-alkenyl; on the other hand, it may also be preferred to employ larger alkenyl groups, such as (C₅-C₈)-alkenyl. Examples of alkenyl groups are, for example, C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
**alkynyl and the alkynyl moieties in composite groups**: straight-chain or branched hydrocarbon groups having 2 to 10 carbon atoms and one or two triple bonds in any position, for example C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
**cycloalkyl and also the cycloalkyl moieties in composite groups**: mono- or bicyclic saturated hydrocarbon groups having 3 to 10, in particular 3 to 6, carbon ring members, for example C₃-C₆-cycloalkyl, such as cyclopropyl, cyclobutyl, cyclopentyl, cyclohexyl, cycloheptyl or cyclooctyl. Examples of bicyclic radicals comprise bicyclo[2.2.1]heptyl, bicyclo[3.1.1]heptyl, bicyclo[2.2.2]octyl and bicyclo[3.2.1]octyl. In this connection, optionally substituted C₃-C₈-cycloalkyl means a cycloalkyl radical having from 3 to 8 carbon atoms, in which at least one hydrogen atom, for example 1, 2, 3, 4 or 5 hydrogen atoms, is/are replaced by substituents which are inert under the conditions of the reaction. Examples of inert substituents are CN, C₁-C₆-alkyl, C₁-C₄-haloalkyl, C₁-C₆-alkoxy, C₃-C₆-cycloalkyl, and C₁-C₄-alkoxy-C₁-C₆-alkyl;
**halocycloalkyl and the halocycloalkyl moieties in halocycloalkoxy, halocycloalkylcarbonyl and the like**: monocyclic saturated hydrocarbon groups having 3 to 10 carbon ring members (as mentioned above) in which some or all of the hydrogen atoms may be replaced by halogen atoms as mentioned above, in particular fluorine, chlorine and bromine;
**cycloalkenyl**: monocyclic monounsaturated hydrocarbon groups having 3 to 10, 3 to 8, 3 to 6, preferably 5 to 6, carbon ring members, such as cyclopenten-1-yl, cyclopenten-3-yl, cyclohexen-1-yl, cyclohexen-3-yl, cyclohexen-4-yl and the like;
**alkoxy:** an alkyl group as defined above which is attached via an oxygen, preferably having 1 to 10, more preferably 2 to 6, carbon atoms. Examples are: methoxy, ethoxy, n-propoxy, 1-methylethoxy, butoxy, 1-methylpropoxy, 2-methylpropoxy or 1,1-dimethylethoxy, and also for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy;
**haloalkoxy:** alkoxy as defined above, where some or all of the hydrogen atoms in these groups are replaced by halogen atoms as described above under haloalkyl, in particular by fluorine, chlorine or bromine. Examples are OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy; and also 5-fluoropentoxy, 5-chloropentoxy, 5-bromopentoxy, 5-iodopentoxy, undecafluoropentoxy, 6-fluorohexoxy, 6-chlorohexoxy, 6-bromohexoxy, 6-iodohexoxy or dodecafluorohexoxy;
**6- to 10-membered aryl:** aromatic cyclus with 6, 7, 8, 9 oder 10 C atoms. Examples of preferred aryl are phenyl or naphthyl;
**5-, 6-, 7-, 8- , 9- or 10-membered saturated, partially unsaturated or aromatic heterocycle**
which contains 1, 2, 3 or 4 heteroatoms from the group consisting of O, N and S as ring members, and may furthermore contain one or two CO, SO, SO₂ groups as ring members, where the heterocycle in question may be attached via a carbon atom or, if present, via a nitrogen atom. In particular:
- a five- or six-membered saturated or partially unsaturated heterocycle which comprises one, two, three or four heteroatoms from the group consisting of O, N and S as ring members: for example monocyclic saturated or partially unsaturated heterocycles which, in addition to carbon ring members, comprise one, two or three nitrogen atoms and/or one oxygen or sulfur atom or one or two oxygen and/or sulfur atoms, for example 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals;
- a seven-membered saturated or partially unsaturated heterocycle which comprises one, two, three or four heteroatoms from the group consisting of O, N and S as ring members: for example mono- and bicyclic heterocycles having 7 ring members which, in addition to carbon ring members, comprise one, two or three nitrogen atoms and/or one oxygen or sulfur atom or one or two oxygen and/or sulfur atoms, for example tetra- and hexahydroazepinyl, such as 2,3,4,5-tetrahydro[1H]azepin-1-, -2-, -3-, -4-, -5-, -6- or -7-yl, 3,4,5,6-tetrahydro[2H]azepin-2-, -3-, -4-, -5-, -6- or -7-yl, 2,3,4,7-tetrahydro[1H]azepin-1-, -2-, -3-, -4-, -5-, -6- or -7-yl, 2,3,6,7-tetrahydro[1H]azepin-1-, -2-, -3-, -4-, -5-, -6- or -7-yl, hexahydroazepin-1-, -2-, -3- or -4-yl, tetra- and hexahydrooxepinyl such as 2,3,4,5-tetrahydro[1H]oxepin-2-, -3-, -4-, -5-, -6- or -7-yl, 2,3,4,7-tetrahydro[1H]oxepin-2-, -3-, -4-, -5-, -6- or -7-yl, 2,3,6,7-tetrahydro[1H]oxepin-2-, -3-, -4-, -5-, -6- or -7-yl, hexahydroazepin-1-, -2-, -3- or -4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding ylidene radicals;
- a five- or six-membered aromatic heterocycle (= heteroaromatic radical) which contains one, two, three or four heteroatoms from the group consisting of oxygen, nitrogen and sulfur, for example 5-membered heteroaryl which is attached via carbon and contains one to three nitrogen atoms or one or two nitrogen atoms and one sulfur or oxygen atom as ring members, such as 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 5-membered heteroaryl which is attached via nitrogen and contains one to three nitrogen atoms as ring members, such as pyrrol-1-yl, pyrazol-1-yl, imidazol-1-yl, 1,2,3-triazol-1-yl and 1,2,4-triazol-1-yl; 6-membered heteroaryl, which contains one, two or three nitrogen atoms as ring members, such as pyridin-2-yl, pyridin-3-yl, pyridin-4-yl, 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl;
**C₁-C₁₀-alkoxy-C₁-C₁₀-alkyl**: C₁-C₁₀-alkyl (as defined above) where one hydrogen atom is replaced by a C₁-C₁₀-alkoxy group as defined above;
**amino-C₁-C₁₀-alkyl**: C₁-C₁₀-alkyl (as defined above) where one hydrogen atom is replaced by a NA¹A² group as defined above;
**mono-(C₁-C₁₀-alkyl)amino**: group of the formula NA¹A² group in which A¹ or A² is an C₁-C₁₀-alkyl group as defined above.
**di-(C₁-C₁₀-alkyl)amino**: group of the formula NA¹A² group in which each A¹ and A² are an C₁-C₁₀-alkyl group as defined above.
**hydroxyl:** OH group which is attached via an O atom;
**cyano:** CN group which is attached via an C atom;
**nitro:** NO₂ group which is attached via an N atom.

Depending on the substitution pattern, the compounds of the formula I used according to the invention and the compounds according to the invention may have one or more centers of chirality, and are generally obtained in the form of racemates or as diastereomer mixtures of erythro and threo forms. The erythro and threo diastereomers of the compounds according to the invention can be separated and isolated in pure form, for example, on the basis of their different solubilities or by column chromatography. Using known methods, such uniform pairs of diastereomers can be used to obtain uniform enantiomers. Suitable for use as antimicrobial agents are both the uniform diastereomers or enantiomers and mixtures thereof obtained in the synthesis. This applies correspondingly to the fungicidal compositions. Accordingly, the invention provides both the pure enantiomers or diastereomers and mixtures thereof. This applies to the compounds of the formula I used according to the invention and the compounds according to the invention and, if appropriate, correspondingly to their precursors. The scope of the present invention includes in particular the (R) and (S) isomers and the racemates of the compounds according to the invention, in particular of the formula I, which have centers of chirality. Suitable compounds of the formula I used according to the invention and compounds according to the invention also comprise all possible stereoisomers (cis/trans isomers) and mixtures thereof.

The compounds of the formula I used according to the invention and the compounds according to the invention may be present in various crystal modifications which may differ in their biological activity. They are likewise provided by the present invention.

Owing to the basic character of their nitrogen atoms, the compounds of the formula I used according to the invention and the compounds according to the invention are capable of forming salts or adducts with inorganic or organic acids or with metal ions.

Suitable agriculturally useful salts are especially the salts of those cations or the acid addition salts of those acids whose cations and anions, respectively, have no adverse effect on the fungicidal action of the compounds of the formula I. Thus, suitable cations are in particular the ions of the alkali metals, preferably sodium and potassium, of the alkaline earth metals, preferably calcium, magnesium and barium, and of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium and sulfoxonium ions, preferably tri(C₁-C₄-alkyl)sulfoxonium.

Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and also the anions of C₁-C₄-alkanoic acids, preferably formate, acetate, propionate and butyrate. They can be formed by reacting with an acid of the corresponding anion, preferably hydrochloric acid, hydrobromic acid, sulfuric acid, phosphoric acid or nitric acid.

In the compounds of the formula I used according to the invention and the compounds according to the invention particular preference is given to the following meanings of the substituents, in each case on their own or in combination. 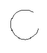 C in the compounds according to the invention or the compounds used according to the invention is, according to one embodiment, C₅-cycloalkyl. 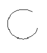 in the compounds according to the invention or the compounds used according to the invention is, according to one further embodiment, C₆-cycloalkyl.

R¹ in the compounds according to the invention or the compounds used according to the invention is, according to one embodiment, H, halogen, cyano, nitro or N₃. In a special embodiment of the invention, R¹ is H.

R¹ in the compounds according to the invention or the compounds used according to the invention is, according to a further embodiment, C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalky or C₃-C₁₀-cycloalkenyl.

According to one embodiment R¹ is C₁-C₁₀-alkyl, preferrerably methyl, ethyl, n-propyl, i-propyl, n-butyl, i-butyl, t-butyl, in particular methyl, ethyl. According to a further embodiment R¹ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment R¹ is C₂-C₁₀-alkenyl, preferably ethylene. According to a further embodiment R¹ is C₂-C₁₀-alkynyl, preferred ethynyl, 1-propynyl. According to a further embodiment R¹ is C₃-C₁₀-cycloalkyl, preferrably cyclopropyl, cyclobutyl, cyclopentyl, in particular cyclopropyl or cyclobutyl. According to a further embodiment R¹ is C₃-C₁₀-halocycloalkyl, preferrably fully or partially halogenated cyclopropyl, cyclobutyl, cyclopentyl, in particular fully or partially halogenated cyclopropyl. According to a further embodiment R¹ is C₃-C₁₀-cycloalkenyl, preferably cyclopropenyl.

R¹ in the compounds according to the invention or the compounds used according to the invention is, according to a further embodiment, phenyl, benzyl, naphthyl, a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment R¹ is phenyl. According to a further embodiment R¹ is benzyl. According to a further embodiment R¹ is naphthyl. According to one embodiment R¹ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃. In a special embodiment of the invention, R¹ is phenyl. In a further special embodiment of the invention, R¹ is phenyl substituted by Cl, F, Br, I, CH₃, OCH₃, CF₃ or OCF₃. In a further special embodiment of the invention, R¹ is benzyl substituted by Cl, F, Br, I, CH₃, OCH₃, CF₃ or OCF₃. In a further special embodiment of the invention, R¹ is naphthyl substituted by Cl, F, Br, I, CH₃, OCH₃, CF₃ or OCF₃.

R¹ in the compounds according to the invention or the compounds used according to the invention is, according to a further embodiment, NA¹A² where A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A¹ and A² independently of one another are hydrogen. According to a further embodiment A¹ and A² independently of one another are hydrogen or C₁-C₁₀-alkyl, preferably methyl, ethyl, n-propyl, i-propyl, n-butyl, i-butyl, t-butyl. In a special embodiment of the invention, R¹ is NH₂, NHCH₃, N(CH₃)₂, NHC₂H₅, NHn-C₃H₇, NHi-C₃H₇, NHn-C₄H₉, NHi-C₄H₉, NHt-C₄H₉. According to a further embodiment A¹ and A² independently of one another are hydrogen or C₃-C₁₀-cycloalkyl, preferably cyclopropyl, cyclobutyl, cyclopenyl, cyclohexyl. In a special embodiment of the invention, R¹ is cyclopropylamino, cyclobutylamino, cyclopentylamino, cyclohexylamino. According to a further embodiment A¹ and A² independently of one another are C₁-C₁₀-alkoxy-C₁-C₁₀-alkyl, preferably C₁-C₄-alkoxy-C₁-C₄-alkyl. In a special embodiment of the invention, R¹ is methoxyethylamino, methoxypropylamino, methoxybutylamino, ethoxyethylamino, ethoxypropylamino or ethoxybutylamino.

According to a further embodiment A¹ and A² independently of one another are hydrogen or amino-C₁-C₁₀-alkyl, wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl. In one embodiment B¹ and B² independently of one another are hydrogen. In a special embodiment of the invention, R¹ is amineethylamino, aminepropylamino, aminebutylamino. In a further embodiment B¹ and B² independently of one another are hydrogen, C₁-C₁₀-alkyl, preferrably methyl, ethyl, propyl, butyl. In a special embodiment of the invention, R¹ is methylamineethylamino, methylaminepropylamino, methylaminebutylamino, ethylamineethylamino, ethylaminepropylamino, ethylaminebutylamino, dimethylamineethylamino, dimethylaminepropylamino, dimethylaminebutylamino. In a further embodiment B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members. In a special embodiment of the invention, R¹ is morpholin-4-ylethylamino, morpholin-4-ylpropylamino, morpholin-4-ylbutylamino, piperazine-4-ylethylamino, piperazine-4-ylpropylamino piperazine-4-ylbutylamino.

According to one embodiment A¹ and A² independently of one another are hydrogen or phenyl. According to one embodiment A¹ and A² independently of one another are hydrogen or benzyl. According to a further embodiment A¹ and A² independently of one another are hydrogen or naphthyl. According to a further embodiment A¹ and A² independently of one another are hydrogen or a saturated partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO, SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃. In a special embodiment of the invention, A¹ and/or A² are phenyl. In a further special embodiment of the invention, A¹ and/or A² are phenyl substituted by Cl, F, Br, I, CH₃, OCH₃, CF₃ or OCF₃. In a further special embodiment of the invention, A¹ and/or A² are benzyl substituted by Cl, F, Br, I, CH₃, OCH₃, CF₃ or OCF₃. In a further special embodiment of the invention, A¹ and/or A² are naphthyl substituted by Cl, F, Br, I, CH₃, OCH₃, CF₃ or OCF₃.

R¹ in the compounds according to the invention or the compounds used according to the invention is, according to a further embodiment, OA³, where A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment A³ is H or C₁-C₁₀-alkyl, preferably methyl, ethyl, n-propyl, i-propyl, n-butyl, i-butyl, t-butyl. In a special embodiment of the invention, R¹ is OCH₃, OC₂H₅, On-C₃H₇, Oi-C₃H₇, On-C₄H₉, Oi-C₄H₉, Ot-C₄H₉. According to a further embodiment A³ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A³ is C₂-C₁₀-alkenyl, preferably ethylene. According to a further embodiment A³ is C₂-C₁₀-alkynyl, preferred ethynyl, 1-propynyl. According to a further embodiment A³ is C₃-C₁₀-cycloalkyl, preferrably cyclopropyl, cyclobutyl, cyclopentyl, in particular cyclopropyl or cyclobutyl. According to a further embodiment A³ is C₃-C₁₀-halocycloalkyl, preferrably fully or partially halogenated cyclopropyl, cyclobutyl, cyclopentyl, in particular fully or partially halogenated cyclopropyl. According to a further embodiment A³ is C₃-C₁₀-cycloalkenyl, preferably cyclopropenyl.

According to one embodiment A³ is phenyl. According to a further embodiment A³ is benzyl. According to a further embodiment A³ is naphthyl. According to one embodiment A³ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃. In a special embodiment of the invention, R¹ is OPh, wherein phenyl can be substituted by Cl, F, Br, I, CH₃, OCH₃, CF₃ or OCF₃.

R¹ in the compounds according to the invention or the compounds used according to the invention is, according to a further embodiment, a radical of the formula -C(=O)A⁴, -OC(=O)A⁴, -NA⁴C(=O)A⁴, -N=OA⁴ where A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁴ is H or C₁-C₁₀-alkyl, preferably methyl, ethyl, n-propyl, i-propyl, n-butyl, i-butyl, t-butyl. According to a further embodiment A⁴ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁴ is C₂-C₁₀-alkenyl, preferably ethylene. According to a further embodiment A⁴ is C₂-C₁₀-alkynyl, preferred ethynyl, 1-propynyl. According to a further embodiment A⁴ is C₃-C₁₀-cycloalkyl, preferrably cyclopropyl, cyclobutyl, cyclopentyl, in particular cyclopropyl or cyclobutyl. According to a further embodiment A⁴ is C₃-C₁₀-halocycloalkyl, preferrably fully or partially halogenated cyclopropyl, cyclobutyl, cyclopentyl, in particular fully or partially halogenated cyclopropyl. According to a further embodiment A⁴ is C₃-C₁₀-cycloalkenyl, preferably cyclopropenyl. According to a further embodiment A⁴ is NH₂. According to a further embodiment A⁴ is mono-(C₁₀-C₁₀-alkyl)amino. According to a further embodiment A⁴ is di-(C₁-C₁₀-alkyl)amino.

According to one embodiment A⁴ is phenyl. According to a further embodiment A⁴ is benzyl. According to a further embodiment A⁴ is naphthyl. According to one embodiment A⁴ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R¹ in the compounds according to the invention or the compounds used according to the invention is, according to a further embodiment, a radical of the formula -S(O)ₙA⁵, -OS(O)ₙA^{S}, -NA⁵S(O)ₙA⁵ where n = 0, 1, 2 and A⁵ independently of one another are hydrogen, hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated or unsaturated aromatic or non-aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁵ is H or C₁-C₁₀-alkyl, preferably methyl, ethyl, n-propyl, i-propyl, n-butyl, i-butyl, t-butyl. According to a further embodiment A⁵ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁵ is C₂-C₁₀-alkenyl, preferably ethylene. According to a further embodiment A⁵ is C₂-C₁₀-alkynyl, preferred ethynyl, 1-propynyl. According to a further embodiment A⁵ is C₃-C₁₀-cycloalkyl, preferrably cyclopropyl, cyclobutyl, cyclopentyl, in particular cyclopropyl or cyclobutyl. According to a further embodiment A⁵ is C₃-C₁₀-halocycloalkyl, preferrably fully or partially halogenated cyclopropyl, cyclobutyl, cyclopentyl, in particular fully or partially halogenated cyclopropyl. According to a further embodiment A⁵ is C₃-C₁₀-cycloalkenyl, preferably cyclopropenyl. According to a further embodiment A⁵ is NH₂. According to a further embodiment A⁵ is mono-(C₁-C₁₀-alkyl)amino. According to a further embodiment A⁵ is di-(C₁-C₁₀-alkyl)amino.

According to one embodiment A⁵ is phenyl. According to a further embodiment A⁵ is benzyl. According to a further embodiment A⁵ is naphthyl. According to one embodiment A⁵ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

X in the compounds according to the invention or the compounds used according to the invention is as defined for R¹. In a special embodiment of the invention, X is H.

R³ to R⁶ in the compounds according to the invention or the compounds used according to the invention are independently of another, according to one embodiment, H, halogen, cyano, nitro or N₃. In a special embodiment of the invention, R³ to R⁶ are independently of another H.

R³ to R⁶ in the compounds according to the invention or the compounds used according to the invention are independently of another, according to a further embodiment, C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalky or C₃-C₁₀-cycloalkenyl.

According to one embodiment R³ to R⁶ independently of another are C₁-C₁₀-alkyl, preferrerably methyl, ethyl, n-propyl, i-propyl, n-butyl, i-butyl, t-butyl, in particular methyl, ethyl. According to a further embodiment R³ to R⁶ independently of another are C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment R³ to R⁶ independently of another are C₂-C₁₀-alkenyl, preferably ethylene. According to a further embodiment R³ to R⁶ independently of another are C₂-C₁₀-alkynyl, preferred ethynyl, 1-propynyl. According to a further embodiment R³ to R⁶ independently of another are C₃-C₁₀-cycloalkyl, preferrably cyclopropyl, cyclobutyl, cyclopentyl, in particular cyclopropyl or cyclobutyl. According to a further embodiment R³ to R⁶ independently of another are C₃-C₁₀-halocycloalkyl, preferrably fully or partially halogenated cyclopropyl, cyclobutyl, cyclopentyl, in particular fully or partially halogenated cyclopropyl. According to a further embodiment R³ to R⁶ independently of another are C₃-C₁₀-cycloalkenyl, preferably cyclopropenyl.

R³ to R⁶ in the compounds according to the invention or the compounds used according to the invention are independently of another, according to a further embodiment, phenyl, benzyl, naphthyl, a saturated, partially unsaturated or aromatic 5, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment R³ to R⁶ independently of another are phenyl. According to a further embodiment R³ to R⁶ independently of another are benzyl. According to a further embodiment R³ to R⁶ independently of another are naphthyl. According to one embodiment R³ to R⁶ independently of another are a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R³ to R⁶ in the compounds according to the invention or the compounds used according to the invention are independently of another, according to a further embodiment, NA¹A² where A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy. According to one embodiment A¹ and A² independently of one another are hydrogen. According to a further embodiment A¹ and A² independently of one another are hydrogen or C₁-C₁₀-alkyl, preferably methyl, ethyl, n-propyl, i-propyl, n-butyl, i-butyl, t-butyl. According to a further embodiment A¹ and A² independently of one another are hydrogen or C₃-C₁₀-cycloalkyl, preferably cyclopropyl, cyclobutyl, cyclopenyl, cyclohexyl. According to a further embodiment A¹ and A² independently of one another are C₁-C₁₀-alkoxy-C₁-C₁₀-alkyl, preferably C₁-C₄-alkoxy-C₁-C₄-alkyl. According to a further embodiment A¹ and A² independently of one another are hydrogen or amino-Ci-Cio-alkyl, wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl. In one embodiment B¹ and B² independently of one another are hydrogen. In a further embodiment B¹ and B² independently of one another are hydrogen, C₁-C₁₀-alkyl, preferrably methyl, ethyl, propyl, butyl In a further embodiment B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members

According to one embodiment A¹ and A² independently of one another are hydrogen or phenyl. According to one embodiment A¹ and A² independently of one another are hydrogen or benzyl. According to a further embodiment A¹ and A² independently of one another are naphthyl. According to a further embodiment A¹ and A² independently of one another are a saturated partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO, SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R³ to R⁶ in the compounds according to the invention or the compounds used according to the invention are independently of another, according to a further embodiment, OA³, where A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment A³ is H or C₁-C₁₀-alkyl, preferably methyl, ethyl, n-propyl, i-propyl, n-butyl, i-butyl, t-butyl. According to a further embodiment A³ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A³ is C₂-C₁₀-alkenyl, preferably ethylene. According to a further embodiment A³ is C₂-C₁₀-alkynyl, preferred ethynyl, 1-propynyl. According to a further embodiment A³ is C₃-C₁₀-cycloalkyl, preferrably cyclopropyl, cyclobutyl, cyclopentyl, in particular cyclopropyl or cyclobutyl. According to a further embodiment A³ is C₃-C₁₀-halocycloalkyl, preferrably fully or partially halogenated cyclopropyl, cyclobutyl, cyclopentyl, in particular fully or partially halogenated cyclopropyl. According to a further embodiment A³ is C₃-C₁₀-cycloalkenyl, preferably cyclopropenyl.

According to one embodiment A³ is phenyl. According to a further embodiment A³ is benzyl. According to a further embodiment A³ is naphthyl. According to one embodiment A³ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R³ to R⁶ in the compounds according to the invention or the compounds used according to the invention are independently of another, according to a further embodiment, a radical of the formula -C(=O)A⁴, -OC(=O)A⁴, -NA⁴C(=O)A⁴, -N=OA⁴ where A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁴ is H or C₁-C₁₀-alkyl, preferably methyl, ethyl, n-propyl, i-propyl, n-butyl, i-butyl, t-butyl. According to a further embodiment A⁴ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁴ is C₂-C₁₀-alkenyl, preferably ethylene. According to a further embodiment A⁴ is C₂-C₁₀-alkynyl, preferred ethynyl, 1-propynyl. According to a further embodiment A⁴ is C₃-C₁₀-cycloalkyl, preferrably cyclopropyl, cyclobutyl, cyclopentyl, in particular cyclopropyl or cyclobutyl. According to a further embodiment A⁴ is C₃-C₁₀-halocycloalkyl, preferrably fully or partially halogenated cyclopropyl, cyclobutyl, cyclopentyl, in particular fully or partially halogenated cyclopropyl. According to a further embodiment A⁴ is C₃-C₁₀-cycloalkenyl, preferably cyclopropenyl. According to a further embodiment A⁴ is NH₂. According to a further embodiment A⁴ is mono-(C₁-C₁₀-alkyl)amino. According to a further embodiment A⁴ is di-(C₁-C₁₀-alkyl)amino.

According to one embodiment A⁴ is phenyl. According to a further embodiment A⁴ is benzyl. According to a further embodiment A⁴ is naphthyl. According to one embodiment A⁴ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R³ to R⁶ in the compounds according to the invention or the compounds used according to the invention are independently of another, according to a further embodiment, a radical of the formula -S(O)ₙA⁵, - OS(O)ₙA⁵, -NA⁵S(O)ₙA⁵ where n = 0, 1, 2 and A⁵ independently of one another are hydrogen, hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated or unsaturated aromatic or non-aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁵ is H or C₁-C₁₀-alkyl, preferably methyl, ethyl, n-propyl, i-propyl, n-butyl, i-butyl, t-butyl. According to a further embodiment A⁵ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁵ is C₂-C₁₀-alkenyl, preferably ethylene. According to a further embodiment A⁵ is C₂-C₁₀-alkynyl, preferred ethynyl, 1-propynyl. According to a further embodiment A⁵ is C₃-C₁₀-cycloalkyl, preferrably cyclopropyl, cyclobutyl, cyclopentyl, in particular cyclopropyl or cyclobutyl. According to a further embodiment A⁵ is C₃-C₁₀-halocycloalkyl, preferrably fully or partially halogenated cyclopropyl, cyclobutyl, cyclopentyl, in particular fully or partially halogenated cyclopropyl. According to a further embodiment A⁵ is C₃-C₁₀-cycloalkenyl, preferably cyclopropenyl. According to a further embodiment A⁵ is NH_{2.}According to a further embodiment A⁵ is mono-(C₁-C₁₀-alkyl)amino. According to a further embodiment A⁵ is di-(C₁-C₁₀-alkyl)amino.

According to one embodiment A⁵ is phenyl. According to a further embodiment A⁵ is benzyl. According to a further embodiment A⁵ is naphthyl. According to one embodiment A⁵ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁷ in the compounds according to the invention or the compounds used according to the invention is, according to one embodiment, H, halogen, cyano, nitro or N₃. In a special embodiment of the invention, R⁷ is H.

R⁷ in the compounds according to the invention or the compounds used according to the invention is, according to a further embodiment, C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalky or C₃-C₁₀-cycloalkenyl.

According to one embodiment R⁷ is C₁-C₁₀-alkyl, preferrerably methyl, ethyl, n-propyl, i-propyl, n-butyl, i-butyl, t-butyl, in particular methyl, ethyl. According to a further embodiment R⁷ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment R⁷ is C₂-C₁₀-alkenyl, preferably ethylene. According to a further embodiment R⁷ is C₂-C₁₀-alkynyl, preferred ethynyl, 1-propynyl. According to a further embodiment R⁷ is C₃-C₁₀-cycloalkyl, preferrably cyclopropyl, cyclobutyl, cyclopentyl, in particular cyclopropyl or cyclobutyl. According to a further embodiment R⁷ is C₃-C₁₀-halocycloalkyl, preferrably fully or partially halogenated cyclopropyl, cyclobutyl, cyclopentyl, in particular fully or partially halogenated cyclopropyl. According to a further embodiment R⁷ is C₃-C₁₀-cycloalkenyl, preferably cyclopropenyl.

R⁷ in the compounds according to the invention or the compounds used according to the invention is, according to a further embodiment, phenyl, benzyl, naphthyl, a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment R⁷ is phenyl. According to a further embodiment R⁷ is benzyl. According to a further embodiment R⁷ is naphthyl. According to one embodiment R⁷ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃. In a special embodiment of the invention, R⁷ is phenyl. In a further special embodiment of the invention, R⁷ is phenyl substituted by Cl, F, Br, I, CH₃, OCH₃, CF₃ or OCF₃. In a further special embodiment of the invention, R⁷ is benzyl substituted by Cl, F, Br, I, CH₃, OCH₃, CF₃ or OCF₃. In a further special embodiment of the invention, R⁷ is naphthyl substituted by Cl, F, Br, I, CH₃, OCH₃, CF₃ or OCF₃.

R⁷ in the compounds according to the invention or the compounds used according to the invention is, according to a further embodiment, NA¹A² where A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A¹ and A² independently of one another are hydrogen. According to a further embodiment A¹ and A² independently of one another are hydrogen or C₁-C₁₀-alkyl, preferably methyl, ethyl, n-propyl, i-propyl, n-butyl, i-butyl, t-butyl. In a special embodiment of the invention, R⁷ is NH₂, NHCH₃, N(CH₃)₂, NHC₂H₅, NHn-C₃H₇, NHi-C₃H₇, NHn-C₄H₉, NHi-C₄H₉, NHt-C₄H₉. According to a further embodiment A¹ and A² independently of one another are hydrogen or C₃-C₁₀-cycloalkyl, preferably cyclopropyl, cyclobutyl, cyclopenyl, cyclohexyl. In a special embodiment of the invention, R⁷ is cyclopropylamino, cyclobutylamino, cyclopentylamino, cyclohexylamino. According to a further embodiment A¹ and A² independently of one another are C₁-C₁₀-alkoxy-C₁-C₁₀-alkyl, preferably C₁-C₄-alkoxy-C₁-C₄-alkyl. In a special embodiment of the invention, R⁷ is methoxyethylamino, methoxypropylamino, methoxybutylamino, ethoxyethylamino, ethoxypropylamino or ethoxybutylamino.

According to a further embodiment A¹ and A² independently of one another are hydrogen or amino-C₁-C₁₀-alkyl, wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl. In one embodiment B¹ and B² independently of one another are hydrogen. In a special embodiment of the invention, R⁷ is amineethylamino, aminepropylamino, aminebutylamino. In a further embodiment B¹ and B² independently of one another are hydrogen, C₁-C₁₀-alkyl, preferrably methyl, ethyl, propyl, butyl. In a special embodiment of the invention, R⁷ is methylamineethylamino, methylaminepropylamino, methylaminebutylamino, ethylamineethylamino, ethylaminepropylamino, ethylaminebutylamino, dimethylamineethylamino, dimethylaminepropylamino, dimethylaminebutylamino. In a further embodiment B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members. In a special embodiment of the invention, R⁷ is morpholin-4-ylethylamino, morpholin-4-ylpropylamino, morpholin-4-ylbutylamino, piperazine-4-ylethylamino, piperazine-4-ylpropylamino piperazine-4-ylbutylamino.

According to one embodiment A¹ and A² independently of one another are hydrogen or phenyl. According to one embodiment A¹ and A² independently of one another are hydrogen or benzyl. According to a further embodiment A¹ and A² independently of one another are hydrogen or naphthyl. According to a further embodiment A¹ and A² independently of one another are hydrogen or a saturated partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO, SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃. In a special embodiment of the invention, A¹ and/or A² are phenyl. In a further special embodiment of the invention, A¹ and/or A² are phenyl substituted by Cl, F, Br, I, CH₃, OCH₃, CF₃ or OCF₃. In a further special embodiment of the invention, A¹ and/or A² are benzyl substituted by Cl, F, Br, I, CH₃, OCH₃, CF₃ or OCF₃. In a further special embodiment of the invention, A¹ and/or A² are naphthyl substituted by Cl, F, Br, I, CH₃, OCH₃, CF₃ or OCF₃.

R⁷ in the compounds according to the invention or the compounds used according to the invention is, according to a further embodiment, OA³, where A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment A³ is H or C₁-C₁₀-alkyl, preferably methyl, ethyl, n-propyl, i-propyl, n-butyl, i-butyl, t-butyl. In a special embodiment of the invention, R⁷ is OCH₃, OC₂H₅, On-C₃H₇, Oi-C₃H₇, On-C₄H₉, Oi-C₄H₉, Ot-C₄H₉. According to a further embodiment A³ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A³ is C₂-C₁₀-alkenyl, preferably ethylene. According to a further embodiment A³ is C₂-C₁₀-alkynyl, preferred ethynyl, 1-propynyl. According to a further embodiment A³ is C₃-C₁₀-cycloalkyl, preferrably cyclopropyl, cyclobutyl, cyclopentyl, in particular cyclopropyl or cyclobutyl. According to a further embodiment A³ is C₃-C₁₀-halocycloalkyl, preferrably fully or partially halogenated cyclopropyl, cyclobutyl, cyclopentyl, in particular fully or partially halogenated cyclopropyl. According to a further embodiment A³ is C₃-C₁₀-cycloalkenyl, preferably cyclopropenyl.

According to one embodiment A³ is phenyl. According to a further embodiment A³ is benzyl. According to a further embodiment A³ is naphthyl. According to one embodiment A³ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃. In a special embodiment of the invention, R⁷ is OPh, wherein phenyl can be substituted by Cl, F, Br, I, CH₃, OCH₃, CF₃ or OCF₃.

R⁷ in the compounds according to the invention or the compounds used according to the invention is, according to a further embodiment, a radical of the formula -C(=O)A⁴, -OC(=O)A⁴, -NA⁴C(=O)A⁴, -N=OA⁴ where A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁴ is H or C₁-C₁₀-alkyl, preferably methyl, ethyl, n-propyl, i-propyl, n-butyl, i-butyl, t-butyl. According to a further embodiment A⁴ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁴ is C₂-C₁₀-alkenyl, preferably ethylene. According to a further embodiment A⁴ is C₂-C₁₀-alkynyl, preferred ethynyl, 1-propynyl. According to a further embodiment A⁴ is C₃-C₁₀-cycloalkyl, preferrably cyclopropyl, cyclobutyl, cyclopentyl, in particular cyclopropyl or cyclobutyl. According to a further embodiment A⁴ is C₃-C₁₀-halocycloalkyl, preferrably fully or partially halogenated cyclopropyl, cyclobutyl, cyclopentyl, in particular fully or partially halogenated cyclopropyl. According to a further embodiment A⁴ is C₃-C₁₀-cycloalkenyl, preferably cyclopropenyl. According to a further embodiment A⁴ is NH₂. According to a further embodiment A⁴ is mono-(C₁-C₁₀-alkyl)amino. According to a further embodiment A⁴ is di-(C₁-C₁₀-alkyl)amino.

According to one embodiment A⁴ is phenyl. According to a further embodiment A⁴ is benzyl. According to a further embodiment A⁴ is naphthyl. According to one embodiment A⁴ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁷ in the compounds according to the invention or the compounds used according to the invention is, according to a further embodiment, a radical of the formula -S(O)ₙA⁵, -OS(O)ₙA⁵, -NA⁵S(O)ₙA⁵ where n = 0, 1, 2 and A⁵ independently of one another are hydrogen, hydrogen, C₁-C₁₀-alkyl, C₁-C₁₀-haloalkyl, C₃-C₁₀-cycloalkyl, C₃-C₁₀-halocycloalkyl, NH_{2,} mono-(C₁-C₁₀-alkyl)amino, di-(C₁-C₁₀-alkyl)amino, phenyl, benzyl, naphthyl or a saturated or unsaturated aromatic or non-aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁵ is H or C₁-C₁₀-alkyl, preferably methyl, ethyl, n-propyl, i-propyl, n-butyl, i-butyl, t-butyl. According to a further embodiment A⁵ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁵ is C₂-C₁₀-alkenyl, preferably ethylene. According to a further embodiment A⁵ is C₂-C₁₀-alkynyl, preferred ethynyl, 1-propynyl. According to a further embodiment A⁵ is C₃-C₁₀-cycloalkyl, preferrably cyclopropyl, cyclobutyl, cyclopentyl, in particular cyclopropyl or cyclobutyl. According to a further embodiment A⁵ is C₃-C₁₀-halocycloalkyl, preferrably fully or partially halogenated cyclopropyl, cyclobutyl, cyclopentyl, in particular fully or partially halogenated cyclopropyl. According to a further embodiment A⁵ is C₃-C₁₀-cycloalkenyl, preferably cyclopropenyl. According to a further embodiment A⁵ is NH₂. According to a further embodiment A⁵ is mono-(C₁-C₁₀-alkyl)amino. According to a further embodiment A⁵ is di-(C₁-C₁₀-alkyl)amino.

According to one embodiment A⁵ is phenyl. According to a further embodiment A⁵ is benzyl. According to a further embodiment A⁵ is naphthyl. According to one embodiment A⁵ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁸ and R¹² in the compounds used according to the invention are, according to one embodiment, H, halogen, cyano, nitro or N₃. In a special embodiment of the invention, R⁸ and R¹² are independently of another H. In a further special embodiment of the invention, R⁸ and R¹² are independently of another F or Cl.

R⁸ and R¹² in the compounds used according to the invention are independently of another, according to a further embodiment, C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalky or C₃-C₁₀-cycloalkenyl.

According to one embodiment R⁸ and R¹² independently of another are C₁-C₁₀-alkyl, preferrerably methyl, ethyl, n-propyl, i-propyl, n-butyl, i-butyl, t-butyl, in particular methyl, ethyl. According to a further embodiment R⁸ and R¹² independently of another are C₁-C₁₀-haloalkyl; preferrably fully or partially halogenated methyl, ethyl, n-propyl, i-propyl, n-butyl, i-butyl, t-butyl, in particular fully or partially halogenated methyl. In a special embodiment of the invention, R⁸ and R¹² are independently of another CF₃. According to a further embodiment R⁸ and R¹² independently of another are C₂-C₁₀-alkenyl, preferably ethylene. According to a further embodiment R⁸ and R¹² independently of another are C₂-C₁₀-alkynyl, preferred ethynyl, 1-propynyl. According to a further embodiment R⁸ and R¹² independently of another are C₃-C₁₀-cycloalkyl, preferrably cyclopropyl, cyclobutyl, cyclopentyl, in particular cyclopropyl or cyclobutyl. According to a further embodiment R⁸ and R¹² independently of another are C₃-C₁₀-halocycloalkyl, preferrably fully or partially halogenated cyclopropyl, cyclobutyl, cyclopentyl, in particular fully or partially halogenated cyclopropyl. According to a further embodiment R⁸ and R¹² independently of another are C₃-C₁₀-cycloalkenyl, preferably cyclopropenyl.

R⁸ and R¹² in the compounds used according to the invention are independently of another, according to a further embodiment, phenyl, benzyl, naphthyl, a saturated, partially unsaturated or aromatic 5, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment R⁸ and R¹² independently of another are phenyl. According to a further embodiment R⁸ and R¹² independently of another are benzyl. According to a further embodiment R⁸ and R¹² independently of another are naphthyl. According to one embodiment R⁸ and R¹² independently of another are a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁸ and R¹² in the compounds used according to the invention are independently of another, according to a further embodiment, NA¹A² where A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A¹ and A² independently of one another are hydrogen. According to a further embodiment A¹ and A² independently of one another are hydrogen or C₁-C₁₀-alkyl, preferably methyl, ethyl, n-propyl, i-propyl, n-butyl, i-butyl, t-butyl. According to a further embodiment A¹ and A² independently of one another are hydrogen or C₃-C₁₀-cycloalkyl, preferably cyclopropyl, cyclobutyl, cyclopenyl, cyclohexyl. According to a further embodiment A¹ and A² independently of one another are C₁-C₁₀-alkoxy-C₁-C₁₀-alkyl, preferably C₁-C₄-alkoxy-C₁-C₄-alkyl. According to a further embodiment A¹ and A² independently of one another are hydrogen or amino-C₁-C₁₀-alkyl, wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl. In one embodiment B¹ and B² independently of one another are hydrogen. In a further embodiment B¹ and B² independently of one another are hydrogen, C₁-C₁₀-alkyl, preferrably methyl, ethyl, propyl, butyl In a further embodiment B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members

According to one embodiment A¹ and A² independently of one another are hydrogen or phenyl. According to one embodiment A¹ and A² independently of one another are hydrogen or benzyl. According to a further embodiment A¹ and A² independently of one another are naphthyl. According to a further embodiment A¹ and A² independently of one another are a saturated partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO, SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁸ and R¹² in the compounds used according to the invention are independently of another, according to a further embodiment, OA³, where A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment A³ is H or C₁-C₁₀-alkyl, preferably methyl, ethyl, n-propyl, i-propyl, n-butyl, i-butyl, t-butyl. According to a further embodiment A³ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A³ is C₂-C₁₀-alkenyl, preferably ethylene. According to a further embodiment A³ is C₂-C₁₀-alkynyl, preferred ethynyl, 1-propynyl. According to a further embodiment A³ is C₃-C₁₀-cycloalkyl, preferrably cyclopropyl, cyclobutyl, cyclopentyl, in particular cyclopropyl or cyclobutyl. According to a further embodiment A³ is C₃-C₁₀-halocycloalkyl, preferrably fully or partially halogenated cyclopropyl, cyclobutyl, cyclopentyl, in particular fully or partially halogenated cyclopropyl. According to a further embodiment A³ is C₃-C₁₀-cycloalkenyl, preferably cyclopropenyl.

According to one embodiment A³ is phenyl. According to a further embodiment A³ is benzyl. According to a further embodiment A³ is naphthyl. According to one embodiment A³ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁸ and R¹² in the compounds used according to the invention are independently of another, according to a further embodiment, a radical of the formula -C(=O)A⁴, -OC(=O)A⁴, -NA⁴C(=O)A⁴, -N=OA⁴ where A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁴ is H or C₁-C₁₀-alkyl, preferably methyl, ethyl, n-propyl, i-propyl, n-butyl, i-butyl, t-butyl. According to a further embodiment A⁴ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁴ is C₂-C₁₀-alkenyl, preferably ethylene. According to a further embodiment A⁴ is C₂-C₁₀-alkynyl, preferred ethynyl, 1-propynyl. According to a further embodiment A⁴ is C₃-C₁₀-cycloalkyl, preferrably cyclopropyl, cyclobutyl, cyclopentyl, in particular cyclopropyl or cyclobutyl. According to a further embodiment A⁴ is C₃-C₁₀-halocycloalkyl, preferrably fully or partially halogenated cyclopropyl, cyclobutyl, cyclopentyl, in particular fully or partially halogenated cyclopropyl. According to a further embodiment A⁴ is C₃-C₁₀-cycloalkenyl, preferably cyclopropenyl. According to a further embodiment A⁴ is NH_{2.} According to a further embodiment A⁴ is mono-(C₁-C₁₀-alkyl)amino. According to a further embodiment A⁴ is di-(C₁-C₁₀-alkyl)amino.

According to one embodiment A⁴ is phenyl. According to a further embodiment A⁴ is benzyl. According to a further embodiment A⁴ is naphthyl. According to one embodiment A⁴ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁸ and R¹² in the compounds used according to the invention are independently of another, according to a further embodiment, a radical of the formula -S(O)ₙA⁵, -OS(O)ₙA⁵, -NA⁵S(O)ₙA⁵ where n = 0, 1, 2 and A⁵ independently of one another are hydrogen, hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated or unsaturated aromatic or non-aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁵ is H or C₁-C₁₀-alkyl, preferably methyl, ethyl, n-propyl, i-propyl, n-butyl, i-butyl, t-butyl. According to a further embodiment A⁵ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁵ is C₂-C₁₀-alkenyl, preferably ethylene. According to a further embodiment A⁵ is C₂-C₁₀-alkynyl, preferred ethynyl, 1-propynyl. According to a further embodiment A⁵ is C₃-C₁₀-cycloalkyl, preferrably cyclopropyl, cyclobutyl, cyclopentyl, in particular cyclopropyl or cyclobutyl. According to a further embodiment A⁵ is C₃-C₁₀-halocycloalkyl, preferrably fully or partially halogenated cyclopropyl, cyclobutyl, cyclopentyl, in particular fully or partially halogenated cyclopropyl. According to a further embodiment A⁵ is C₃-C₁₀-cycloalkenyl, preferably cyclopropenyl. According to a further embodiment A⁵ is NH₂. According to a further embodiment A⁵ is mono-(C₁-C₁₀-alkyl)amino. According to a further embodiment A⁵ is di-(C₁-C₁₀-alkyl)amino.

According to one embodiment A⁵ is phenyl. According to a further embodiment A⁵ is benzyl. According to a further embodiment A⁵ is naphthyl. According to one embodiment A⁵ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁸ and R¹² in the compounds according to the invention are as defined above with the proviso that if 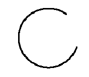 is C₅-cycloalkyl;
- R⁸ to R¹²: can not be simultaneously H; and
- R⁸: is not F, methoxy; and
- R¹²: is not F, methoxy.

In one embodiment of the invention R⁸ and R¹² are not halogen. In a further embodiment of the invention R⁸ and R¹² are not C₁-C₄-alkyl. In a further embodiment of the invention R⁸ and R¹² are not C₁-C₁₀-alkyl. In a further embodiment of the invention R⁸ and R¹² are not C₁-C₄-haloalkyl. In a further embodiment of the invention R⁸ and R¹² are not C₁-C₁₀-haloalkyl. In a further embodiment of the invention R⁸ and R¹² are not NA¹A², where A¹ and A² are indepemdly of another C₁-C₄-alkyl. In a further embodiment of the invention R⁸ and R¹² are not OA³ where A³ is C₁-C₄-alkyl. In a further embodiment of the invention R⁸ and R¹² are not OA³ where A³ is C₃-C₄-cycloalkyl. In a further embodiment of the invention R⁸ and R¹² are not 5- or 6-membered aromatic heterocycle.

R⁸ and R¹² in the compounds according to the invention are as defined above with the proviso that if 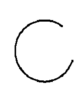 is C₆-cycloalkyl;
- R⁸ to R¹²: can not be simultaneously H; and
- R⁸: is not F; and
- R¹²: is not F.

In one embodiment of the invention R⁸ and R¹² are not halogen. In a further embodiment of the invention R⁸ and R¹² are not C₁-C₄-alkyl. In a further embodiment of the invention R⁸ and R¹² are not C₁-C₁₀-alkyl. In a further embodiment of the invention R⁸ and R¹² are not C₁-C₄-haloalkyl. In a further embodiment of the invention R⁸ and R¹² are not C₁-C₁₀-haloalkyl. In a further embodiment of the invention R⁸ and R¹² are not NA¹A², where A¹ and A² are indepemdly of another C₁-C₄-alkyl. In a further embodiment of the invention R⁸ and R¹² are not OA³ where A³ is C₁-C₄-alkyl. In a further embodiment of the invention R⁸ and R¹² are not OA³ where A³ is C₃-C₄-cycloalkyl. In a further embodiment of the invention R⁸ and R¹² are not 5- or 6-membered aromatic heterocycle.

R⁹ and R¹¹ in the compounds used according to the invention are, according to one embodiment, H, halogen, cyano, nitro or N₃. In a special embodiment of the invention, R⁹ and R¹¹ are independently of another F or Cl.

R⁹ and R¹¹ in the compounds used according to the invention are independently of another, according to a further embodiment, C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalky or C₃-C₁₀-cycloalkenyl.

According to one embodiment R⁹ and R¹¹ independently of another are C₁-C₁₀-alkyl, preferrerably methyl, ethyl, n-propyl, i-propyl, n-butyl, i-butyl, t-butyl, in particular methyl, ethyl. In a special embodiment of the invention, R⁹ and R¹¹ are independently of another CH₃. According to a further embodiment R⁹ and R¹¹ independently of another are C₁-C₁₀-haloalkyl; preferrably fully or partially halogenated methyl, ethyl, n-propyl, i-propyl, n-butyl, i-butyl, t-butyl, in particular fully or partially halogenated methyl. In a special embodiment of the invention, R⁹ and R¹¹ are independently of another CF₃. According to a further embodiment R⁹ and R¹¹ independently of another are C₂-C₁₀-alkenyl, preferably ethylene. According to a further embodiment R⁹ and R¹¹ independently of another are C₂-C₁₀-alkynyl, preferred ethynyl, 1-propynyl. According to a further embodiment R⁹ and R¹¹ independently of another are C₃-C₁₀-cycloalkyl, preferrably cyclopropyl, cyclobutyl, cyclopentyl, in particular cyclopropyl or cyclobutyl. According to a further embodiment R⁹ and R¹¹ independently of another are C₃-C₁₀-halocycloalkyl, preferrably fully or partially halogenated cyclopropyl, cyclobutyl, cyclopentyl, in particular fully or partially halogenated cyclopropyl. According to a further embodiment R⁹ and R¹¹ independently of another are C₃-C₁₀-cycloalkenyl, preferably cyclopropenyl.

R⁹ and R¹¹ in the compounds used according to the invention are independently of another, according to a further embodiment, phenyl, benzyl, naphthyl, a saturated, partially unsaturated or aromatic 5, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members. According to one embodiment R⁹ and R¹¹ independently of another are phenyl. According to a further embodiment R⁹ and R¹¹ independently of another are benzyl. According to a further embodiment R⁹ and R¹¹ independently of another are naphthyl. According to one embodiment R⁹ and R¹¹ independently of another are a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁹ and R¹¹ in the compounds used according to the invention are independently of another, according to a further embodiment, NA¹A² where A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A¹ and A² independently of one another are hydrogen. According to a further embodiment A¹ and A² independently of one another are hydrogen or

C₁-C₁₀-alkyl, preferably methyl, ethyl, n-propyl, i-propyl, n-butyl, i-butyl, t-butyl. According to a further embodiment A¹ and A² independently of one another are hydrogen or C₃-C₁₀-cycloalkyl, preferably cyclopropyl, cyclobutyl, cyclopenyl, cyclohexyl. According to a further embodiment A¹ and A² independently of one another are C₁-C₁₀-alkoxy-C₁-C₁₀-alkyl, preferably C₁-C₄-alkoxy-C₁-C₄-alkyl. According to a further embodiment A¹ and A² independently of one another are hydrogen or amino-C₁-C₁₀-alkyl, wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl. In one embodiment B¹ and B² independently of one another are hydrogen. In a further embodiment B¹ and B² independently of one another are hydrogen, C₁-C₁₀-alkyl, preferrably methyl, ethyl, propyl, butyl In a further embodiment B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members

According to one embodiment A¹ and A² independently of one another are hydrogen or phenyl. According to one embodiment A¹ and A² independently of one another are hydrogen or benzyl. According to a further embodiment A¹ and A² independently of one another are naphthyl. According to a further embodiment A¹ and A² independently of one another are a saturated partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO, SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁹ and R¹¹ in the compounds used according to the invention are independently of another, according to a further embodiment, OA³, where A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment A³ is H or C₁-C₁₀-alkyl, preferably methyl, ethyl, n-propyl, i-propyl, n-butyl, i-butyl, t-butyl. In a special embodiment of the invention, R⁹ and R¹¹ are independently of another OCH₃, OC₂H₅. According to a further embodiment A³ is C₁-C₁₀-haloalkyl; preferrably fully or partially halogenated methyl, ethyl, n-propyl, i-propyl, n-butyl, i-butyl, t-butyl, in particular fully or partially halogenated methyl. In a special embodiment of the invention, R⁹ and R¹¹ are independently of another OCF₃. According to a further embodiment A³ is C₂-C₁₀-alkenyl, preferably ethylene. According to a further embodiment A³ is C₂-C₁₀-alkynyl, preferred ethynyl, 1-propynyl. According to a further embodiment A³ is C₃-C₁₀-cycloalkyl, preferrably cyclopropyl, cyclobutyl, cyclopentyl, in particular cyclopropyl or cyclobutyl. According to a further embodiment A³ is C₃-C₁₀-halocycloalkyl, preferrably fully or partially halogenated cyclopropyl, cyclobutyl, cyclopentyl, in particular fully or partially halogenated cyclopropyl. According to a further embodiment A³ is C₃-C₁₀-cycloalkenyl, preferably cyclopropenyl.

According to one embodiment A³ is phenyl. According to a further embodiment A³ is benzyl. According to a further embodiment A³ is naphthyl. According to one embodiment A³ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁹ and R¹¹ in the compounds used according to the invention are independently of another, according to a further embodiment, a radical of the formula -C(=O)A⁴, -OC(=O)A⁴, - NA⁴C(=O)A⁴, -N=OA⁴ where A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁴ is H or C₁-C₁₀-alkyl, preferably methyl, ethyl, n-propyl, i-propyl, n-butyl, i-butyl, t-butyl. According to a further embodiment A⁴ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁴ is C₂-C₁₀-alkenyl, preferably ethylene. According to a further embodiment A⁴ is C₂-C₁₀-alkynyl, preferred ethynyl, 1-propynyl. According to a further embodiment A⁴ is C₃-C₁₀-cycloalkyl, preferrably cyclopropyl, cyclobutyl, cyclopentyl, in particular cyclopropyl or cyclobutyl. According to a further embodiment A⁴ is C₃-C₁₀-halocycloalkyl, preferrably fully or partially halogenated cyclopropyl, cyclobutyl, cyclopentyl, in particular fully or partially halogenated cyclopropyl. According to a further embodiment A⁴ is C₃-C₁₀-cycloalkenyl, preferably cyclopropenyl. According to a further embodiment A⁴ is NH₂. According to a further embodiment A⁴ is mono-(C₁-C₁₀-alkyl)amino. According to a further embodiment A⁴ is di-(C₁-C₁₀-alkyl)amino.

According to one embodiment A⁴ is phenyl. According to a further embodiment A⁴ is benzyl. According to a further embodiment A⁴ is naphthyl. According to one embodiment A⁴ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁹ and R¹¹ in the compounds used according to the invention are independently of another, according to a further embodiment, a radical of the formula -S(O)ₙA⁵, -OS(O)ₙA⁵, -NA⁵S(O)ₙA⁵ where n = 0, 1, 2 and A⁵ independently of one another are hydrogen, hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated or unsaturated aromatic or non-aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁵ is H or C₁-C₁₀-alkyl, preferably methyl, ethyl, n-propyl, i-propyl, n-butyl, i-butyl, t-butyl. According to a further embodiment A⁵ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁵ is C₂-C₁₀-alkenyl, preferably ethylene. According to a further embodiment A⁵ is C₂-C₁₀-alkynyl, preferred ethynyl, 1-propynyl. According to a further embodiment A⁵ is C₃-C₁₀-cycloalkyl, preferrably cyclopropyl, cyclobutyl, cyclopentyl, in particular cyclopropyl or cyclobutyl. According to a further embodiment A⁵ is C₃-C₁₀-halocycloalkyl, preferrably fully or partially halogenated cyclopropyl, cyclobutyl, cyclopentyl, in particular fully or partially halogenated cyclopropyl. According to a further embodiment A⁵ is C₃-C₁₀-cycloalkenyl, preferably cyclopropenyl. According to a further embodiment A⁵ is NH₂. According to a further embodiment A⁵ is mono-(C₁-C₁₀-alkyl)amino. According to a further embodiment A⁵ is di-(C₁-C₁₀-alkyl)amino.

According to one embodiment A⁵ is phenyl. According to a further embodiment A⁵ is benzyl. According to a further embodiment A⁵ is naphthyl. According to one embodiment A⁵ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R⁹ and R¹¹ in the compounds according to the invention are as defined above with the proviso that iif 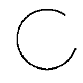 is C₅-cycloalkyl;
- R⁹: is not Cl, F, C₁-haloalkyl, OA³ where A³ is CH₃; C₁-haloalkoxy; and
- R¹¹: is not Cl, F, C₁-haloalkyl, OA³ where A³ is CH₃; C₁-haloalkoxy.

In one embodiment of the invention R⁹ and R¹¹ are not halogen. In a further embodiment of the invention R⁹ and R¹¹ are not C₁-C₄-alkyl. In a further embodiment of the invention R⁹ and R¹¹ are not C₁-C₁₀-alkyl. In a further embodiment of the invention R⁹ and R¹¹ are not C₁-C₄-haloalkyl. In a further embodiment of the invention R⁹ and R¹¹ are not C₁-C₁₀-haloalkyl. In a further embodiment of the invention R⁹ and R¹¹ are not NA¹A², where A¹ and A² are indepemdly of another C₁-C₄-alkyl. In a further embodiment of the invention R⁹ and R¹¹ are not OA³ where A³ is C₁-C₄-alkyl. In a further embodiment of the invention R⁹ and R¹¹ are not OA³ where A³ is C₃-C₄-cycloalkyl. In a further embodiment of the invention R⁹ and R¹¹ are not OA³ where A³ is C₁-C₄-haloalkyl. In a further embodiment of the invention R⁹ and R¹¹ are not 5- or 6-membered aromatic heterocycle.

R⁹ and R¹¹ in the compounds according to the invention are as defined above with the proviso that if 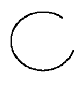 is C₆-cycloalkyl;
- R⁹: is not Cl, F, C₁-haloalkyl, C₁-haloalkoxy; and
- R¹¹: is not Cl, F, C₁-haloalkyl, C₁-haloalkoxy.

In one embodiment of the invention R⁹ and R¹¹ are not halogen. In a further embodiment of the invention R⁹ and R¹¹ are not C₁-C₄-alkyl. In a further embodiment of the invention R⁹ and R¹¹ are not C₁-C₁₀-alkyl. In a further embodiment of the invention R⁹ and R¹¹ are not C₁-C₄-haloalkyl. In a further embodiment of the invention R⁹ and R¹¹ are not C₁-C₁₀-haloalkyl. In a further embodiment of the invention R⁹ and R¹¹ are not NA¹A², where A¹ and A² are indepemdly of another C₁-C₄-alkyl. In a further embodiment of the invention R⁹ and R¹¹are not OA³ where A³ is C₁-C₄-alkyl. In a further embodiment of the invention R⁹ and R¹¹ are not OA³ where A³ is C₃-C₄-cycloalkyl. In a further embodiment of the invention R⁹ and R¹¹ are not OA³ where A³ is C₁-C₄-haloalkyl. In a further embodiment of the invention R⁹ and R¹¹ are not 5- or 6-membered aromatic heterocycle.

R¹⁰ in the compounds used according to the invention is, according to one embodiment, H, halogen, cyano, nitro or N₃. In a special embodiment of the invention, R¹⁰ is F or Cl.

R¹⁰ in the compounds used according to the invention is, according to a further embodiment, C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalky or C₃-C₁₀-cycloalkenyl.

According to one embodiment R¹⁰ is C₁-C₁₀-alkyl, preferrerably methyl, ethyl, n-propyl, i-propyl, n-butyl, i-butyl, t-butyl, in particular methyl, ethyl. In a special embodiment of the invention, R¹⁰ is CH₃. According to a further embodiment R¹⁰ is C₁-C₁₀-haloalkyl; preferrably fully or partially halogenated methyl, ethyl, n-propyl, i-propyl, n-butyl, i-butyl, t-butyl, in particular fully or partially halogenated methyl. In a special embodiment of the invention, R¹⁰ is CF₃. According to a further embodiment R¹⁰ is C₂-C₁₀-alkenyl, preferably ethylene. According to a further embodiment R¹⁰ is C₂-C₁₀-alkynyl, preferred ethynyl, 1-propynyl. According to a further embodiment R¹⁰ is C₃-C₁₀-cycloalkyl, preferrably cyclopropyl, cyclobutyl, cyclopentyl, in particular cyclopropyl or cyclobutyl. According to a further embodiment R¹⁰ is C₃-C₁₀-halocycloalkyl, preferrably fully or partially halogenated cyclopropyl, cyclobutyl, cyclopentyl, in particular fully or partially halogenated cyclopropyl. According to a further embodiment R¹⁰ is C₃-C₁₀-cycloalkenyl, preferably cyclopropenyl.

R¹⁰ in the compounds used according to the invention are independently of another, according to a further embodiment, phenyl, benzyl, naphthyl, a saturated, partially unsaturated or aromatic 5, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment R¹⁰ is phenyl. According to a further embodiment R¹⁰ is benzyl. According to a further embodiment R¹⁰ is naphthyl. According to one embodiment R¹⁰ is saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R¹⁰ is in the compounds used according to the invention is, according to a further embodiment, NA¹A² where A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A¹ and A² independently of one another are hydrogen. According to a further embodiment A¹ and A² independently of one another are hydrogen or C₁-C₁₀-alkyl, preferably methyl, ethyl, n-propyl, i-propyl, n-butyl, i-butyl, t-butyl. According to a further embodiment A¹ and A² independently of one another are hydrogen or C₃-C₁₀-cycloalkyl, preferably cyclopropyl, cyclobutyl, cyclopenyl, cyclohexyl. According to a further embodiment A¹ and A² independently of one another are C₁-C₁₀-alkoxy-C₁-C₁₀-alkyl, preferably C₁-C₄-alkoxy-C₁-C₄-alkyl. According to a further embodiment A¹ and A² independently of one another are hydrogen or amino-C₁-C₁₀-alkyl, wherein the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl. In one embodiment B¹ and B² independently of one another are hydrogen. In a further embodiment B¹ and B² independently of one another are hydrogen, C₁-C₁₀-alkyl, preferrably methyl, ethyl, propyl, butyl In a further embodiment B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members

According to one embodiment A¹ and A² independently of one another are hydrogen or phenyl. According to one embodiment A¹ and A² independently of one another are hydrogen or benzyl. According to a further embodiment A¹ and A² independently of one another are naphthyl. According to a further embodiment A¹ and A² independently of one another are a saturated partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO, SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably CI, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R¹⁰ in the compounds used according to the invention is, according to a further embodiment, OA³, where A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment A³ is H or C₁-C₁₀-alkyl, preferably methyl, ethyl, n-propyl, i-propyl, n-butyl, i-butyl, t-butyl. In a special embodiment of the invention, R¹⁰ is OCH₃, OC₂H₅. According to a further embodiment A³ is C₁-C₁₀-haloalkyl; preferrably fully or partially halogenated methyl, ethyl, n-propyl, i-propyl, n-butyl, i-butyl, t-butyl, in particular fully or partially halogenated methyl. In a special embodiment of the invention, R¹⁰ is OCF₃. According to a further embodiment A³ is C₂-C₁₀-alkenyl, preferably ethylene. According to a further embodiment A³ is C₂-C₁₀-alkynyl, preferred ethynyl, 1-propynyl. According to a further embodiment A³ is C₃-C₁₀-cycloalkyl, preferrably cyclopropyl, cyclobutyl, cyclopentyl, in particular cyclopropyl or cyclobutyl. According to a further embodiment A³ is C₃-C₁₀-halocycloalkyl, preferrably fully or partially halogenated cyclopropyl, cyclobutyl, cyclopentyl, in particular fully or partially halogenated cyclopropyl. According to a further embodiment A³ is C₃-C₁₀-cycloalkenyl, preferably cyclopropenyl.

According to one embodiment A³ is phenyl. According to a further embodiment A³ is benzyl. According to a further embodiment A³ is naphthyl. According to one embodiment A³ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R¹⁰ in the compounds used according to the invention is, according to a further embodiment, a radical of the formula -C(=O)A⁴, -OC(=O)A⁴, -NA⁴C(=O)A⁴, -N=OA⁴ where A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁴ is H or C₁-C₁₀-alkyl, preferably methyl, ethyl, n-propyl, i-propyl, n-butyl, i-butyl, t-butyl. According to a further embodiment A⁴ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁴ is C₂-C₁₀-alkenyl, preferably ethylene. According to a further embodiment A⁴ is C₂-C₁₀-alkynyl, preferred ethynyl, 1-propynyl. According to a further embodiment A⁴ is C₃-C₁₀-cycloalkyl, preferrably cyclopropyl, cyclobutyl, cyclopentyl, in particular cyclopropyl or cyclobutyl. According to a further embodiment A⁴ is C₃-C₁₀-halocycloalkyl, preferrably fully or partially halogenated cyclopropyl, cyclobutyl, cyclopentyl, in particular fully or partially halogenated cyclopropyl. According to a further embodiment A⁴ is C₃-C₁₀-cycloalkenyl, preferably cyclopropenyl. According to a further embodiment A⁴ is NH₂. According to a further embodiment A⁴ is mono-(C₁-C₁₀-alkyl)amino. According to a further embodiment A⁴ is di-(C₁-C₁₀-alkyl)amino.

According to one embodiment A⁴ is phenyl. According to a further embodiment A⁴ is benzyl. According to a further embodiment A⁴ is naphthyl. According to one embodiment A⁴ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R¹⁰ in the compounds used according to the invention is, according to a further embodiment, a radical of the formula -S(O)ₙA⁵, - OS(O)ₙA⁵, -NA⁵S(O)ₙA⁵ where n = 0, 1, 2 and A⁵ independently of one another are hydrogen, hydrogen, C₁-C₁₀-alkyl, C₁-C₁₀-haloalkyl, C₃-C₁₀-cycloalkyl, C₃-C₁₀-halocycloalkyl, NH_{2,} mono-(C₁-C₁₀-alkyl)amino, di-(C₁-C₁₀-alkyl)amino, phenyl, benzyl, naphthyl or a saturated or unsaturated aromatic or non-aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO groups as ring members, wherein the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

According to one embodiment A⁵ is H or C₁-C₁₀-alkyl, preferably methyl, ethyl, n-propyl, i-propyl, n-butyl, i-butyl, t-butyl. According to a further embodiment A⁵ is C₁-C₁₀-haloalkyl; preferrably fully or partially halogenated methyl, ethyl, n-propyl, i-propyl, n-butyl, i-butyl, t-butyl, in particular fully or partially halogenated methyl. According to a further embodiment A⁵ is C₂-C₁₀-alkenyl, preferably ethylene. According to a further embodiment A⁵ is C₂-C₁₀-alkynyl, preferred ethynyl, 1-propynyl. According to a further embodiment A⁵ is C₃-C₁₀-cycloalkyl, preferrably cyclopropyl, cyclobutyl, cyclopentyl, in particular cyclopropyl or cyclobutyl. According to a further embodiment A⁵ is C₃-C₁₀-halocycloalkyl, preferrably fully or partially halogenated cyclopropyl, cyclobutyl, cyclopentyl, in particular fully or partially halogenated cyclopropyl. According to a further embodiment A⁵ is C₃-C₁₀-cycloalkenyl, preferably cyclopropenyl. According to a further embodiment A⁵ is NH₂. According to a further embodiment A⁵ is mono-(C₁-C₁₀-alkyl)amino. According to a further embodiment A⁵ is di-(C₁-C₁₀-alkyl)amino.

According to one embodiment A⁵ is phenyl. According to a further embodiment A⁵ is benzyl. According to a further embodiment A⁵ is naphthyl. According to one embodiment A⁵ is a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members.

According to one embodiment phenyl, benzyl, naphthyl and the saturated, partially unsaturated or aromatic 5-, 6-, 7-, 8-, 9- or 10-membered heterocycle may carry one, two, three or four identical or different substituents selected from the group consisting of Cl, I, F, Br, preferably Cl, F. According to a further embodiment the above mentioned groups may carry one, two, three or four identical or different substituents selected from the group consisting of methyl, ethyl, propyl, butyl, preferably methyl. According to a further embodiment the above mentioned groups may carry one, two, three or four identical or different substituents selected from the group consisting of partially or fully halogenated methyl, ethyl, propyl, butyl, preferably partially or fully halogenated methyl, in particular CF₃. According to a further embodiment the above mentioned groups may carry one, two, three or four identical or different substituents selected from the group consisting of methoxy, ethoxy, propoxy, butoxy, preferably methoxy. According to a further embodiment the above mentioned groups may carry one, two, three or four identical or different substituents selected from the group consisting of partially or fully halogenated methoxy, ethoxy, propoxy, butoxy, preferably partially or fully halogenated methoxy, in particular OCF₃.

R¹⁰ in the compounds according to the invention is as defined above with the proviso that, that if 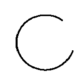 is C₅-cycloalkyl;
- R¹⁰: is not Cl, F, C₁-haloalkyl.

In one embodiment of the invention R¹⁰ is not halogen. In a further embodiment of the invention R¹⁰ is not C₁-C₄-alkyl. In a further embodiment of the invention R¹⁰ is not C₁-C₁₀-alkyl. In a further embodiment of the invention R¹⁰ is not C₁-C₄-haloalkyl. In a further embodiment of the invention R¹⁰ is not C₁-C₁₀-haloalkyl. In a further embodiment of the invention R¹⁰ is not C₂-C₄-alkenyl. In a further embodiment of the invention R¹⁰ is not NA¹A², where A¹ and A² are indepemdly of another C₁-C₄-alkyl. In a further embodiment of the invention R¹⁰ is not NA¹A², where A¹ and A² are independently of another C₃-C₄-cycloalkyl. In a further embodiment of the invention R¹⁰ is not NA¹A², where A¹ and A² are independently of another 5- or 6-membered aromatic ring. In a further embodiment of the invention R¹⁰ is not OA³ where A³ is C₁-C₄-alkyl. In a further embodiment of the invention R¹⁰ is not OA³ where A³ is C₃-C₆-cycloalkyl. In a further embodiment of the invention R¹⁰ is not OA³ where A³ is substituted phenyl. In a further embodiment of the invention R¹⁰ is not 5- or 6-membered aromatic ring.

R¹⁰ in the compounds according to the invention is as defined above with the proviso that if 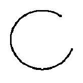 is C₆-cycloalkyl;
- R¹⁰: is not Cl, F, C₁-haloalkyl.

In one embodiment of the invention R¹⁰ is not halogen. In a further embodiment of the invention R¹⁰ is not C₁-C₄-alkyl. In a further embodiment of the invention R¹⁰ is not C₁-C₁₀-alkyl. In a further embodiment of the invention R¹⁰ is not C₁-C₄-haloalkyl. In a further embodiment of the invention R¹⁰ is not C₁-C₁₀-haloalkyl. In a further embodiment of the invention R¹⁰ is not C₂-C₄-alkenyl. In a further embodiment of the invention R¹⁰ is not NA¹A², where A¹ and A² are indepemdly of another C₁-C₄-alkyl. In a further embodiment of the invention R¹⁰ is not NA¹A², where A¹ and A² are independently of another C₃-C₄-cycloalkyl. In a further embodiment of the invention R¹⁰ is not NA¹A², where A¹ and A² are independently of another 5- or 6-membered aromatic ring. In a further embodiment of the invention R¹⁰ is not OA³ where A³ is C₁-C₄-alkyl. In a further embodiment of the invention R¹⁰ is not OA³ where A³ is C₃-C₆-cycloalkyl. In a further embodiment of the invention R¹⁰ is not OA³ where A³ is substituted phenyl. In a further embodiment of the invention R¹⁰ is not 5- or 6-membered aromatic ring.

According to one embodiment, the present invention relates to compounds of the formula la 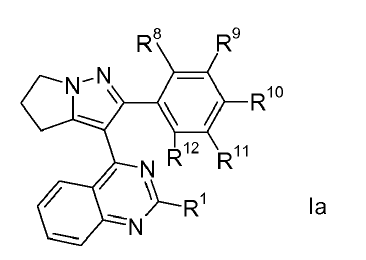 or to the use of the compounds of the formula la for controlling phytopathogenic fungi. Here, the variables are as defined elsewhere herein for formula I, or as defined as being preferred for formula I.

According to a further embodiment, the present invention relates to compounds of the formula Ib 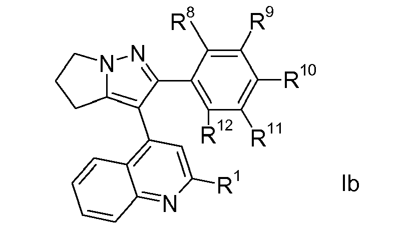 or to the use of the compounds of the formula Ib for controlling phytopathogenic fungi. Here, the variables are as defined elsewhere herein for formula I, or as defined as being preferred for formula I.

According to one embodiment, the present invention relates to compounds of the formula Ic 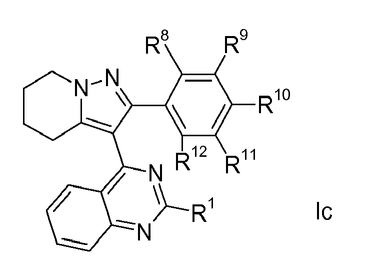 or to the use of the compounds of the formula Ic for controlling phytopathogenic fungi. Here, the variables are as defined elsewhere herein for formula I, or as defined as being preferred for formula I.

According to one embodiment, the present invention relates to compounds of the formula Id 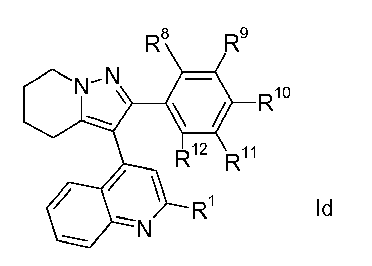 or to the use of the compounds of the formula Id for controlling phytopathogenic fungi. Here, the variables are as defined elsewhere herein for formula I, or as defined as being preferred for formula I.

Preference is given to the compounds of the formula I used according to the invention and to the compounds according to the invention compiled in Tables 1 to 28 below. With regard to the compounds according to the invention the exception as deifned above will be considered. The groups mentioned for a substituent in the tables are furthermore per se, independently of the combination in which they are mentioned, a particularly preferred aspect of the substituent in question.

### Table 1

Compounds of the formula Ia to Id in which R¹ is H and the combination of R⁸ to R¹² for a compound corresponds in each case to one row of Table A

### Table 2

Compounds of the formula Ia to Id in which R¹ is OCH₃ and the combination of R⁸ to R¹² for a compound corresponds in each case to one row of Table A

### Table 3

Compounds of the formula Ia to Id in which R¹ is OC₂H₅ and the combination of R⁸ to R¹² for a compound corresponds in each case to one row of Table A

### Table 4

Compounds of the formula Ia to Id in which R¹ is Oi-C₃H₇ and the combination of R⁸ to R¹² for a compound corresponds in each case to one row of Table A

### Table 5

Compounds of the formula Ia to Id in which R¹ is NHCH₃ and the combination of R⁸ to R¹² for a compound corresponds in each case to one row of Table A

### Table 6

Compounds of the formula Ia to Id in which R¹ is N(CH₃)₂ and the combination of R⁸ to R¹² for a compound corresponds in each case to one row of Table A

### Table 7

Compounds of the formula Ia to Id in which R¹ is NHC₂H₅ and the combination of R⁸ to R¹² for a compound corresponds in each case to one row of Table A

### Table 8

Compounds of the formula Ia to Id in which R¹ is NHi-C₃H₇ and the combination of R⁸ to R¹² for a compound corresponds in each case to one row of Table A

### Table 9

Compounds of the formula Ia to Id in which R¹ is NHn-C₄H₉ and the combination of R⁸ to R¹² for a compound corresponds in each case to one row of Table A

### Table 10

Compounds of the formula Ia to Id in which R¹ is NHC₆H₅ and the combination of R⁸ to R¹² for a compound corresponds in each case to one row of Table A

### Table 11

Compounds of the formula la to Id in which R¹ is NHC₆H₄p-OCH₃ and the combination of R⁸ to R¹² for a compound corresponds in each case to one row of Table A

### Table 12

Compounds of the formula la to Id in which R¹ is NHC₆H₄m-OCH₃ and the combination of R⁸ to R¹² for a compound corresponds in each case to one row of Table A

### Table 13

Compounds of the formula la to Id in which R¹ is NHC₆H₄o-OCH₃ and the combination of R⁸ to R¹² for a compound corresponds in each case to one row of Table A

### Table 14

Compounds of the formula la to Id in which R¹ is NHC₆H₄p-F and the combination of R⁸ to R¹² for a compound corresponds in each case to one row of Table A

### Table 15

Compounds of the formula la to Id in which R¹ is NHC₆H₄m-F and the combination of R⁸ to R¹² for a compound corresponds in each case to one row of Table A

### Table 16

Compounds of the formula la to Id in which R¹ is NHC₆H₄o-F and the combination of R⁸ to R¹² for a compound corresponds in each case to one row of Table A

### Table 17

Compounds of the formula la to Id in which R¹ is NHC₆H₄p-Cl and the combination of R⁸ to R¹² for a compound corresponds in each case to one row of Table A

### Table 18

Compounds of the formula la to Id in which R¹ is NHC₆H₄m-Cl and the combination of R⁸ to R¹² for a compound corresponds in each case to one row of Table A

### Table 19

Compounds of the formula la to Id in which R¹ is NHC₆H₄o-Cl and the combination of R⁸ to R¹² for a compound corresponds in each case to one row of Table A

### Table 20

Compounds of the formula la to Id in which R¹ is cyclopropylamino and the combination of R⁸ to R¹² for a compound corresponds in each case to one row of Table A

### Table 21

Compounds of the formula la to Id in which R¹ is cyclopentylamino and the combination of R⁸ to R¹² for a compound corresponds in each case to one row of Table A

### Table 22

Compounds of the formula la to Id in which R¹ is cyclohexylamino and the combination of R⁸ to R¹² for a compound corresponds in each case to one row of Table A

### Table 23

Compounds of the formula la to Id in which R¹ is methoxyethylamino and the combination of R⁸ to R¹² for a compound corresponds in each case to one row of Table A

### Table 24

Compounds of the formula la to Id in which R¹ is amineethylamino and the combination of R⁸ to R¹² for a compound corresponds in each case to one row of Table A

### Table 25

Compounds of the formula la to Id in which R¹ is methylamineethylamino and the combination of R⁸ to R¹² for a compound corresponds in each case to one row of Table A

### Table 26

Compounds of the formula la to Id in which R¹ is dimethylamineethylamino and the combination of R⁸ to R¹² for a compound corresponds in each case to one row of Table A

### Table 27

Compounds of the formula la to Id in which R¹ is morpholin-4-ylethylamino and the combination of R⁸ to R¹² for a compound corresponds in each case to one row of Table A

### Table 28

Compounds of the formula la to Id in which R¹ is piperazine-4-ylethylamino and the combination of R⁸ to R¹² for a compound corresponds in each case to one row of Table A

**Table A**

| | **R⁸** | **R⁹** | **R¹⁰** | **R¹¹** | **R¹²** |
|---|---|---|---|---|---|
| A-1 | F | H | H | H | H |
| A-2 | Cl | H | H | H | H |
| A-3 | CH₃ | H | H | H | H |
| A-4 | CF₃ | H | H | H | H |
| A-5 | OCH₃ | H | H | H | H |
| A-6 | OC₂H₅ | H | H | H | H |
| A-7 | OCF₃ | H | H | H | H |
| A-8 | H | F | H | H | H |
| A-9 | H | Cl | H | H | H |
| A-10 | H | CH₃ | H | H | H |
| A-11 | H | CF₃ | H | H | H |
| A-12 | H | OCH₃ | H | H | H |
| A-13 | H | OC₂H₅ | H | H | H |
| A-14 | H | OCF₃ | H | H | H |
| A-15 | H | H | F | H | H |
| A-16 | H | H | Cl | H | H |
| A-17 | H | H | CH₃ | H | H |
| A-18 | H | H | CF₃ | H | H |
| A-19 | H | H | OCH₃ | H | H |
| A-20 | H | H | OC₂H₅ | H | H |
| A-21 | H | H | OCF₃ | H | H |
| A-22 | H | H | H | F | H |
| A-23 | H | H | H | Cl | H |
| A-24 | H | H | H | CH₃ | H |
| A-25 | H | H | H | CF₃ | H |
| A-26 | H | H | H | OCH₃ | H |
| A-27 | H | H | H | OC₂H₅ | H |
| A-28 | H | H | H | OCF₃ | H |
| A-29 | H | H | H | H | F |
| A-30 | H | H | H | H | Cl |
| A-31 | H | H | H | H | CH₃ |
| A-32 | H | H | H | H | CF₃ |
| A-33 | H | H | H | H | OCH₃ |
| A-34 | H | H | H | H | OC₂H₅ |
| A-35 | H | H | H | H | OCF₃ |
| A-36 | F | F | H | H | H |
| A-37 | Cl | F | H | H | H |
| A-38 | CH₃ | F | H | H | H |
| A-39 | CF₃ | F | H | H | H |
| A-40 | OCH₃ | F | H | H | H |
| A-41 | OC₂H₅ | F | H | H | H |
| A-42 | OCF₃ | F | H | H | H |
| A-43 | F | Cl | H | H | H |
| A-44 | Cl | Cl | H | H | H |
| A-45 | CH₃ | Cl | H | H | H |
| A-46 | CF₃ | Cl | H | H | H |
| A-47 | OCH₃ | Cl | H | H | H |
| A-48 | OC₂H₅ | Cl | H | H | H |
| A-49 | OCF₃ | Cl | H | H | H |
| A-50 | F | CH₃ | H | H | H |
| A-51 | Cl | CH₃ | H | H | H |
| A-52 | CH₃ | CH₃ | H | H | H |
| A-53 | CF₃ | CH₃ | H | H | H |
| A-54 | OCH₃ | CH₃ | H | H | H |
| A-55 | OC₂H₅ | CH₃ | H | H | H |
| A-56 | OCF₃ | CH₃ | H | H | H |
| A-57 | F | CF₃ | H | H | H |
| A-58 | Cl | CF₃ | H | H | H |
| A-59 | CH₃ | CF₃ | H | H | H |
| A-60 | CF₃ | CF₃ | H | H | H |
| A-61 | OCH₃ | CF₃ | H | H | H |
| A-62 | OC₂H₅ | CF₃ | H | H | H |
| A-63 | OCF₃ | CF₃ | H | H | H |
| A-64 | F | OCH₃ | H | H | H |
| A-65 | Cl | OCH₃ | H | H | H |
| A-66 | CH₃ | OCH₃ | H | H | H |
| A-67 | CF₃ | OCH₃ | H | H | H |
| A-68 | OCH₃ | OCH₃ | H | H | H |
| A-69 | OC₂H₅ | OCH₃ | H | H | H |
| A-70 | OCF₃ | OCH₃ | H | H | H |
| A-71 | F | OC₂H₅ | H | H | H |
| A-72 | Cl | OC₂H₅ | H | H | H |
| A-73 | CH₃ | OC₂H₅ | H | H | H |
| A-74 | CF₃ | OC₂H₅ | H | H | H |
| A-75 | OCH₃ | OC₂H₅ | H | H | H |
| A-76 | OC₂H₅ | OC₂H₅ | H | H | H |
| A-77 | OCF₃ | OC₂H₅ | H | H | H |
| A-78 | F | OCF₃ | H | H | H |
| A-79 | Cl | OCF₃ | H | H | H |
| A-80 | CH₃ | OCF₃ | H | H | H |
| A-81 | CF₃ | OCF₃ | H | H | H |
| A-82 | OCH₃ | OCF₃ | H | H | H |
| A-83 | OC₂H₅ | OCF₃ | H | H | H |
| A-84 | OCF₃ | OCF₃ | H | H | H |
| A-85 | F | H | F | H | H |
| A-86 | Cl | H | F | H | H |
| A-87 | CH₃ | H | F | H | H |
| A-88 | CF₃ | H | F | H | H |
| A-89 | OCH₃ | H | F | H | H |
| A-90 | OC₂H₅ | H | F | H | H |
| A-91 | OCF₃ | H | F | H | H |
| A-92 | F | H | Cl | H | H |
| A-93 | Cl | H | Cl | H | H |
| A-94 | CH₃ | H | Cl | H | H |
| A-95 | CF₃ | H | Cl | H | H |
| A-96 | OCH₃ | H | Cl | H | H |
| A-97 | OC₂H₅ | H | Cl | H | H |
| A-98 | OCF₃ | H | Cl | H | H |
| A-99 | F | H | CH₃ | H | H |
| A-100 | Cl | H | CH₃ | H | H |
| A-101 | CH₃ | H | CH₃ | H | H |
| A-102 | CF₃ | H | CH₃ | H | H |
| A-103 | OCH₃ | H | CH₃ | H | H |
| A-104 | OC₂H₅ | H | CH₃ | H | H |
| A-105 | OCF₃ | H | CH₃ | H | H |
| A-106 | F | H | CF₃ | H | H |
| A-107 | Cl | H | CF₃ | H | H |
| A-108 | CH₃ | H | CF₃ | H | H |
| A-109 | CF₃ | H | CF₃ | H | H |
| A-110 | OCH₃ | H | CF₃ | H | H |
| A-111 | OC₂H₅ | H | CF₃ | H | H |
| A-112 | OCF₃ | H | CF₃ | H | H |
| A-113 | F | H | OCH₃ | H | H |
| A-114 | Cl | H | OCH₃ | H | H |
| A-115 | CH₃ | H | OCH₃ | H | H |
| A-116 | CF₃ | H | OCH₃ | H | H |
| A-117 | OCH₃ | H | OCH₃ | H | H |
| A-118 | OC₂H₅ | H | OCH₃ | H | H |
| A-119 | OCF₃ | H | OCH₃ | H | H |
| A-120 | F | H | OC₂H₅ | H | H |
| A-121 | Cl | H | OC₂H₅ | H | H |
| A-122 | CH₃ | H | OC₂H₅ | H | H |
| A-123 | CF₃ | H | OC₂H₅ | H | H |
| A-124 | OCH₃ | H | OC₂H₅ | H | H |
| A-125 | OC₂H₅ | H | OC₂H₅ | H | H |
| A-126 | OCF₃ | H | OC₂H₅ | H | H |
| A-127 | F | H | OCF₃ | H | H |
| A-128 | Cl | H | OCF₃ | H | H |
| A-129 | CH₃ | H | OCF₃ | H | H |
| A-130 | CF₃ | H | OCF₃ | H | H |
| A-131 | OCH₃ | H | OCF₃ | H | H |
| A-132 | OC₂H₅ | H | OCF₃ | H | H |
| A-133 | OCF₃ | H | OCF₃ | H | H |
| A-134 | F | H | H | F | H |
| A-135 | Cl | H | H | F | H |
| A-136 | CH₃ | H | H | F | H |
| A-137 | CF₃ | H | H | F | H |
| A-138 | OCH₃ | H | H | F | H |
| A-139 | OC₂H₅ | H | H | F | H |
| A-140 | OCF₃ | H | H | F | H |
| A-141 | F | H | H | Cl | H |
| A-142 | Cl | H | H | Cl | H |
| A-143 | CH₃ | H | H | Cl | H |
| A-144 | CF₃ | H | H | Cl | H |
| A-145 | OCH₃ | H | H | Cl | H |
| A-146 | OC₂H₅ | H | H | Cl | H |
| A-147 | OCF₃ | H | H | Cl | H |
| A-148 | F | H | H | CH₃ | H |
| A-149 | Cl | H | H | CH₃ | H |
| A-150 | CH₃ | H | H | CH₃ | H |
| A-151 | CF₃ | H | H | CH₃ | H |
| A-152 | OCH₃ | H | H | CH₃ | H |
| A-153 | OC₂H₅ | H | H | CH₃ | H |
| A-154 | OCF₃ | H | H | CH₃ | H |
| A-155 | F | H | H | CF₃ | H |
| A-156 | Cl | H | H | CF₃ | H |
| A-157 | CH₃ | H | H | CF₃ | H |
| A-158 | CF₃ | H | H | CF₃ | H |
| A-159 | OCH₃ | H | H | CF₃ | H |
| A-160 | OC₂H₅ | H | H | CF₃ | H |
| A-161 | OCF₃ | H | H | CF₃ | H |
| A-162 | F | H | H | OCH₃ | H |
| A-163 | Cl | H | H | OCH₃ | H |
| A-164 | CH₃ | H | H | OCH₃ | H |
| A-165 | CF₃ | H | H | OCH₃ | H |
| A-166 | OCH₃ | H | H | OCH₃ | H |
| A-167 | OC₂H₅ | H | H | OCH₃ | H |
| A-168 | OCF₃ | H | H | OCH₃ | H |
| A-169 | F | H | H | OC₂H₅ | H |
| A-170 | Cl | H | H | OC₂H₅ | H |
| A-171 | CH₃ | H | H | OC₂H₅ | H |
| A-172 | CF₃ | H | H | OC₂H₅ | H |
| A-173 | OCH₃ | H | H | OC₂H₅ | H |
| A-174 | OC₂H₅ | H | H | OC₂H₅ | H |
| A-175 | OCF₃ | H | H | OC₂H₅ | H |
| A-176 | F | H | H | OCF₃ | H |
| A-177 | Cl | H | H | OCF₃ | H |
| A-178 | CH₃ | H | H | OCF₃ | H |
| A-179 | CF₃ | H | H | OCF₃ | H |
| A-180 | OCH₃ | H | H | OCF₃ | H |
| A-181 | OC₂H₅ | H | H | OCF₃ | H |
| A-182 | OCF₃ | H | H | OCF₃ | H |
| A-183 | F | H | H | H | F |
| A-184 | Cl | H | H | H | F |
| A-185 | CH₃ | H | H | H | F |
| A-186 | CF₃ | H | H | H | F |
| A-187 | OCH₃ | H | H | H | F |
| A-188 | OC₂H₅ | H | H | H | F |
| A-189 | OCF₃ | H | H | H | F |
| A-190 | F | H | H | H | Cl |
| A-191 | Cl | H | H | H | Cl |
| A-192 | CH₃ | H | H | H | Cl |
| A-193 | CF₃ | H | H | H | Cl |
| A-194 | OCH₃ | H | H | H | Cl |
| A-195 | OC₂H₅ | H | H | H | Cl |
| A-196 | OCF₃ | H | H | H | Cl |
| A-197 | F | H | H | H | CH₃ |
| A-198 | Cl | H | H | H | CH₃ |
| A-199 | CH₃ | H | H | H | CH₃ |
| A-200 | CF₃ | H | H | H | CH₃ |
| A-201 | OCH₃ | H | H | H | CH₃ |
| A-202 | OC₂H₅ | H | H | H | CH₃ |
| A-203 | OCF₃ | H | H | H | CH₃ |
| A-204 | F | H | H | H | CF₃ |
| A-205 | Cl | H | H | H | CF₃ |
| A-206 | CH₃ | H | H | H | CF₃ |
| A-207 | CF₃ | H | H | H | CF₃ |
| A-208 | OCH₃ | H | H | H | CF₃ |
| A-209 | OC₂H₅ | H | H | H | CF₃ |
| A-210 | OCF₃ | H | H | H | CF₃ |
| A-211 | F | H | H | H | OCH₃ |
| A-212 | Cl | H | H | H | OCH₃ |
| A-213 | CH₃ | H | H | H | OCH₃ |
| A-214 | CF₃ | H | H | H | OCH₃ |
| A-215 | OCH₃ | H | H | H | OCH₃ |
| A-216 | OC₂H₅ | H | H | H | OCH₃ |
| A-217 | OCF₃ | H | H | H | OCH₃ |
| A-218 | F | H | H | H | OC₂H₅ |
| A-219 | Cl | H | H | H | OC₂H₅ |
| A-220 | CH₃ | H | H | H | OC₂H₅ |
| A-221 | CF₃ | H | H | H | OC₂H₅ |
| A-222 | OCH₃ | H | H | H | OC₂H₅ |
| A-223 | OC₂H₅ | H | H | H | OC₂H₅ |
| A-224 | OCF₃ | H | H | H | OC₂H₅ |
| A-225 | F | H | H | H | OCF₃ |
| A-226 | Cl | H | H | H | OCF₃ |
| A-227 | CH₃ | H | H | H | OCF₃ |
| A-228 | CF₃ | H | H | H | OCF₃ |
| A-229 | OCH₃ | H | H | H | OCF₃ |
| A-230 | OC₂H₅ | H | H | H | OCF₃ |
| A-231 | OCF₃ | H | H | H | OCF₃ |
| A-232 | H | F | F | H | H |
| A-233 | H | Cl | F | H | H |
| A-234 | H | CH₃ | F | H | H |
| A-235 | H | CF₃ | F | H | H |
| A-236 | H | OCH₃ | F | H | H |
| A-237 | H | OC₂H₅ | F | H | H |
| A-238 | H | OCF₃ | F | H | H |
| A-239 | H | F | Cl | H | H |
| A-240 | H | Cl | Cl | H | H |
| A-241 | H | CH₃ | Cl | H | H |
| A-242 | H | CF₃ | Cl | H | H |
| A-243 | H | OCH₃ | Cl | H | H |
| A-244 | H | OC₂H₅ | Cl | H | H |
| A-245 | H | OCF₃ | Cl | H | H |
| A-246 | H | F | CH₃ | H | H |
| A-247 | H | Cl | CH₃ | H | H |
| A-248 | H | CH₃ | CH₃ | H | H |
| A-249 | H | CF₃ | CH₃ | H | H |
| A-250 | H | OCH₃ | CH₃ | H | H |
| A-251 | H | OC₂H₅ | CH₃ | H | H |
| A-252 | H | OCF₃ | CH₃ | H | H |
| A-253 | H | F | CF₃ | H | H |
| A-254 | H | Cl | CF₃ | H | H |
| A-255 | H | CH₃ | CF₃ | H | H |
| A-256 | H | CF₃ | CF₃ | H | H |
| A-257 | H | OCH₃ | CF₃ | H | H |
| A-258 | H | OC₂H₅ | CF₃ | H | H |
| A-259 | H | OCF₃ | CF₃ | H | H |
| A-260 | H | F | OCH₃ | H | H |
| A-261 | H | Cl | OCH₃ | H | H |
| A-262 | H | CH₃ | OCH₃ | H | H |
| A-263 | H | CF₃ | OCH₃ | H | H |
| A-264 | H | OCH₃ | OCH₃ | H | H |
| A-265 | H | OC₂H₅ | OCH₃ | H | H |
| A-266 | H | OCF₃ | OCH₃ | H | H |
| A-267 | H | F | OC₂H₅ | H | H |
| A-268 | H | Cl | OC₂H₅ | H | H |
| A-269 | H | CH₃ | OC₂H₅ | H | H |
| A-270 | H | CF₃ | OC₂H₅ | H | H |
| A-271 | H | OCH₃ | OC₂H₅ | H | H |
| A-272 | H | OC₂H₅ | OC₂H₅ | H | H |
| A-273 | H | OCF₃ | OC₂H₅ | H | H |
| A-274 | H | F | OCF₃ | H | H |
| A-275 | H | Cl | OCF₃ | H | H |
| A-276 | H | CH₃ | OCF₃ | H | H |
| A-277 | H | CF₃ | OCF₃ | H | H |
| A-278 | H | OCH₃ | OCF₃ | H | H |
| A-279 | H | OC₂H₅ | OCF₃ | H | H |
| A-280 | H | OCF₃ | OCF₃ | H | H |
| A-281 | H | F | H | F | H |
| A-282 | H | Cl | H | F | H |
| A-283 | H | CH₃ | H | F | H |
| A-284 | H | CF₃ | H | F | H |
| A-285 | H | OCH₃ | H | F | H |
| A-286 | H | OC₂H₅ | H | F | H |
| A-287 | H | OCF₃ | H | F | H |
| A-288 | H | F | H | Cl | H |
| A-289 | H | Cl | H | Cl | H |
| A-290 | H | CH₃ | H | Cl | H |
| A-291 | H | CF₃ | H | Cl | H |
| A-292 | H | OCH₃ | H | Cl | H |
| A-293 | H | OC₂H₅ | H | Cl | H |
| A-294 | H | OCF₃ | H | Cl | H |
| A-295 | H | F | H | CH₃ | H |
| A-296 | H | Cl | H | CH₃ | H |
| A-297 | H | CH₃ | H | CH₃ | H |
| A-298 | H | CF₃ | H | CH₃ | H |
| A-299 | H | OCH₃ | H | CH₃ | H |
| A-300 | H | OC₂H₅ | H | CH₃ | H |
| A-301 | H | OCF₃ | H | CH₃ | H |
| A-302 | H | F | H | CF₃ | H |
| A-303 | H | Cl | H | CF₃ | H |
| A-304 | H | CH₃ | H | CF₃ | H |
| A-305 | H | CF₃ | H | CF₃ | H |
| A-306 | H | OCH₃ | H | CF₃ | H |
| A-307 | H | OC₂H₅ | H | CF₃ | H |
| A-308 | H | OCF₃ | H | CF₃ | H |
| A-309 | H | F | H | OCH₃ | H |
| A-310 | H | Cl | H | OCH₃ | H |
| A-311 | H | CH₃ | H | OCH₃ | H |
| A-312 | H | CF₃ | H | OCH₃ | H |
| A-313 | H | OCH₃ | H | OCH₃ | H |
| A-314 | H | OC₂H₅ | H | OCH₃ | H |
| A-315 | H | OCF₃ | H | OCH₃ | H |
| A-316 | H | F | H | OC₂H₅ | H |
| A-317 | H | Cl | H | OC₂H₅ | H |
| A-318 | H | CH₃ | H | OC₂H₅ | H |
| A-319 | H | CF₃ | H | OC₂H₅ | H |
| A-320 | H | OCH₃ | H | OC₂H₅ | H |
| A-321 | H | OC₂H₅ | H | OC₂H₅ | H |
| A-322 | H | OCF₃ | H | OC₂H₅ | H |
| A-323 | H | F | H | OCF₃ | H |
| A-324 | H | Cl | H | OCF₃ | H |
| A-325 | H | CH₃ | H | OCF₃ | H |
| A-326 | H | CF₃ | H | OCF₃ | H |
| A-327 | H | OCH₃ | H | OCF₃ | H |
| A-328 | H | OC₂H₅ | H | OCF₃ | H |
| A-329 | H | OCF₃ | H | OCF₃ | H |
| A-330 | H | F | H | H | F |
| A-331 | H | Cl | H | H | F |
| A-332 | H | CH₃ | H | H | F |
| A-333 | H | CF₃ | H | H | F |
| A-334 | H | OCH₃ | H | H | F |
| A-335 | H | OC₂H₅ | H | H | F |
| A-336 | H | OCF₃ | H | H | F |
| A-337 | H | F | H | H | Cl |
| A-338 | H | Cl | H | H | Cl |
| A-339 | H | CH₃ | H | H | Cl |
| A-340 | H | CF₃ | H | H | Cl |
| A-341 | H | OCH₃ | H | H | Cl |
| A-342 | H | OC₂H₅ | H | H | Cl |
| A-343 | H | OCF₃ | H | H | Cl |
| A-344 | H | F | H | H | CH₃ |
| A-345 | H | Cl | H | H | CH₃ |
| A-346 | H | CH₃ | H | H | CH₃ |
| A-347 | H | CF₃ | H | H | CH₃ |
| A-348 | H | OCH₃ | H | H | CH₃ |
| A-349 | H | OC₂H₅ | H | H | CH₃ |
| A-350 | H | OCF₃ | H | H | CH₃ |
| A-351 | H | F | H | H | CF₃ |
| A-352 | H | Cl | H | H | CF₃ |
| A-353 | H | CH₃ | H | H | CF₃ |
| A-354 | H | CF₃ | H | H | CF₃ |
| A-355 | H | OCH₃ | H | H | CF₃ |
| A-356 | H | OC₂H₅ | H | H | CF₃ |
| A-357 | H | OCF₃ | H | H | CF₃ |
| A-358 | H | F | H | H | OCH₃ |
| A-359 | H | Cl | H | H | OCH₃ |
| A-360 | H | CH₃ | H | H | OCH₃ |
| A-361 | H | CF₃ | H | H | OCH₃ |
| A-362 | H | OCH₃ | H | H | OCH₃ |
| A-363 | H | OC₂H₅ | H | H | OCH₃ |
| A-364 | H | OCF₃ | H | H | OCH₃ |
| A-365 | H | F | H | H | OC₂H₅ |
| A-366 | H | Cl | H | H | OC₂H₅ |
| A-367 | H | CH₃ | H | H | OC₂H₅ |
| A-368 | H | CF₃ | H | H | OC₂H₅ |
| A-369 | H | OCH₃ | H | H | OC₂H₅ |
| A-370 | H | OC₂H₅ | H | H | OC₂H₅ |
| A-371 | H | OCF₃ | H | H | OC₂H₅ |
| A-372 | H | F | H | H | OCF₃ |
| A-373 | H | Cl | H | H | OCF₃ |
| A-374 | H | CH₃ | H | H | OCF₃ |
| A-375 | H | CF₃ | H | H | OCF₃ |
| A-376 | H | OCH₃ | H | H | OCF₃ |
| A-377 | H | OC₂H₅ | H | H | OCF₃ |
| A-378 | H | OCF₃ | H | H | OCF₃ |
| A-379 | H | H | F | F | H |
| A-380 | H | H | Cl | F | H |
| A-381 | H | H | CH₃ | F | H |
| A-382 | H | H | CF₃ | F | H |
| A-383 | H | H | OCH₃ | F | H |
| A-384 | H | H | OC₂H₅ | F | H |
| A-385 | H | H | OCF₃ | F | H |
| A-386 | H | H | F | Cl | H |
| A-387 | H | H | Cl | Cl | H |
| A-388 | H | H | CH₃ | Cl | H |
| A-389 | H | H | CF₃ | Cl | H |
| A-390 | H | H | OCH₃ | Cl | H |
| A-391 | H | H | OC₂H₅ | Cl | H |
| A-392 | H | H | OCF₃ | Cl | H |
| A-393 | H | H | F | CH₃ | H |
| A-394 | H | H | Cl | CH₃ | H |
| A-395 | H | H | CH₃ | CH₃ | H |
| A-396 | H | H | CF₃ | CH₃ | H |
| A-397 | H | H | OCH₃ | CH₃ | H |
| A-398 | H | H | OC₂H₅ | CH₃ | H |
| A-399 | H | H | OCF₃ | CH₃ | H |
| A-400 | H | H | F | CF₃ | H |
| A-401 | H | H | Cl | CF₃ | H |
| A-402 | H | H | CH₃ | CF₃ | H |
| A-403 | H | H | CF₃ | CF₃ | H |
| A-404 | H | H | OCH₃ | CF₃ | H |
| A-405 | H | H | OC₂H₅ | CF₃ | H |
| A-406 | H | H | OCF₃ | CF₃ | H |
| A-407 | H | H | F | OCH₃ | H |
| A-408 | H | H | Cl | OCH₃ | H |
| A-409 | H | H | CH₃ | OCH₃ | H |
| A-410 | H | H | CF₃ | OCH₃ | H |
| A-411 | H | H | OCH₃ | OCH₃ | H |
| A-412 | H | H | OC₂H₅ | OCH₃ | H |
| A-413 | H | H | OCF₃ | OCH₃ | H |
| A-414 | H | H | F | OC₂H₅ | H |
| A-415 | H | H | Cl | OC₂H₅ | H |
| A-416 | H | H | CH₃ | OC₂H₅ | H |
| A-417 | H | H | CF₃ | OC₂H₅ | H |
| A-418 | H | H | OCH₃ | OC₂H₅ | H |
| A-419 | H | H | OC₂H₅ | OC₂H₅ | H |
| A-420 | H | H | OCF₃ | OC₂H₅ | H |
| A-421 | H | H | F | OCF₃ | H |
| A-422 | H | H | Cl | OCF₃ | H |
| A-423 | H | H | CH₃ | OCF₃ | H |
| A-424 | H | H | CF₃ | OCF₃ | H |
| A-425 | H | H | OCH₃ | OCF₃ | H |
| A-426 | H | H | OC₂H₅ | OCF₃ | H |
| A-427 | H | H | OCF₃ | OCF₃ | H |
| A-428 | H | H | F | H | F |
| A-429 | H | H | Cl | H | F |
| A-430 | H | H | CH₃ | H | F |
| A-431 | H | H | CF₃ | H | F |
| A-432 | H | H | OCH₃ | H | F |
| A-433 | H | H | OC₂H₅ | H | F |
| A-434 | H | H | OCF₃ | H | F |
| A-435 | H | H | F | H | Cl |
| A-436 | H | H | Cl | H | Cl |
| A-437 | H | H | CH₃ | H | Cl |
| A-438 | H | H | CF₃ | H | Cl |
| A-439 | H | H | OCH₃ | H | Cl |
| A-440 | H | H | OC₂H₅ | H | Cl |
| A-441 | H | H | OCF₃ | H | Cl |
| A-442 | H | H | F | H | CH₃ |
| A-443 | H | H | Cl | H | CH₃ |
| A-444 | H | H | CH₃ | H | CH₃ |
| A-445 | H | H | CF₃ | H | CH₃ |
| A-446 | H | H | OCH₃ | H | CH₃ |
| A-447 | H | H | OC₂H₅ | H | CH₃ |
| A-448 | H | H | OCF₃ | H | CH₃ |
| A-449 | H | H | F | H | CF₃ |
| A-450 | H | H | Cl | H | CF₃ |
| A-451 | H | H | CH₃ | H | CF₃ |
| A-452 | H | H | CF₃ | H | CF₃ |
| A-453 | H | H | OCH₃ | H | CF₃ |
| A-454 | H | H | OC₂H₅ | H | CF₃ |
| A-455 | H | H | OCF₃ | H | CF₃ |
| A-456 | H | H | F | H | OCH₃ |
| A-457 | H | H | Cl | H | OCH₃ |
| A-458 | H | H | CH₃ | H | OCH₃ |
| A-459 | H | H | CF₃ | H | OCH₃ |
| A-460 | H | H | OCH₃ | H | OCH₃ |
| A-461 | H | H | OC₂H₅ | H | OCH₃ |
| A-462 | H | H | OCF₃ | H | OCH₃ |
| A-463 | H | H | F | H | OC₂H₅ |
| A-464 | H | H | Cl | H | OC₂H₅ |
| A-465 | H | H | CH₃ | H | OC₂H₅ |
| A-466 | H | H | CF₃ | H | OC₂H₅ |
| A-467 | H | H | OCH₃ | H | OC₂H₅ |
| A-468 | H | H | OC₂H₅ | H | OC₂H₅ |
| A-469 | H | H | OCF₃ | H | OC₂H₅ |
| A-470 | H | H | F | H | OCF₃ |
| A-471 | H | H | Cl | H | OCF₃ |
| A-472 | H | H | CH₃ | H | OCF₃ |
| A-473 | H | H | CF₃ | H | OCF₃ |
| A-474 | H | H | OCH₃ | H | OCF₃ |
| A-475 | H | H | OC₂H₅ | H | OCF₃ |
| A-476 | H | H | OCF₃ | H | OCF₃ |
| A-477 | H | H | H | F | F |
| A-478 | H | H | H | Cl | F |
| A-479 | H | H | H | CH₃ | F |
| A-480 | H | H | H | CF₃ | F |
| A-481 | H | H | H | OCH₃ | F |
| A-482 | H | H | H | OC₂H₅ | F |
| A-483 | H | H | H | OCF₃ | F |
| A-484 | H | H | H | F | Cl |
| A-485 | H | H | H | Cl | Cl |
| A-486 | H | H | H | CH₃ | Cl |
| A-487 | H | H | H | CF₃ | Cl |
| A-488 | H | H | H | OCH₃ | Cl |
| A-489 | H | H | H | OC₂H₅ | Cl |
| A-490 | H | H | H | OCF₃ | Cl |
| A-491 | H | H | H | F | CH₃ |
| A-492 | H | H | H | Cl | CH₃ |
| A-493 | H | H | H | CH₃ | CH₃ |
| A-494 | H | H | H | CF₃ | CH₃ |
| A-495 | H | H | H | OCH₃ | CH₃ |
| A-496 | H | H | H | OC₂H₅ | CH₃ |
| A-497 | H | H | H | OCF₃ | CH₃ |
| A-498 | H | H | H | F | CF₃ |
| A-499 | H | H | H | Cl | CF₃ |
| A-500 | H | H | H | CH₃ | CF₃ |
| A-501 | H | H | H | CF₃ | CF₃ |
| A-502 | H | H | H | OCH₃ | CF₃ |
| A-503 | H | H | H | OC₂H₅ | CF₃ |
| A-504 | H | H | H | OCF₃ | CF₃ |
| A-505 | H | H | H | F | OCH₃ |
| A-506 | H | H | H | Cl | OCH₃ |
| A-507 | H | H | H | CH₃ | OCH₃ |
| A-508 | H | H | H | CF₃ | OCH₃ |
| A-509 | H | H | H | OCH₃ | OCH₃ |
| A-510 | H | H | H | OC₂H₅ | OCH₃ |
| A-511 | H | H | H | OCF₃ | OCH₃ |
| A-512 | H | H | H | F | OC₂H₅ |
| A-513 | H | H | H | Cl | OC₂H₅ |
| A-514 | H | H | H | CH₃ | OC₂H₅ |
| A-515 | H | H | H | CF₃ | OC₂H₅ |
| A-516 | H | H | H | OCH₃ | OC₂H₅ |
| A-517 | H | H | H | OC₂H₅ | OC₂H₅ |
| A-518 | H | H | H | OCF₃ | OC₂H₅ |
| A-519 | H | H | H | F | OCF₃ |
| A-520 | H | H | H | Cl | OCF₃ |
| A-521 | H | H | H | CH₃ | OCF₃ |
| A-522 | H | H | H | CF₃ | OCF₃ |
| A-523 | H | H | H | OCH₃ | OCF₃ |
| A-524 | H | H | H | OC₂H₅ | OCF₃ |
| A-525 | H | H | H | OCF₃ | OCF₃ |
| A-526 | F | H | F | H | F |
| A-527 | Cl | H | F | H | F |
| A-528 | CH₃ | H | F | H | F |
| A-529 | CF₃ | H | F | H | F |
| A-530 | OCH₃ | H | F | H | F |
| A-531 | OC₂H₅ | H | F | H | F |
| A-532 | OCF₃ | H | F | H | F |
| A-533 | F | H | Cl | H | F |
| A-534 | Cl | H | Cl | H | F |
| A-535 | CH₃ | H | Cl | H | F |
| A-536 | CF₃ | H | Cl | H | F |
| A-537 | OCH₃ | H | Cl | H | F |
| A-538 | OC₂H₅ | H | Cl | H | F |
| A-539 | OCF₃ | H | Cl | H | F |
| A-540 | F | H | CH₃ | H | F |
| A-541 | Cl | H | CH₃ | H | F |
| A-542 | CH₃ | H | CH₃ | H | F |
| A-543 | CF₃ | H | CH₃ | H | F |
| A-544 | OCH₃ | H | CH₃ | H | F |
| A-545 | OC₂H₅ | H | CH₃ | H | F |
| A-546 | OCF₃ | H | CH₃ | H | F |
| A-547 | F | H | CF₃ | H | F |
| A-548 | Cl | H | CF₃ | H | F |
| A-549 | CH₃ | H | CF₃ | H | F |
| A-550 | CF₃ | H | CF₃ | H | F |
| A-551 | OCH₃ | H | CF₃ | H | F |
| A-552 | OC₂H₅ | H | CF₃ | H | F |
| A-553 | OCF₃ | H | CF₃ | H | F |
| A-554 | F | H | OCH₃ | H | F |
| A-555 | Cl | H | OCH₃ | H | F |
| A-556 | CH₃ | H | OCH₃ | H | F |
| A-557 | CF₃ | H | OCH₃ | H | F |
| A-558 | OCH₃ | H | OCH₃ | H | F |
| A-559 | OC₂H₅ | H | OCH₃ | H | F |
| A-560 | OCF₃ | H | OCH₃ | H | F |
| A-561 | F | H | OC₂H₅ | H | F |
| A-562 | Cl | H | OC₂H₅ | H | F |
| A-563 | CH₃ | H | OC₂H₅ | H | F |
| A-564 | CF₃ | H | OC₂H₅ | H | F |
| A-565 | OCH₃ | H | OC₂H₅ | H | F |
| A-566 | OC₂H₅ | H | OC₂H₅ | H | F |
| A-567 | OCF₃ | H | OC₂H₅ | H | F |
| A-568 | F | H | OCF₃ | H | F |
| A-569 | Cl | H | OCF₃ | H | F |
| A-570 | CH₃ | H | OCF₃ | H | F |
| A-571 | CF₃ | H | OCF₃ | H | F |
| A-572 | OCH₃ | H | OCF₃ | H | F |
| A-573 | OC₂H₅ | H | OCF₃ | H | F |
| A-574 | OCF₃ | H | OCF₃ | H | F |
| A-575 | F | H | F | H | Cl |
| A-576 | Cl | H | F | H | Cl |
| A-577 | CH₃ | H | F | H | Cl |
| A-578 | CF₃ | H | F | H | Cl |
| A-579 | OCH₃ | H | F | H | Cl |
| A-580 | OC₂H₅ | H | F | H | Cl |
| A-581 | OCF₃ | H | F | H | Cl |
| A-582 | F | H | Cl | H | Cl |
| A-583 | Cl | H | Cl | H | Cl |
| A-584 | CH₃ | H | Cl | H | Cl |
| A-585 | CF₃ | H | Cl | H | Cl |
| A-586 | OCH₃ | H | Cl | H | Cl |
| A-587 | OC₂H₅ | H | Cl | H | Cl |
| A-588 | OCF₃ | H | Cl | H | Cl |
| A-589 | F | H | CH₃ | H | Cl |
| A-590 | Cl | H | CH₃ | H | Cl |
| A-591 | CH₃ | H | CH₃ | H | Cl |
| A-592 | CF₃ | H | CH₃ | H | Cl |
| A-593 | OCH₃ | H | CH₃ | H | Cl |
| A-594 | OC₂H₅ | H | CH₃ | H | Cl |
| A-595 | OCF₃ | H | CH₃ | H | Cl |
| A-596 | F | H | CF₃ | H | Cl |
| A-597 | Cl | H | CF₃ | H | Cl |
| A-598 | CH₃ | H | CF₃ | H | Cl |
| A-599 | CF₃ | H | CF₃ | H | Cl |
| A-600 | OCH₃ | H | CF₃ | H | Cl |
| A-601 | OC₂H₅ | H | CF₃ | H | Cl |
| A-602 | OCF₃ | H | CF₃ | H | Cl |
| A-603 | F | H | OCH₃ | H | Cl |
| A-604 | Cl | H | OCH₃ | H | Cl |
| A-605 | CH₃ | H | OCH₃ | H | Cl |
| A-606 | CF₃ | H | OCH₃ | H | Cl |
| A-607 | OCH₃ | H | OCH₃ | H | Cl |
| A-608 | OC₂H₅ | H | OCH₃ | H | Cl |
| A-609 | OCF₃ | H | OCH₃ | H | Cl |
| A-610 | F | H | OC₂H₅ | H | Cl |
| A-611 | Cl | H | OC₂H₅ | H | Cl |
| A-612 | CH₃ | H | OC₂H₅ | H | Cl |
| A-613 | CF₃ | H | OC₂H₅ | H | Cl |
| A-614 | OCH₃ | H | OC₂H₅ | H | Cl |
| A-615 | OC₂H₅ | H | OC₂H₅ | H | Cl |
| A-616 | OCF₃ | H | OC₂H₅ | H | Cl |
| A-617 | F | H | OCF₃ | H | Cl |
| A-618 | Cl | H | OCF₃ | H | Cl |
| A-619 | CH₃ | H | OCF₃ | H | Cl |
| A-620 | CF₃ | H | OCF₃ | H | Cl |
| A-621 | OCH₃ | H | OCF₃ | H | Cl |
| A-622 | OC₂H₅ | H | OCF₃ | H | Cl |
| A-623 | OCF₃ | H | OCF₃ | H | Cl |
| A-624 | F | H | F | H | CH₃ |
| A-625 | Cl | H | F | H | CH₃ |
| A-626 | CH₃ | H | F | H | CH₃ |
| A-627 | CF₃ | H | F | H | CH₃ |
| A-628 | OCH₃ | H | F | H | CH₃ |
| A-629 | OC₂H₅ | H | F | H | CH₃ |
| A-630 | OCF₃ | H | F | H | CH₃ |
| A-631 | F | H | Cl | H | CH₃ |
| A-632 | Cl | H | Cl | H | CH₃ |
| A-633 | CH₃ | H | Cl | H | CH₃ |
| A-634 | CF₃ | H | Cl | H | CH₃ |
| A-635 | OCH₃ | H | Cl | H | CH₃ |
| A-636 | OC₂H₅ | H | Cl | H | CH₃ |
| A-637 | OCF₃ | H | Cl | H | CH₃ |
| A-638 | F | H | CH₃ | H | CH₃ |
| A-639 | Cl | H | CH₃ | H | CH₃ |
| A-640 | CH₃ | H | CH₃ | H | CH₃ |
| A-641 | CF₃ | H | CH₃ | H | CH₃ |
| A-642 | OCH₃ | H | CH₃ | H | CH₃ |
| A-643 | OC₂H₅ | H | CH₃ | H | CH₃ |
| A-644 | OCF₃ | H | CH₃ | H | CH₃ |
| A-645 | F | H | CF₃ | H | CH₃ |
| A-646 | Cl | H | CF₃ | H | CH₃ |
| A-647 | CH₃ | H | CF₃ | H | CH₃ |
| A-648 | CF₃ | H | CF₃ | H | CH₃ |
| A-649 | OCH₃ | H | CF₃ | H | CH₃ |
| A-650 | OC₂H₅ | H | CF₃ | H | CH₃ |
| A-651 | OCF₃ | H | CF₃ | H | CH₃ |
| A-652 | F | H | OCH₃ | H | CH₃ |
| A-653 | Cl | H | OCH₃ | H | CH₃ |
| A-654 | CH₃ | H | OCH₃ | H | CH₃ |
| A-655 | CF₃ | H | OCH₃ | H | CH₃ |
| A-656 | OCH₃ | H | OCH₃ | H | CH₃ |
| A-657 | OC₂H₅ | H | OCH₃ | H | CH₃ |
| A-658 | OCF₃ | H | OCH₃ | H | CH₃ |
| A-659 | F | H | OC₂H₅ | H | CH₃ |
| A-660 | Cl | H | OC₂H₅ | H | CH₃ |
| A-661 | CH₃ | H | OC₂H₅ | H | CH₃ |
| A-662 | CF₃ | H | OC₂H₅ | H | CH₃ |
| A-663 | OCH₃ | H | OC₂H₅ | H | CH₃ |
| A-664 | OC₂H₅ | H | OC₂H₅ | H | CH₃ |
| A-665 | OCF₃ | H | OC₂H₅ | H | CH₃ |
| A-666 | F | H | OCF₃ | H | CH₃ |
| A-667 | Cl | H | OCF₃ | H | CH₃ |
| A-668 | CH₃ | H | OCF₃ | H | CH₃ |
| A-669 | CF₃ | H | OCF₃ | H | CH₃ |
| A-670 | OCH₃ | H | OCF₃ | H | CH₃ |
| A-671 | OC₂H₅ | H | OCF₃ | H | CH₃ |
| A-672 | OCF₃ | H | OCF₃ | H | CH₃ |
| A-673 | F | H | F | H | CF₃ |
| A-674 | Cl | H | F | H | CF₃ |
| A-675 | CH₃ | H | F | H | CF₃ |
| A-676 | CF₃ | H | F | H | CF₃ |
| A-677 | OCH₃ | H | F | H | CF₃ |
| A-678 | OC₂H₅ | H | F | H | CF₃ |
| A-679 | OCF₃ | H | F | H | CF₃ |
| A-680 | F | H | Cl | H | CF₃ |
| A-681 | Cl | H | Cl | H | CF₃ |
| A-682 | CH₃ | H | Cl | H | CF₃ |
| A-683 | CF₃ | H | Cl | H | CF₃ |
| A-684 | OCH₃ | H | Cl | H | CF₃ |
| A-685 | OC₂H₅ | H | Cl | H | CF₃ |
| A-686 | OCF₃ | H | Cl | H | CF₃ |
| A-687 | F | H | CH₃ | H | CF₃ |
| A-688 | Cl | H | CH₃ | H | CF₃ |
| A-689 | CH₃ | H | CH₃ | H | CF₃ |
| A-690 | CF₃ | H | CH₃ | H | CF₃ |
| A-691 | OCH₃ | H | CH₃ | H | CF₃ |
| A-692 | OC₂H₅ | H | CH₃ | H | CF₃ |
| A-693 | OCF₃ | H | CH₃ | H | CF₃ |
| A-694 | F | H | CF₃ | H | CF₃ |
| A-695 | Cl | H | CF₃ | H | CF₃ |
| A-696 | CH₃ | H | CF₃ | H | CF₃ |
| A-697 | CF₃ | H | CF₃ | H | CF₃ |
| A-698 | OCH₃ | H | CF₃ | H | CF₃ |
| A-699 | OC₂H₅ | H | CF₃ | H | CF₃ |
| A-700 | OCF₃ | H | CF₃ | H | CF₃ |
| A-701 | F | H | OCH₃ | H | CF₃ |
| A-702 | Cl | H | OCH₃ | H | CF₃ |
| A-703 | CH₃ | H | OCH₃ | H | CF₃ |
| A-704 | CF₃ | H | OCH₃ | H | CF₃ |
| A-705 | OCH₃ | H | OCH₃ | H | CF₃ |
| A-706 | OC₂H₅ | H | OCH₃ | H | CF₃ |
| A-707 | OCF₃ | H | OCH₃ | H | CF₃ |
| A-708 | F | H | OC₂H₅ | H | CF₃ |
| A-709 | Cl | H | OC₂H₅ | H | CF₃ |
| A-710 | CH₃ | H | OC₂H₅ | H | CF₃ |
| A-711 | CF₃ | H | OC₂H₅ | H | CF₃ |
| A-712 | OCH₃ | H | OC₂H₅ | H | CF₃ |
| A-713 | OC₂H₅ | H | OC₂H₅ | H | CF₃ |
| A-714 | OCF₃ | H | OC₂H₅ | H | CF₃ |
| A-715 | F | H | OCF₃ | H | CF₃ |
| A-716 | Cl | H | OCF₃ | H | CF₃ |
| A-717 | CH₃ | H | OCF₃ | H | CF₃ |
| A-718 | CF₃ | H | OCF₃ | H | CF₃ |
| A-719 | OCH₃ | H | OCF₃ | H | CF₃ |
| A-720 | OC₂H₅ | H | OCF₃ | H | CF₃ |
| A-721 | OCF₃ | H | OCF₃ | H | CF₃ |
| A-722 | F | H | F | H | OCH₃ |
| A-723 | Cl | H | F | H | OCH₃ |
| A-724 | CH₃ | H | F | H | OCH₃ |
| A-725 | CF₃ | H | F | H | OCH₃ |
| A-726 | OCH₃ | H | F | H | OCH₃ |
| A-727 | OC₂H₅ | H | F | H | OCH₃ |
| A-728 | OCF₃ | H | F | H | OCH₃ |
| A-729 | F | H | Cl | H | OCH₃ |
| A-730 | Cl | H | Cl | H | OCH₃ |
| A-731 | CH₃ | H | Cl | H | OCH₃ |
| A-732 | CF₃ | H | Cl | H | OCH₃ |
| A-733 | OCH₃ | H | Cl | H | OCH₃ |
| A-734 | OC₂H₅ | H | Cl | H | OCH₃ |
| A-735 | OCF₃ | H | Cl | H | OCH₃ |
| A-736 | F | H | CH₃ | H | OCH₃ |
| A-737 | Cl | H | CH₃ | H | OCH₃ |
| A-738 | CH₃ | H | CH₃ | H | OCH₃ |
| A-739 | CF₃ | H | CH₃ | H | OCH₃ |
| A-740 | OCH₃ | H | CH₃ | H | OCH₃ |
| A-741 | OC₂H₅ | H | CH₃ | H | OCH₃ |
| A-742 | OCF₃ | H | CH₃ | H | OCH₃ |
| A-743 | F | H | CF₃ | H | OCH₃ |
| A-744 | Cl | H | CF₃ | H | OCH₃ |
| A-745 | CH₃ | H | CF₃ | H | OCH₃ |
| A-746 | CF₃ | H | CF₃ | H | OCH₃ |
| A-747 | OCH₃ | H | CF₃ | H | OCH₃ |
| A-748 | OC₂H₅ | H | CF₃ | H | OCH₃ |
| A-749 | OCF₃ | H | CF₃ | H | OCH₃ |
| A-750 | F | H | OCH₃ | H | OCH₃ |
| A-751 | Cl | H | OCH₃ | H | OCH₃ |
| A-752 | CH₃ | H | OCH₃ | H | OCH₃ |
| A-753 | CF₃ | H | OCH₃ | H | OCH₃ |
| A-754 | OCH₃ | H | OCH₃ | H | OCH₃ |
| A-755 | OC₂H₅ | H | OCH₃ | H | OCH₃ |
| A-756 | OCF₃ | H | OCH₃ | H | OCH₃ |
| A-757 | F | H | OC₂H₅ | H | OCH₃ |
| A-758 | Cl | H | OC₂H₅ | H | OCH₃ |
| A-759 | CH₃ | H | OC₂H₅ | H | OCH₃ |
| A-760 | CF₃ | H | OC₂H₅ | H | OCH₃ |
| A-761 | OCH₃ | H | OC₂H₅ | H | OCH₃ |
| A-762 | OC₂H₅ | H | OC₂H₅ | H | OCH₃ |
| A-763 | OCF₃ | H | OC₂H₅ | H | OCH₃ |
| A-764 | F | H | OCF₃ | H | OCH₃ |
| A-765 | Cl | H | OCF₃ | H | OCH₃ |
| A-766 | CH₃ | H | OCF₃ | H | OCH₃ |
| A-767 | CF₃ | H | OCF₃ | H | OCH₃ |
| A-768 | OCH₃ | H | OCF₃ | H | OCH₃ |
| A-769 | OC₂H₅ | H | OCF₃ | H | OCH₃ |
| A-770 | OCF₃ | H | OCF₃ | H | OCH₃ |
| A-771 | F | H | F | H | OC₂H₅ |
| A-772 | Cl | H | F | H | OC₂H₅ |
| A-773 | CH₃ | H | F | H | OC₂H₅ |
| A-774 | CF₃ | H | F | H | OC₂H₅ |
| A-775 | OCH₃ | H | F | H | OC₂H₅ |
| A-776 | OC₂H₅ | H | F | H | OC₂H₅ |
| A-777 | OCF₃ | H | F | H | OC₂H₅ |
| A-778 | F | H | Cl | H | OC₂H₅ |
| A-779 | Cl | H | Cl | H | OC₂H₅ |
| A-780 | CH₃ | H | Cl | H | OC₂H₅ |
| A-781 | CF₃ | H | Cl | H | OC₂H₅ |
| A-782 | OCH₃ | H | Cl | H | OC₂H₅ |
| A-783 | OC₂H₅ | H | Cl | H | OC₂H₅ |
| A-784 | OCF₃ | H | Cl | H | OC₂H₅ |
| A-785 | F | H | CH₃ | H | OC₂H₅ |
| A-786 | Cl | H | CH₃ | H | OC₂H₅ |
| A-787 | CH₃ | H | CH₃ | H | OC₂H₅ |
| A-788 | CF₃ | H | CH₃ | H | OC₂H₅ |
| A-789 | OCH₃ | H | CH₃ | H | OC₂H₅ |
| A-790 | OC₂H₅ | H | CH₃ | H | OC₂H₅ |
| A-791 | OCF₃ | H | CH₃ | H | OC₂H₅ |
| A-792 | F | H | CF₃ | H | OC₂H₅ |
| A-793 | Cl | H | CF₃ | H | OC₂H₅ |
| A-794 | CH₃ | H | CF₃ | H | OC₂H₅ |
| A-795 | CF₃ | H | CF₃ | H | OC₂H₅ |
| A-796 | OCH₃ | H | CF₃ | H | OC₂H₅ |
| A-797 | OC₂H₅ | H | CF₃ | H | OC₂H₅ |
| A-798 | OCF₃ | H | CF₃ | H | OC₂H₅ |
| A-799 | F | H | OCH₃ | H | OC₂H₅ |
| A-800 | Cl | H | OCH₃ | H | OC₂H₅ |
| A-801 | CH₃ | H | OCH₃ | H | OC₂H₅ |
| A-802 | CF₃ | H | OCH₃ | H | OC₂H₅ |
| A-803 | OCH₃ | H | OCH₃ | H | OC₂H₅ |
| A-804 | OC₂H₅ | H | OCH₃ | H | OC₂H₅ |
| A-805 | OCF₃ | H | OCH₃ | H | OC₂H₅ |
| A-806 | F | H | OC₂H₅ | H | OC₂H₅ |
| A-807 | Cl | H | OC₂H₅ | H | OC₂H₅ |
| A-808 | CH₃ | H | OC₂H₅ | H | OC₂H₅ |
| A-809 | CF₃ | H | OC₂H₅ | H | OC₂H₅ |
| A-810 | OCH₃ | H | OC₂H₅ | H | OC₂H₅ |
| A-811 | OC₂H₅ | H | OC₂H₅ | H | OC₂H₅ |
| A-812 | OCF₃ | H | OC₂H₅ | H | OC₂H₅ |
| A-813 | F | H | OCF₃ | H | OC₂H₅ |
| A-814 | Cl | H | OCF₃ | H | OC₂H₅ |
| A-815 | CH₃ | H | OCF₃ | H | OC₂H₅ |
| A-816 | CF₃ | H | OCF₃ | H | OC₂H₅ |
| A-817 | OCH₃ | H | OCF₃ | H | OC₂H₅ |
| A-818 | OC₂H₅ | H | OCF₃ | H | OC₂H₅ |
| A-819 | OCF₃ | H | OCF₃ | H | OC₂H₅ |
| A-820 | F | H | F | H | OCF₃ |
| A-821 | Cl | H | F | H | OCF₃ |
| A-822 | CH₃ | H | F | H | OCF₃ |
| A-823 | CF₃ | H | F | H | OCF₃ |
| A-824 | OCH₃ | H | F | H | OCF₃ |
| A-825 | OC₂H₅ | H | F | H | OCF₃ |
| A-826 | OCF₃ | H | F | H | OCF₃ |
| A-827 | F | H | Cl | H | OCF₃ |
| A-828 | Cl | H | Cl | H | OCF₃ |
| A-829 | CH₃ | H | Cl | H | OCF₃ |
| A-830 | CF₃ | H | Cl | H | OCF₃ |
| A-831 | OCH₃ | H | Cl | H | OCF₃ |
| A-832 | OC₂H₅ | H | Cl | H | OCF₃ |
| A-833 | OCF₃ | H | Cl | H | OCF₃ |
| A-834 | F | H | CH₃ | H | OCF₃ |
| A-835 | Cl | H | CH₃ | H | OCF₃ |
| A-836 | CH₃ | H | CH₃ | H | OCF₃ |
| A-837 | CF₃ | H | CH₃ | H | OCF₃ |
| A-838 | OCH₃ | H | CH₃ | H | OCF₃ |
| A-839 | OC₂H₅ | H | CH₃ | H | OCF₃ |
| A-840 | OCF₃ | H | CH₃ | H | OCF₃ |
| A-841 | F | H | CF₃ | H | OCF₃ |
| A-842 | Cl | H | CF₃ | H | OCF₃ |
| A-843 | CH₃ | H | CF₃ | H | OCF₃ |
| A-844 | CF₃ | H | CF₃ | H | OCF₃ |
| A-845 | OCH₃ | H | CF₃ | H | OCF₃ |
| A-846 | OC₂H₅ | H | CF₃ | H | OCF₃ |
| A-847 | OCF₃ | H | CF₃ | H | OCF₃ |
| A-848 | F | H | OCH₃ | H | OCF₃ |
| A-849 | Cl | H | OCH₃ | H | OCF₃ |
| A-850 | CH₃ | H | OCH₃ | H | OCF₃ |
| A-851 | CF₃ | H | OCH₃ | H | OCF₃ |
| A-852 | OCH₃ | H | OCH₃ | H | OCF₃ |
| A-853 | OC₂H₅ | H | OCH₃ | H | OCF₃ |
| A-854 | OCF₃ | H | OCH₃ | H | OCF₃ |
| A-855 | F | H | OC₂H₅ | H | OCF₃ |
| A-856 | Cl | H | OC₂H₅ | H | OCF₃ |
| A-857 | CH₃ | H | OC₂H₅ | H | OCF₃ |
| A-858 | CF₃ | H | OC₂H₅ | H | OCF₃ |
| A-859 | OCH₃ | H | OC₂H₅ | H | OCF₃ |
| A-860 | OC₂H₅ | H | OC₂H₅ | H | OCF₃ |
| A-861 | OCF₃ | H | OC₂H₅ | H | OCF₃ |
| A-862 | F | H | OCF₃ | H | OCF₃ |
| A-863 | Cl | H | OCF₃ | H | OCF₃ |
| A-864 | CH₃ | H | OCF₃ | H | OCF₃ |
| A-865 | CF₃ | H | OCF₃ | H | OCF₃ |
| A-866 | OCH₃ | H | OCF₃ | H | OCF₃ |
| A-867 | OC₂H₅ | H | OCF₃ | H | OCF₃ |
| A-868 | OCF₃ | H | OCF₃ | H | OCF₃ |
| A-869 | H | F | F | F | H |
| A-870 | H | Cl | F | F | H |
| A-871 | H | CH₃ | F | F | H |
| A-872 | H | CF₃ | F | F | H |
| A-873 | H | OCH₃ | F | F | H |
| A-874 | H | OC₂H₅ | F | F | H |
| A-875 | H | OCF₃ | F | F | H |
| A-876 | H | F | Cl | F | H |
| A-877 | H | Cl | Cl | F | H |
| A-878 | H | CH₃ | Cl | F | H |
| A-879 | H | CF₃ | Cl | F | H |
| A-880 | H | OCH₃ | Cl | F | H |
| A-881 | H | OC₂H₅ | Cl | F | H |
| A-882 | H | OCF₃ | Cl | F | H |
| A-883 | H | F | CH₃ | F | H |
| A-884 | H | Cl | CH₃ | F | H |
| A-885 | H | CH₃ | CH₃ | F | H |
| A-886 | H | CF₃ | CH₃ | F | H |
| A-887 | H | OCH₃ | CH₃ | F | H |
| A-888 | H | OC₂H₅ | CH₃ | F | H |
| A-889 | H | OCF₃ | CH₃ | F | H |
| A-890 | H | F | CF₃ | F | H |
| A-891 | H | Cl | CF₃ | F | H |
| A-892 | H | CH₃ | CF₃ | F | H |
| A-893 | H | CF₃ | CF₃ | F | H |
| A-894 | H | OCH₃ | CF₃ | F | H |
| A-895 | H | OC₂H₅ | CF₃ | F | H |
| A-896 | H | OCF₃ | CF₃ | F | H |
| A-897 | H | F | OCH₃ | F | H |
| A-898 | H | Cl | OCH₃ | F | H |
| A-899 | H | CH₃ | OCH₃ | F | H |
| A-900 | H | CF₃ | OCH₃ | F | H |
| A-901 | H | OCH₃ | OCH₃ | F | H |
| A-902 | H | OC₂H₅ | OCH₃ | F | H |
| A-903 | H | OCF₃ | OCH₃ | F | H |
| A-904 | H | F | OC₂H₅ | F | H |
| A-905 | H | Cl | OC₂H₅ | F | H |
| A-906 | H | CH₃ | OC₂H₅ | F | H |
| A-907 | H | CF₃ | OC₂H₅ | F | H |
| A-908 | H | OCH₃ | OC₂H₅ | F | H |
| A-909 | H | OC₂H₅ | OC₂H₅ | F | H |
| A-910 | H | OCF₃ | OC₂H₅ | F | H |
| A-911 | H | F | OCF₃ | F | H |
| A-912 | H | Cl | OCF₃ | F | H |
| A-913 | H | CH₃ | OCF₃ | F | H |
| A-914 | H | CF₃ | OCF₃ | F | H |
| A-915 | H | OCH₃ | OCF₃ | F | H |
| A-916 | H | OC₂H₅ | OCF₃ | F | H |
| A-917 | H | OCF₃ | OCF₃ | F | H |
| A-918 | H | F | F | Cl | H |
| A-919 | H | Cl | F | Cl | H |
| A-920 | H | CH₃ | F | Cl | H |
| A-921 | H | CF₃ | F | Cl | H |
| A-922 | H | OCH₃ | F | Cl | H |
| A-923 | H | OC₂H₅ | F | Cl | H |
| A-924 | H | OCF₃ | F | Cl | H |
| A-925 | H | F | Cl | Cl | H |
| A-926 | H | Cl | Cl | Cl | H |
| A-927 | H | CH₃ | Cl | Cl | H |
| A-928 | H | CF₃ | Cl | Cl | H |
| A-929 | H | OCH₃ | Cl | Cl | H |
| A-930 | H | OC₂H₅ | Cl | Cl | H |
| A-931 | H | OCF₃ | Cl | Cl | H |
| A-932 | H | F | CH₃ | Cl | H |
| A-933 | H | Cl | CH₃ | Cl | H |
| A-934 | H | CH₃ | CH₃ | Cl | H |
| A-935 | H | CF₃ | CH₃ | Cl | H |
| A-936 | H | OCH₃ | CH₃ | Cl | H |
| A-937 | H | OC₂H₅ | CH₃ | Cl | H |
| A-938 | H | OCF₃ | CH₃ | Cl | H |
| A-939 | H | F | CF₃ | Cl | H |
| A-940 | H | Cl | CF₃ | Cl | H |
| A-941 | H | CH₃ | CF₃ | Cl | H |
| A-942 | H | CF₃ | CF₃ | Cl | H |
| A-943 | H | OCH₃ | CF₃ | Cl | H |
| A-944 | H | OC₂H₅ | CF₃ | Cl | H |
| A-945 | H | OCF₃ | CF₃ | Cl | H |
| A-946 | H | F | OCH₃ | Cl | H |
| A-947 | H | Cl | OCH₃ | Cl | H |
| A-948 | H | CH₃ | OCH₃ | Cl | H |
| A-949 | H | CF₃ | OCH₃ | Cl | H |
| A-950 | H | OCH₃ | OCH₃ | Cl | H |
| A-951 | H | OC₂H₅ | OCH₃ | Cl | H |
| A-952 | H | OCF₃ | OCH₃ | Cl | H |
| A-953 | H | F | OC₂H₅ | Cl | H |
| A-954 | H | Cl | OC₂H₅ | Cl | H |
| A-955 | H | CH₃ | OC₂H₅ | Cl | H |
| A-956 | H | CF₃ | OC₂H₅ | Cl | H |
| A-957 | H | OCH₃ | OC₂H₅ | Cl | H |
| A-958 | H | OC₂H₅ | OC₂H₅ | Cl | H |
| A-959 | H | OCF₃ | OC₂H₅ | Cl | H |
| A-960 | H | F | OCF₃ | Cl | H |
| A-961 | H | Cl | OCF₃ | Cl | H |
| A-962 | H | CH₃ | OCF₃ | Cl | H |
| A-963 | H | CF₃ | OCF₃ | Cl | H |
| A-964 | H | OCH₃ | OCF₃ | Cl | H |
| A-965 | H | OC₂H₅ | OCF₃ | Cl | H |
| A-966 | H | OCF₃ | OCF₃ | Cl | H |
| A-967 | H | F | F | CH₃ | H |
| A-968 | H | Cl | F | CH₃ | H |
| A-969 | H | CH₃ | F | CH₃ | H |
| A-970 | H | CF₃ | F | CH₃ | H |
| A-971 | H | OCH₃ | F | CH₃ | H |
| A-972 | H | OC₂H₅ | F | CH₃ | H |
| A-973 | H | OCF₃ | F | CH₃ | H |
| A-974 | H | F | Cl | CH₃ | H |
| A-975 | H | Cl | Cl | CH₃ | H |
| A-976 | H | CH₃ | Cl | CH₃ | H |
| A-977 | H | CF₃ | Cl | CH₃ | H |
| A-978 | H | OCH₃ | Cl | CH₃ | H |
| A-979 | H | OC₂H₅ | Cl | CH₃ | H |
| A-980 | H | OCF₃ | Cl | CH₃ | H |
| A-981 | H | F | CH₃ | CH₃ | H |
| A-982 | H | Cl | CH₃ | CH₃ | H |
| A-983 | H | CH₃ | CH₃ | CH₃ | H |
| A-984 | H | CF₃ | CH₃ | CH₃ | H |
| A-985 | H | OCH₃ | CH₃ | CH₃ | H |
| A-986 | H | OC₂H₅ | CH₃ | CH₃ | H |
| A-987 | H | OCF₃ | CH₃ | CH₃ | H |
| A-988 | H | F | CF₃ | CH₃ | H |
| A-989 | H | Cl | CF₃ | CH₃ | H |
| A-990 | H | CH₃ | CF₃ | CH₃ | H |
| A-991 | H | CF₃ | CF₃ | CH₃ | H |
| A-992 | H | OCH₃ | CF₃ | CH₃ | H |
| A-993 | H | OC₂H₅ | CF₃ | CH₃ | H |
| A-994 | H | OCF₃ | CF₃ | CH₃ | H |
| A-995 | H | F | OCH₃ | CH₃ | H |
| A-996 | H | Cl | OCH₃ | CH₃ | H |
| A-997 | H | CH₃ | OCH₃ | CH₃ | H |
| A-998 | H | CF₃ | OCH₃ | CH₃ | H |
| A-999 | H | OCH₃ | OCH₃ | CH₃ | H |
| A-1000 | H | OC₂H₅ | OCH₃ | CH₃ | H |
| A-1001 | H | OCF₃ | OCH₃ | CH₃ | H |
| A-1002 | H | F | OC₂H₅ | CH₃ | H |
| A-1003 | H | Cl | OC₂H₅ | CH₃ | H |
| A-1004 | H | CH₃ | OC₂H₅ | CH₃ | H |
| A-1005 | H | CF₃ | OC₂H₅ | CH₃ | H |
| A-1006 | H | OCH₃ | OC₂H₅ | CH₃ | H |
| A-1007 | H | OC₂H₅ | OC₂H₅ | CH₃ | H |
| A-1008 | H | OCF₃ | OC₂H₅ | CH₃ | H |
| A-1009 | H | F | OCF₃ | CH₃ | H |
| A-1010 | H | Cl | OCF₃ | CH₃ | H |
| A-1011 | H | CH₃ | OCF₃ | CH₃ | H |
| A-1012 | H | CF₃ | OCF₃ | CH₃ | H |
| A-1013 | H | OCH₃ | OCF₃ | CH₃ | H |
| A-1014 | H | OC₂H₅ | OCF₃ | CH₃ | H |
| A-1015 | H | OCF₃ | OCF₃ | CH₃ | H |
| A-1016 | H | F | F | CF₃ | H |
| A-1017 | H | Cl | F | CF₃ | H |
| A-1018 | H | CH₃ | F | CF₃ | H |
| A-1019 | H | CF₃ | F | CF₃ | H |
| A-1020 | H | OCH₃ | F | CF₃ | H |
| A-1021 | H | OC₂H₅ | F | CF₃ | H |
| A-1022 | H | OCF₃ | F | CF₃ | H |
| A-1023 | H | F | Cl | CF₃ | H |
| A-1024 | H | Cl | Cl | CF₃ | H |
| A-1025 | H | CH₃ | Cl | CF₃ | H |
| A-1026 | H | CF₃ | Cl | CF₃ | H |
| A-1027 | H | OCH₃ | Cl | CF₃ | H |
| A-1028 | H | OC₂H₅ | Cl | CF₃ | H |
| A-1029 | H | OCF₃ | Cl | CF₃ | H |
| A-1030 | H | F | CH₃ | CF₃ | H |
| A-1031 | H | Cl | CH₃ | CF₃ | H |
| A-1032 | H | CH₃ | CH₃ | CF₃ | H |
| A-1033 | H | CF₃ | CH₃ | CF₃ | H |
| A-1034 | H | OCH₃ | CH₃ | CF₃ | H |
| A-1035 | H | OC₂H₅ | CH₃ | CF₃ | H |
| A-1036 | H | OCF₃ | CH₃ | CF₃ | H |
| A-1037 | H | F | CF₃ | CF₃ | H |
| A-1038 | H | Cl | CF₃ | CF₃ | H |
| A-1039 | H | CH₃ | CF₃ | CF₃ | H |
| A-1040 | H | CF₃ | CF₃ | CF₃ | H |
| A-1041 | H | OCH₃ | CF₃ | CF₃ | H |
| A-1042 | H | OC₂H₅ | CF₃ | CF₃ | H |
| A-1043 | H | OCF₃ | CF₃ | CF₃ | H |
| A-1044 | H | F | OCH₃ | CF₃ | H |
| A-1045 | H | Cl | OCH₃ | CF₃ | H |
| A-1046 | H | CH₃ | OCH₃ | CF₃ | H |
| A-1047 | H | CF₃ | OCH₃ | CF₃ | H |
| A-1048 | H | OCH₃ | OCH₃ | CF₃ | H |
| A-1049 | H | OC₂H₅ | OCH₃ | CF₃ | H |
| A-1050 | H | OCF₃ | OCH₃ | CF₃ | H |
| A-1051 | H | F | OC₂H₅ | CF₃ | H |
| A-1052 | H | Cl | OC₂H₅ | CF₃ | H |
| A-1053 | H | CH₃ | OC₂H₅ | CF₃ | H |
| A-1054 | H | CF₃ | OC₂H₅ | CF₃ | H |
| A-1055 | H | OCH₃ | OC₂H₅ | CF₃ | H |
| A-1056 | H | OC₂H₅ | OC₂H₅ | CF₃ | H |
| A-1057 | H | OCF₃ | OC₂H₅ | CF₃ | H |
| A-1058 | H | F | OCF₃ | CF₃ | H |
| A-1059 | H | Cl | OCF₃ | CF₃ | H |
| A-1060 | H | CH₃ | OCF₃ | CF₃ | H |
| A-1061 | H | CF₃ | OCF₃ | CF₃ | H |
| A-1062 | H | OCH₃ | OCF₃ | CF₃ | H |
| A-1063 | H | OC₂H₅ | OCF₃ | CF₃ | H |
| A-1064 | H | OCF₃ | OCF₃ | CF₃ | H |
| A-1065 | H | F | F | OCH₃ | H |
| A-1066 | H | Cl | F | OCH₃ | H |
| A-1067 | H | CH₃ | F | OCH₃ | H |
| A-1068 | H | CF₃ | F | OCH₃ | H |
| A-1069 | H | OCH₃ | F | OCH₃ | H |
| A-1070 | H | OC₂H₅ | F | OCH₃ | H |
| A-1071 | H | OCF₃ | F | OCH₃ | H |
| A-1072 | H | F | Cl | OCH₃ | H |
| A-1073 | H | Cl | Cl | OCH₃ | H |
| A-1074 | H | CH₃ | Cl | OCH₃ | H |
| A-1075 | H | CF₃ | Cl | OCH₃ | H |
| A-1076 | H | OCH₃ | Cl | OCH₃ | H |
| A-1077 | H | OC₂H₅ | Cl | OCH₃ | H |
| A-1078 | H | OCF₃ | Cl | OCH₃ | H |
| A-1079 | H | F | CH₃ | OCH₃ | H |
| A-1080 | H | Cl | CH₃ | OCH₃ | H |
| A-1081 | H | CH₃ | CH₃ | OCH₃ | H |
| A-1082 | H | CF₃ | CH₃ | OCH₃ | H |
| A-1083 | H | OCH₃ | CH₃ | OCH₃ | H |
| A-1084 | H | OC₂H₅ | CH₃ | OCH₃ | H |
| A-1085 | H | OCF₃ | CH₃ | OCH₃ | H |
| A-1086 | H | F | CF₃ | OCH₃ | H |
| A-1087 | H | Cl | CF₃ | OCH₃ | H |
| A-1088 | H | CH₃ | CF₃ | OCH₃ | H |
| A-1089 | H | CF₃ | CF₃ | OCH₃ | H |
| A-1090 | H | OCH₃ | CF₃ | OCH₃ | H |
| A-1091 | H | OC₂H₅ | CF₃ | OCH₃ | H |
| A-1092 | H | OCF₃ | CF₃ | OCH₃ | H |
| A-1093 | H | F | OCH₃ | OCH₃ | H |
| A-1094 | H | Cl | OCH₃ | OCH₃ | H |
| A-1095 | H | CH₃ | OCH₃ | OCH₃ | H |
| A-1096 | H | CF₃ | OCH₃ | OCH₃ | H |
| A-1097 | H | OCH₃ | OCH₃ | OCH₃ | H |
| A-1098 | H | OC₂H₅ | OCH₃ | OCH₃ | H |
| A-1099 | H | OCF₃ | OCH₃ | OCH₃ | H |
| A-1100 | H | F | OC₂H₅ | OCH₃ | H |
| A-1101 | H | Cl | OC₂H₅ | OCH₃ | H |
| A-1102 | H | CH₃ | OC₂H₅ | OCH₃ | H |
| A-1103 | H | CF₃ | OC₂H₅ | OCH₃ | H |
| A-1104 | H | OCH₃ | OC₂H₅ | OCH₃ | H |
| A-1105 | H | OC₂H₅ | OC₂H₅ | OCH₃ | H |
| A-1106 | H | OCF₃ | OC₂H₅ | OCH₃ | H |
| A-1107 | H | F | OCF₃ | OCH₃ | H |
| A-1108 | H | Cl | OCF₃ | OCH₃ | H |
| A-1109 | H | CH₃ | OCF₃ | OCH₃ | H |
| A-1110 | H | CF₃ | OCF₃ | OCH₃ | H |
| A-1111 | H | OCH₃ | OCF₃ | OCH₃ | H |
| A-1112 | H | OC₂H₅ | OCF₃ | OCH₃ | H |
| A-1113 | H | OCF₃ | OCF₃ | OCH₃ | H |
| A-1114 | H | F | F | OC₂H₅ | H |
| A-1115 | H | Cl | F | OC₂H₅ | H |
| A-1116 | H | CH₃ | F | OC₂H₅ | H |
| A-1117 | H | CF₃ | F | OC₂H₅ | H |
| A-1118 | H | OCH₃ | F | OC₂H₅ | H |
| A-1119 | H | OC₂H₅ | F | OC₂H₅ | H |
| A-1120 | H | OCF₃ | F | OC₂H₅ | H |
| A-1121 | H | F | Cl | OC₂H₅ | H |
| A-1122 | H | Cl | Cl | OC₂H₅ | H |
| A-1123 | H | CH₃ | Cl | OC₂H₅ | H |
| A-1124 | H | CF₃ | Cl | OC₂H₅ | H |
| A-1125 | H | OCH₃ | Cl | OC₂H₅ | H |
| A-1126 | H | OC₂H₅ | Cl | OC₂H₅ | H |
| A-1127 | H | OCF₃ | Cl | OC₂H₅ | H |
| A-1128 | H | F | CH₃ | OC₂H₅ | H |
| A-1129 | H | Cl | CH₃ | OC₂H₅ | H |
| A-1130 | H | CH₃ | CH₃ | OC₂H₅ | H |
| A-1131 | H | CF₃ | CH₃ | OC₂H₅ | H |
| A-1132 | H | OCH₃ | CH₃ | OC₂H₅ | H |
| A-1133 | H | OC₂H₅ | CH₃ | OC₂H₅ | H |
| A-1134 | H | OCF₃ | CH₃ | OC₂H₅ | H |
| A-1135 | H | F | CF₃ | OC₂H₅ | H |
| A-1136 | H | Cl | CF₃ | OC₂H₅ | H |
| A-1137 | H | CH₃ | CF₃ | OC₂H₅ | H |
| A-1138 | H | CF₃ | CF₃ | OC₂H₅ | H |
| A-1139 | H | OCH₃ | CF₃ | OC₂H₅ | H |
| A-1140 | H | OC₂H₅ | CF₃ | OC₂H₅ | H |
| A-1141 | H | OCF₃ | CF₃ | OC₂H₅ | H |
| A-1142 | H | F | OCH₃ | OC₂H₅ | H |
| A-1143 | H | Cl | OCH₃ | OC₂H₅ | H |
| A-1144 | H | CH₃ | OCH₃ | OC₂H₅ | H |
| A-1145 | H | CF₃ | OCH₃ | OC₂H₅ | H |
| A-1146 | H | OCH₃ | OCH₃ | OC₂H₅ | H |
| A-1147 | H | OC₂H₅ | OCH₃ | OC₂H₅ | H |
| A-1148 | H | OCF₃ | OCH₃ | OC₂H₅ | H |
| A-1149 | H | F | OC₂H₅ | OC₂H₅ | H |
| A-1150 | H | Cl | OC₂H₅ | OC₂H₅ | H |
| A-1151 | H | CH₃ | OC₂H₅ | OC₂H₅ | H |
| A-1152 | H | CF₃ | OC₂H₅ | OC₂H₅ | H |
| A-1153 | H | OCH₃ | OC₂H₅ | OC₂H₅ | H |
| A-1154 | H | OC₂H₅ | OC₂H₅ | OC₂H₅ | H |
| A-1155 | H | OCF₃ | OC₂H₅ | OC₂H₅ | H |
| A-1156 | H | F | OCF₃ | OC₂H₅ | H |
| A-1157 | H | Cl | OCF₃ | OC₂H₅ | H |
| A-1158 | H | CH₃ | OCF₃ | OC₂H₅ | H |
| A-1159 | H | CF₃ | OCF₃ | OC₂H₅ | H |
| A-1160 | H | OCH₃ | OCF₃ | OC₂H₅ | H |
| A-1161 | H | OC₂H₅ | OCF₃ | OC₂H₅ | H |
| A-1162 | H | OCF₃ | OCF₃ | OC₂H₅ | H |
| A-1163 | H | F | F | OCF₃ | H |
| A-1164 | H | Cl | F | OCF₃ | H |
| A-1165 | H | CH₃ | F | OCF₃ | H |
| A-1166 | H | CF₃ | F | OCF₃ | H |
| A-1167 | H | OCH₃ | F | OCF₃ | H |
| A-1168 | H | OC₂H₅ | F | OCF₃ | H |
| A-1169 | H | OCF₃ | F | OCF₃ | H |
| A-1170 | H | F | Cl | OCF₃ | H |
| A-1171 | H | Cl | Cl | OCF₃ | H |
| A-1172 | H | CH₃ | Cl | OCF₃ | H |
| A-1173 | H | CF₃ | Cl | OCF₃ | H |
| A-1174 | H | OCH₃ | Cl | OCF₃ | H |
| A-1175 | H | OC₂H₅ | Cl | OCF₃ | H |
| A-1176 | H | OCF₃ | Cl | OCF₃ | H |
| A-1177 | H | F | CH₃ | OCF₃ | H |
| A-1178 | H | Cl | CH₃ | OCF₃ | H |
| A-1179 | H | CH₃ | CH₃ | OCF₃ | H |
| A-1180 | H | CF₃ | CH₃ | OCF₃ | H |
| A-1181 | H | OCH₃ | CH₃ | OCF₃ | H |
| A-1182 | H | OC₂H₅ | CH₃ | OCF₃ | H |
| A-1183 | H | OCF₃ | CH₃ | OCF₃ | H |
| A-1184 | H | F | CF₃ | OCF₃ | H |
| A-1185 | H | Cl | CF₃ | OCF₃ | H |
| A-1186 | H | CH₃ | CF₃ | OCF₃ | H |
| A-1187 | H | CF₃ | CF₃ | OCF₃ | H |
| A-1188 | H | OCH₃ | CF₃ | OCF₃ | H |
| A-1189 | H | OC₂H₅ | CF₃ | OCF₃ | H |
| A-1190 | H | OCF₃ | CF₃ | OCF₃ | H |
| A-1191 | H | F | OCH₃ | OCF₃ | H |
| A-1192 | H | Cl | OCH₃ | OCF₃ | H |
| A-1193 | H | CH₃ | OCH₃ | OCF₃ | H |
| A-1194 | H | CF₃ | OCH₃ | OCF₃ | H |
| A-1195 | H | OCH₃ | OCH₃ | OCF₃ | H |
| A-1196 | H | OC₂H₅ | OCH₃ | OCF₃ | H |
| A-1197 | H | OCF₃ | OCH₃ | OCF₃ | H |
| A-1198 | H | F | OC₂H₅ | OCF₃ | H |
| A-1199 | H | Cl | OC₂H₅ | OCF₃ | H |
| A-1200 | H | CH₃ | OC₂H₅ | OCF₃ | H |
| A-1201 | H | CF₃ | OC₂H₅ | OCF₃ | H |
| A-1202 | H | OCH₃ | OC₂H₅ | OCF₃ | H |
| A-1203 | H | OC₂H₅ | OC₂H₅ | OCF₃ | H |
| A-1204 | H | OCF₃ | OC₂H₅ | OCF₃ | H |
| A-1205 | H | F | OCF₃ | OCF₃ | H |
| A-1206 | H | Cl | OCF₃ | OCF₃ | H |
| A-1207 | H | CH₃ | OCF₃ | OCF₃ | H |
| A-1208 | H | CF₃ | OCF₃ | OCF₃ | H |
| A-1209 | H | OCH₃ | OCF₃ | OCF₃ | H |
| A-1210 | H | OC₂H₅ | OCF₃ | OCF₃ | H |
| A-1211 | H | OCF₃ | OCF₃ | OCF₃ | H |

The compounds of the formula I used according to the invention and compounds according to the invention and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds of the formula I used according to the invention and compounds according to the invention and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds of the formula I used according to the invention and compounds according to the invention and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds of the formula I used according to the invention and compounds according to the invention and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://www.bio.org/speeches/pubs/er/agri_products.asp). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxyl-phenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-I⁶ oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pretoxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656,

EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f.

WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds of the formula I used according to the invention and compounds according to the invention and compositions thereof, respectively, are particularly suitable for controlling the following plant diseases:
*Albugo* spp. (white rust) on ornamentals, vegetables (e. g. *A*. *candida*) and sunflowers (e. g. *A*. *tragopogonis*); *Alternaria* spp. (Alternaria leaf spot) on vegetables, rape (*A*. *brassicola* or *brassicae),* sugar beets (*A*. *tenuis*), fruits, rice, soybeans, potatoes (e. g. *A*. *solani* or *A*. *alternata),* tomatoes (e. g. *A*. *solani* or *A*. *alternata*) and wheat; *Aphanomyces* spp. on sugar beets and vegetables; *Ascochyta* spp. on cereals and vegetables, e. g. *A*. *tritici* (anthracnose) on wheat and *A*. *hordei* on barley; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e.g. B. *oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (*Dutch* elm disease) on elms; *Cercospora* spp. (Cercospora leaf spots) on corn (e.g. Gray leaf spot: *C. zeae-maydis*), rice, sugar beets (e. g. *C. beticola*), sugar cane, vegetables, coffee, soybeans (e. g. *C. sojina* or *C*. *kikuchii*) and rice; *Cladosporium* spp. on tomatoes (e. g. *C. fulvum*: leaf mold) and cereals, e. g. *C*. *herbarum* (black ear) on wheat; *Claviceps purpurea* (ergot) on cereals; *Cochliobolus* (anamorph: *Helminthosporium* of *Bipolaris*) spp. (leaf spots) on corn (*C. carbonum*), cereals (e. g. *C. sativus,* anamorph: *B. sorokiniana*) and rice (e. g. *C. miyabeanus,* anamorph: *H. oryzae*); *Colletotrichum* (teleomorph: *Glomerella*) spp. (anthracnose) on cotton (e. g. *C. gossypii*), corn (e. g. *C. graminicola:* Anthracnose stalk rot), soft fruits, potatoes (e. g. *C*. *coccodes:* black dot), beans (e. g. *C. lindemuthianum*) and soybeans (e. g. *C. truncatum* or *C*. *gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C*. *liriodendri,* teleomorph: *Neonectria liriodendri*: Black Foot Disease) and ornamentals; *Dematophora* (teleomorph: *Rosellinia*) necatri⁶ (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. D. *teres,* net blotch) and wheat (e. g. *D*. *tritici-repentis:* tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa; Elsinoe* spp. on pome fruits (*E. pyri*), soft fruits (*E. veneta*: anthracnose) and vines (*E*. *ampelina:* anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi),* such as cucurbits (e. g. *E. cichoracearum*), cabbages, rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* on soybeans and *F*. *verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G*. *gossypii* on cotton; Grainstaining comple⁶ on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatri⁶*(coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M*. *fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina*) and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P*. *tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P*. *phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*), potatoes and tomatoes (e. g. *P. infestans:* late blight) and broad-leaved trees (e. g. *P*. *ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P. cubensis* on cucurbits or *P*. *humili* on hop; *Pseudopezicula tracheiphila* (red fire disease or 'rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or *P*. *teres* (net blotch) on barley; *Pyricularia* spp., e. g. *P. oryzae* (teleomorph: *Magnaporthe grisea,* rice blast) on rice and *P*. *grisea* on turf and cereals; *Pythium* spp. (damping-off) on turf, rice, corn, wheat, cotton, rape, sunflowers, soybeans, sugar beets, vegetables and various other plants (e. g. *P*. *ultimum* or *P*. *aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S*. *attenuatum* (sheath rot) on rice; *Sclerotinia* spp. (stem rot or white mold) on vegetables and field crops, such as rape, sunflowers (e. g. *S. sclerotiorum*) and soybeans (e. g. *S*. *rolfsii* or *S*. *sclerotiorum*); *Septoria* spp. on various plants, e. g. *S. glycines* (brown spot) on soybeans, *S. tritici* (Septoria blotch) on wheat and S. (syn. *Stagonospora*) nodorum (Stagonospora blotch) on cereals; *Uncinula* (*syn. Erysiphe*) *necator* (powdery mildew, anamorph: *Oidium tuckeri*) on vines; *Setospaeria* spp. (leaf blight) on corn (e. g. *S*. *turcicum, syn. Helminthosporium turcicum*) and turf; *Sphacelotheca* spp. (smut) on corn, (e. g. *S. reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria [syn. Phaeosphaeria] nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (*syn. Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus, syn. U. phaseoli*) and sugar beets (e. g. *U. betae*); *Ustilago* spp. (loose smut) on cereals (e. g. *U. nuda* and *U. avaenae*), corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds of the formula I used according to the invention and compounds according to the invention and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromy*ces spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.*

The compounds of the formula I used according to the invention and compounds according to the invention and compositions thereof, resepectively, may be used for improving the health of a plant. The invention also relates to a method for improving plant health by treating a plant, its propagation material and/or the locus where the plant is growing or is to grow with an effective amount of compounds of the formula I used according to the invention and compounds according to the invention and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I can be present in different crystal modifications whose biological activity may differ. They are likewise subject matter of the present invention.

The compounds of the formula I used according to the invention and compounds according to the invention are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds of the formula I used according to the invention and compounds according to the invention as such or a composition comprising at least one compound of the formula I used according to the invention and compounds according to the invention prophylactically either at or before planting or transplanting.

The invention also relates to agrochemical compositions comprising a solvent or solid carrier and at least one and to the use for controlling harmful fungi.

An agrochemical composition comprises a fungicidally effective amount of a compound of the formula I. The term "effective amount" denotes an amount of the composition or of the compounds of the formula I used according to the invention and compounds according to the invention , which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound of the formula I used according to the invention and compounds according to the inventionused.

The compounds of the formula I used according to the invention and compounds according to the invention , their N-oxides and salts can be converted into customary types of agrochemical compositions, e. g. solutions, emulsions, suspensions, dusts, powders, pastes and granules. The composition type depends on the particular intended purpose; in each case, it should ensure a fine and uniform distribution of the compound according to the invention.

Examples for composition types are suspensions (SC, OD, FS), emulsifiable concentrates (EC), emulsions (EW, EO, ES), pastes, pastilles, wettable powders or dusts (WP, SP, SS, WS, DP, DS) or granules (GR, FG, GG, MG), which can be water-soluble or wettable, as well as gel formulations for the treatment of plant propagation materials such as seeds (GF).

Usually the composition types (e. g. SC, OD, FS, EC, WG, SG, WP, SP, SS, WS, GF) are employed diluted. Composition types such as DP, DS, GR, FG, GG and MG are usually used undiluted.

The compositions are prepared in a known manner (cf. US 3,060,084, EP-A 707 445 (for liquid concentrates), Browning: "Agglomeration", Chemical Engineering, Dec. 4, 1967, 147-48, Perry's Chemical Engineer's Handbook, 4th Ed., McGraw-Hill, New York, 1963, S. 8-57 und ff. WO 91/13546, US 4,172,714, US 4,144,050, US 3,920,442, US 5,180,587, US 5,232,701, US 5,208,030, GB 2,095,558, US 3,299,566, Klingman: Weed Control as a Science (J. Wiley & Sons, New York, 1961), Hance et al.: Weed Control Handbook (8th Ed., Blackwell Scientific, Oxford, 1989) and Mollet, H. and Grubemann, A.: Formulation technology (Wiley VCH Verlag, Weinheim, 2001).

The agrochemical compositions may also comprise auxiliaries which are customary in agrochemical compositions. The auxiliaries used depend on the particular application form and active substance, respectively.

Examples for suitable auxiliaries are solvents, solid carriers, dispersants or emulsifiers (such as further solubilizers, protective colloids, surfactants and adhesion agents), organic and anorganic thickeners, bactericides, anti-freezing agents, anti-foaming agents, if appropriate colorants and tackifiers or binders (e. g. for seed treatment formulations).

Suitable solvents are water, organic solvents such as mineral oil fractions of medium to high boiling point, such as kerosene or diesel oil, furthermore coal tar oils and oils of vegetable or animal origin, aliphatic, cyclic and aromatic hydrocarbons, e. g. toluene, xylene, paraffin, tetrahydronaphthalene, alkylated naphthalenes or their derivatives, alcohols such as methanol, ethanol, propanol, butanol and cyclohexanol, glycols, ketones such as cyclohexanone and gamma-butyrolactone, fatty acid dimethylamides, fatty acids and fatty acid esters and strongly polar solvents, e. g. amines such as N-methylpyrrolidone.

Solid carriers are mineral earths such as silicates, silica gels, talc, kaolins, limestone, lime, chalk, bole, loess, clays, dolomite, diatomaceous earth, calcium sulfate, magnesium sulfate, magnesium oxide, ground synthetic materials, fertilizers, such as, e. g., ammonium sulfate, ammonium phosphate, ammonium nitrate, ureas, and products of vegetable origin, such as cereal meal, tree bark meal, wood meal and nutshell meal, cellulose powders and other solid carriers.

Suitable surfactants (adjuvants, wtters, tackifiers, dispersants or emulsifiers) are alkali metal, alkaline earth metal and ammonium salts of aromatic sulfonic acids, such as ligninsoulfonic acid (Borresperse® types, Borregard, Norway) phenolsulfonic acid, naphthalenesulfonic acid (Morwet® types, Akzo Nobel, U.S.A.), dibutylnaphthalene-sulfonic acid (Nekal® types, BASF, Germany),and fatty acids, alkylsulfonates, alkylarylsulfonates, alkyl sulfates, laurylether sulfates, fatty alcohol sulfates, and sulfated hexa-, hepta- and octadecanolates, sulfated fatty alcohol glycol ethers, furthermore condensates of naphthalene or of naphthalenesulfonic acid with phenol and formaldehyde, polyoxy-ethylene octylphenyl ether, ethoxylated isooctylphenol, octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquors and proteins, denatured proteins, polysaccharides (e. g. methylcellulose), hydrophobically modified starches, polyvinyl alcohols (Mowiol^{®} types, Clariant, Switzerland), polycarboxylates (Sokolan^{®} types, BASF, Germany), polyalkoxylates, polyvinylamines (Lupasol^{®} types, BASF, Germany), polyvinylpyrrolidone and the copolymers therof.

Examples for thickeners (i. e. compounds that impart a modified flowability to compositions, i. e. high viscosity under static conditions and low viscosity during agitation) are polysaccharides and organic and anorganic clays such as Xanthan gum (Kelzan^{®}, CP Kelco, U.S.A.), Rhodopol^{®} 23 (Rhodia, France), Veegum^{®} (R.T. Vanderbilt, U.S.A.) or Attaclay^{®} (Engelhard Corp., NJ, USA).

Bactericides may be added for preservation and stabilization of the composition. Examples for suitable bactericides are those based on dichlorophene and benzylalcohol hemi formal (Proxel^{®} from ICI or Acticide^{®} RS from Thor Chemie and Kathon^{®} MK from Rohm & Haas) and isothiazolinone derivatives such as alkylisothiazolinones and benzisothiazolinones (Acticide^{®} MBS from Thor Chemie).

Examples for suitable anti-freezing agents are ethylene glycol, propylene glycol, urea and glycerin.

Examples for anti-foaming agents are silicone emulsions (such as e. g. Silikon^{®} SRE, Wacker, Germany or Rhodorsil^{®}, Rhodia, France), long chain alcohols, fatty acids, salts of fatty acids, fluoroorganic compounds and mixtures thereof.

Suitable colorants are pigments of low water solubility and water-soluble dyes. Examples to be mentioned und the designations rhodamin B, C. I. pigment red 112, C. I. solvent red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples for tackifiers or binders are polyvinylpyrrolidons, polyvinylacetates, polyvinyl alcohols and cellulose ethers (Tylose®, Shin-Etsu, Japan).

Powders, materials for spreading and dusts can be prepared by mixing or concomitantly grinding the compounds of the formula I used according to the invention and compounds according to the invention and, if appropriate, further active substances, with at least one solid carrier.

Granules, e. g. coated granules, impregnated granules and homogeneous granules, can be prepared by binding the active substances to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, e. g., ammonium sulfate, ammonium phosphate, ammonium nitrate, ureas, and products of vegetable origin, such as cereal meal, tree bark meal, wood meal and nutshell meal, cellulose powders and other solid carriers.

Examples for composition types are:
1. Composition types for dilution with water
   i) Water-soluble concentrates (SL, LS)
      10 parts by weight of a compound of the formula I according to the invention are dissolved in 90 parts by weight of water or in a water-soluble solvent. As an alternative, wetting agents or other auxiliaries are added. The active substance dissolves upon dilution with water. In this way, a composition having a content of 10% by weight of active substance is obtained.
   ii) Dispersible concentrates (DC)
      20 parts by weight of a compound of the formula I used according to the invention and compounds according to the inventionaccording to the invention are dissolved in 70 parts by weight of cyclohexanone with addition of 10 parts by weight of a dispersant, e. g. polyvinylpyrrolidone. Dilution with water gives a dispersion. The active substance content is 20% by weight.
   iii) Emulsifiable concentrates (EC)
      15 parts by weight of a compound of the formula I according to the invention are dissolved in 75 parts by weight of xylene with addition of calcium dodecylbenzenesulfonate and castor oil ethoxylate (in each case 5 parts by weight). Dilution with water gives an emulsion. The composition has an active substance content of 15% by weight.
   iv) Emulsions (EW, EO, ES)
      25 parts by weight of a compound of the formula I according to the invention are dissolved in 35 parts by weight of xylene with addition of calcium dodecylbenzenesulfonate and castor oil ethoxylate (in each case 5 parts by weight). This mixture is introduced into 30 parts by weight of water by means of an emulsifying machine (Ultraturrax) and made into a homogeneous emulsion. Dilution with water gives an emulsion. The composition has an active substance content of 25% by weight.
   v) Suspensions (SC, OD, FS)
      In an agitated ball mill, 20 parts by weight of a compound of the formula I according to the invention are comminuted with addition of 10 parts by weight of dispersants and wetting agents and 70 parts by weight of water or an organic solvent to give a fine active substance suspension. Dilution with water gives a stable suspension of the active substance. The active substance content in the composition is 20% by weight.
   vi) Water-dispersible granules and water-soluble granules (WG, SG)
      50 parts by weight of a compound of the formula I according to the invention are ground finely with addition of 50 parts by weight of dispersants and wetting agents and prepared as water-dispersible or water-soluble granules by means of technical appliances (e. g. extrusion, spray tower, fluidized bed). Dilution with water gives a stable dispersion or solution of the active substance. The composition has an active substance content of 50% by weight.
   vii) Water-dispersible powders and water-soluble powders (WP, SP, SS, WS)
      75 parts by weight of a compound of the formula I according to the invention are ground in a rotor-stator mill with addition of 25 parts by weight of dispersants, wetting agents and silica gel. Dilution with water gives a stable dispersion or solution of the active substance. The active substance content of the composition is 75% by weight.
   viii) Gel (GF)
      In an agitated ball mill, 20 parts by weight of a compound of the formula I according to the invention are comminuted with addition of 10 parts by weight of dispersants, 1 part by weight of a gelling agent wetters and 70 parts by weight of water or of an organic solvent to give a fine suspension of the active substance. Dilution with water gives a stable suspension of the active substance, whereby a composition with 20% (w/w) of active substance is obtained.
2. Composition types to be applied undiluted
   ix) Dustable powders (DP, DS)
      5 parts by weight of a compound of the formula I according to the invention are ground finely and mixed intimately with 95 parts by weight of finely divided kaolin. This gives a dustable composition having an active substance content of 5% by weight.
   x) Granules (GR, FG, GG, MG)
      0.5 parts by weight of a compound of the formula I according to the invention is ground finely and associated with 99.5 parts by weight of carriers. Current methods are extrusion, spray-drying or the fluidized bed. This gives granules to be applied undiluted having an active substance content of 0.5% by weight.
   xi) ULV solutions (UL)
      10 parts by weight of a compound of the formula I according to the invention are dissolved in 90 parts by weight of an organic solvent, e. g. xylene. This gives a composition to be applied undiluted having an active substance content of 10% by weight.

The agrochemical compositions generally comprise between 0.01 and 95%, preferably between 0.1 and 90%, most preferably between 0.5 and 90%, by weight of active substance. The active substances are employed in a purity of from 90% to 100%, preferably from 95% to 100% (according to NMR spectrum).

Water-soluble concentrates (LS), flowable concentrates (FS), powders for dry treatment (DS), water-dispersible powders for slurry treatment (WS), water-soluble powders (SS), emulsions (ES) emulsifiable concentrates (EC) and gels (GF) are usually employed for the purposes of treatment of plant propagation materials, particularly seeds. These compositions can be applied to plant propagation materials, particularly seeds, diluted or undiluted. The compositions in question give, after two-to-tenfold dilution, active substance concentrations of from 0.01 to 60% by weight, preferably from 0.1 to 40% by weight, in the ready-to-use preparations. Application can be carried out before or during sowing. Methods for applying or treating agrochemical compounds and compositions thereof, respectively, on to plant propagation material, especially seeds, are known in the art, and include dressing, coating, pelleting, dusting, soaking and in-furrow application methods of the propagation material. In a preferred embodiment, the compounds or the compositions thereof, respectively, are applied on to the plant propagation material by a method such that germination is not induced, e. g. by seed dressing, pelleting, coating and dusting.

In a preferred embodiment, a suspension-type (FS) composition is used for seed treatment. Typcially, a FS composition may comprise 1-800 g/I of active substance, 1-200 g/I Surfactant, 0 to 200 g/I antifreezing agent, 0 to 400 g/I of binder, 0 to 200 g/I of a pigment and up to 1 liter of a solvent, preferably water.

The active substances can be used as such or in the form of their compositions, e. g. in the form of directly sprayable solutions, powders, suspensions, dispersions, emulsions, oil dispersions, pastes, dustable products, materials for spreading, or granules, by means of spraying, atomizing, dusting, spreading, brushing, immersing or pouring. The application forms depend entirely on the intended purposes; it is intended to ensure in each case the finest possible distribution of the active substances according to the invention.

Aqueous application forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active substance concentrations in the ready-to-use preparations can be varied within relatively wide ranges. In general, they are from 0.0001 to 10%, preferably from 0.001 to 1 % by weight of active substance.

The active substances may also be used successfully in the ultra-low-volume process (ULV), it being possible to apply compositions comprising over 95% by weight of active substance, or even to apply the active substance without additives.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seed) are generally required.

When used in the protection of materials or stored products, the amount of active substance applied depends on the kind of application area and on the desired effect. Amounts customarily applied in the protection of materials are, e. g., 0.001 g to 2 kg, preferably 0.005 g to 1 kg, of active substance per cubic meter of treated material.

Various types of oils, wetters, adjuvants, herbicides, bactericides, other fungicides and/or pesticides may be added to the active substances or the compositions comprising them, if appropriate not until immediately prior to use (tank mix). These agents can be admixed with the compositions according to the invention in a weight ratio of 1:100 to 100:1, preferably 1:10 to 10:1.

Adjuvants which can be used are in particular organic modified polysiloxanes such as Break Thru S 240^{®}; alcohol alkoxylates such as Atplus 245^{®}, Atplus MBA 1303^{®}, Plurafac LF 300^{®} and Lutensol ON 30^{®}; EO/PO block polymers, e. g. Pluronic RPE 2035^{®} and Genapol B^{®}; alcohol ethoxylates such as Lutensol XP 80^{®}; and dioctyl sulfosuccinate sodium such as Leophen RA^{®}.

The compositions according to the invention can, in the use form as fungicides, also be present together with other active substances, e. g. with herbicides, insecticides, growth regulators, fungicides or else with fertilizers, as pre-mi⁶ or, if appropriate, not until immeadiately prior to use (tank mix).

Mixing the compounds of the formula I used according to the invention and compounds according to the invention or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of active substances, in conjunction with which the compounds according to the invention can be used, is intended to illustrate the possible combinations but does not limit them:
A) strobilurins
   - azoxystrobin, coumethoxystrobin, coumoxystrobin, dimoxystrobin, enestroburin, fluoxastrobin, kresoxim-methyl, metominostrobin, orysastrobin, picoxystrobin, pyraclostrobin, pyrametostrobin, pyraoxystrobin, pyribencarb, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxymethyl)-phenyl)-2-methoxyimino-N-methyl-acetamide;
B) carboxamides
   - carboxanilides: benalaxyl, benalaxyl-M, benodanil, bixafen, boscalid, carboxin, fenfuram, fenhexamid, flutolanil, fluxapyroxad, furametpyr, isopyrazam, isotianil, kiralaxyl, mepronil, metalaxyl, metalaxyl-M (mefenoxam), ofurace, oxadixyl, oxycarboxin, penflufen, penthiopyrad, sedaxane, tecloftalam, thifluzamide, tiadinil, 2-amino-4-methyl-thiazole-5-carboxanilide, N-(4'-trifluoromethylthiobiphenyl-2-yl)-3-difluoromethyl-1-methyl-1H-pyrazole-4-carboxamide and N-(2-(1,3,3-trimethylbutyl)-phenyl)-1,3-dimethyl-5-fluoro-1 H-pyrazole-4-carboxamide;
   - carboxylic morpholides: dimethomorph, flumorph, pyrimorph;
   - benzoic acid amides: flumetover, fluopicolide, fluopyram, zoxamide;
   - other carboxamides: carpropamid, dicyclomet, mandiproamid, oxytetracyclin, silthiofam and N-(6-methoxy-pyridin-3-yl) cyclopropanecarboxylic acid amide;
C) azoles
   - triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole;
   - imidazoles: cyazofamid, imazalil, pefurazoate, prochloraz, triflumizol;
   - benzimidazoles: benomyl, carbendazim, fuberidazole, thiabendazole;
   - others: ethaboxam, etridiazole, hymexazole and 2-(4-chloro-phenyl)-N-[4-(3,4-di-methoxy-phenyl)-isoxazol-5-yl]-2-prop-2-ynyloxy-acetamide;
D) heterocyclic compounds
   - pyridines: fluazinam, pyrifenox, 3-[5-(4-chloro-phenyl)-2,3-dimethyl-isoxazolidin-3-yl]-pyridine, 3-[5-(4-methyl-phenyl)-2,3-dimethyl-isoxazolidin-3-yl]-pyridine;
   - pyrimidines: bupirimate, cyprodinil, diflumetorim, fenarimol, ferimzone, mepanipyrim, nitrapyrin, nuarimol, pyrimethanil;
   - piperazines: triforine;
   - pyrroles: fenpiclonil, fludioxonil;
   - morpholines: aldimorph, dodemorph, dodemorph-acetate, fenpropimorph, tridemorph;
   - piperidines: fenpropidin;
   - dicarboximides: fluoroimid, iprodione, procymidone, vinclozolin;
   - non-aromatic 5-membered heterocycles: famoxadone, fenamidone, flutianil, octhilinone, probenazole, 5-amino-2-isopropyl-3-oxo-4-ortho-tolyl-2,3-dihydro-pyrazole-1-carbothioic acid S-allyl ester;
   - others: acibenzolar-S-methyl, ametoctradin, amisulbrom, anilazin, blasticidin-S, captafol, captan, chinomethionat, dazomet, debacarb, diclomezine, difenzoquat, difenzoquat-methylsulfate, fenoxanil, Folpet, oxolinic acid, piperalin, proquinazid, pyroquilon, quinoxyfen, triazoxide, tricyclazole, 2-butoxy-6-iodo-3-propylchromen-4-one, 5-chloro-1-(4,6-dimethoxy-pyrimidin-2-yl)-2-methyl-1H-benzoimidazole and 5-chloro-7-(4-methylpiperidin-1-yl)-6-(2,4,6-trifluorophenyl)-[1,2,4]triazolo[1,5-a]pyrimidine;
E) carbamates
   - thio- and dithiocarbamates: ferbam, mancozeb, maneb, metam, methasulphocarb, metiram, propineb, thiram, zineb, ziram;
   - carbamates: benthiavalicarb, diethofencarb, iprovalicarb, propamocarb, propamocarb hydrochlorid, valifenalate and N-(1-(1-(4-cyano-phenyl)ethanesulfonyl)-but-2-yl) carbamic acid-(4-fluorophenyl) ester;
F) other active substances
   - guanidines: guanidine, dodine, dodine free base, guazatine, guazatine-acetate, iminoctadine, iminoctadine-triacetate, iminoctadine-tris(albesilate);
   - antibiotics: kasugamycin, kasugamycin hydrochloride-hydrate, streptomycin, polyoxine, validamycin A;
   - nitrophenyl derivates: binapacryl, dicloran, dinobuton, dinocap, nitrothal-isopropyl, tecnazen,
      organometal compounds: fentin salts, such as fentin-acetate, fentin chloride or fentin hydroxide;
   - sulfur-containing heterocyclyl compounds: dithianon, isoprothiolane;
   - organophosphorus compounds: edifenphos, fosetyl, fosetyl-aluminum, iprobenfos, phosphorous acid and its salts, pyrazophos, tolclofos-methyl;
   - organochlorine compounds: chlorothalonil, dichlofluanid, dichlorophen, flusulfamide, hexachlorobenzene, pencycuron, pentachlorphenole and its salts, phthalide, quintozene, thiophanate-methyl, tolylfluanid, N-(4-chloro-2-nitro-phenyl)-N-ethyl-4-methyl-benzenesulfonamide;
   - inorganic active substances: Bordeau⁶ mixture, copper acetate, copper hydroxide, copper oxychloride, basic copper sulfate, sulfur;
   - antifungal biocontrol agents, plant bioactivators: *Ampelomyces quisqualis* (e.g. AQ 10^{®} from Intrachem Bio GmbH & Co. KG, Germany), *Aspergillus flavus* (e.g. AFLA-GUARD^{®} from Syngenta, CH), *Aureobasidium pullulans* (e.g. BOTECTOR^{®} from bioferm GmbH, Germany), *Bacillus pumilus* (e.g. NRRL Accession No. B-30087 in SONATA^{®} and BALLAD^{®} Plus from AgraQuest Inc., USA), *Bacillus subtilis* (e.g. isolate NRRL-Nr. B-21661 in RHAPSODY^{®}, SERENADE^{®} MA⁶ and SERENADE^{®} ASO from AgraQuest Inc., USA), *Bacillus subtilis* var. *amyloliquefaciens* FZB24 (e.g. TAE-GRO^{®} from Novozyme Biologicals, Inc., USA), *Candida oleophila* I-82 (e.g. ASPIRE^{®} from Ecogen Inc., USA), *Candida saitoana* (e.g. BIOCURE^{®} (in mixture with lysozyme) and BIOCOAT^{®} from Micro Flo Company, USA (BASF SE) and Arysta), Chitosan (e.g. ARMOUR-ZEN from BotriZen Ltd., NZ), *Clonostachys rosea* f. *catenulata*, also named *Gliocladium catenulatum* (e.g. isolate J1446: PRESTOP^{®} from Verdera, Finland), *Coniothyrium minitans* (e.g. CONTANS^{®} from Prophyta, Germany), *Cryphonectria parasitica* (e.g. *Endothia parasitica* from CNICM, France), *Cryptococcus albidus* (e.g. YIELD PLUS^{®} from Anchor Bio-Technologies, South Africa), *Fusarium oxysporum* (e.g. BIOFOX^{®} from S.I.A.P.A., Italy, FUSACLEAN^{®} from Natural Plant Protection, France), *Metschnikowia fructicola* (e.g. SHEMER^{®} from Agrogreen, Israel), *Microdochium dimerum* (e.g. ANTIBOT^{®} from Agrauxine, France), *Phlebiopsis gigantea* (e.g. ROTSOP^{®} from Verdera, Finland), *Pseudozyma flocculosa* (e.g. SPORODEX^{®} from Plant Products Co. Ltd., Canada), *Pythium oligandrum* DV74 (e.g. POLYVERSUM^{®} from Remeslo SSRO, Biopreparaty, Czech Rep.), *Reynoutria sachlinensis* (e.g. REGALIA^{®} from Marrone BioInnovations, USA), *Talaromyces flavus* V117b (e.g. PROTUS^{®} from Prophyta, Germany), *Trichoderma asperellum* SKT-1 (e.g. ECO-HOPE^{®} from Kumiai Chemical Industry Co., Ltd., Japan), *T. atroviride* LC52 (e.g. SENTINEL^{®} from Agrimm Technologies Ltd, NZ), *T. harzianum* T-22 (e.g. PLANTSHIELD^{®} der Firma BioWorks Inc., USA), *T. harzianum* TH 35 (e.g. ROOT PRO^{®} from Mycontrol Ltd., Israel), *T. harzianum* T-39 (e.g. TRICHODEX^{®} and TRICHODERMA 2000^{®} from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER^{®} WP from Isagro Ricerca, Italy), *T. polysporum* and *T. harzianum* (e.g. BINAB^{®} from BI-NAB Bio-Innovation AB, Sweden), *T. stromaticum* (e.g. TRICOVAB^{®} from C.E.P.L.A.C., Brazil), *T. virens* GL-21 (e.g. SOILGARD^{®} from Certis LLC, USA), *T. viride* (e.g. TRIECO^{®} from Ecosense Labs. (India) Pvt. Ltd., Indien, BIO-CURE^{®} F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN^{®} from Botry-Zen Ltd, NZ);
   - others: biphenyl, bronopol, cyflufenamid, cymoxanil, diphenylamin, metrafenone, pyriofenone, mildiomycin, oxin-copper, prohexadione-calcium, spiroxamine, tebufloquin, tolylfluanid, N-(cyclopropylmethoxyimino-(6-difluoro-methoxy-2,3-difluorophenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, 2-{1-[2-(5-methyl-3-trifluoromethyl-pyrazole-1-yl)-acetyl]-piperidin-4-yl}-thiazole-4-carboxylic acid methyl-(1,2,3,4-tetrahydro-naphthalen-1-yl)-amide, 2-{1-[2-(5-methyl-3-trifluoromethyl-pyrazole-1-yl)-acetyl]-piperidin-4-yl}-thiazole-4-carboxylic acid methyl-(R)-1,2,3,4-tetrahydro-naphthalen-1-yl-amide, methoxy-acetic acid 6-tert-butyl-8-fluoro-2,3-dimethyl-quinolin-4-yl ester and *N*-Methyl-2-{1-[(5-methyl-3-trifluoromethyl-1H-pyrazol-1-yl)-acetyl]-piperidin-4-yl}-*N*-[(1R)-1,2,3,4-tetrahydronaphthalen-1-yl]-4-thiazolecarboxamide.
G) growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
H) herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron,tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethlyl, chlorthal, cinmethylin, clomazone, cumyluron, cyprosulfamide, dicamba, difenzoquat, diflufenzopyr, *Drechslera monoceras*, endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
I) insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, imidacloprid, thiamethoxam, nitenpyram, acetamiprid, thiacloprid, 1-(2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - others: benclothiaz, bifenazate, cartap, flonicamid, pyridalyl, pymetrozine, sulfur, thiocyclam, flubendiamide, chlorantraniliprole, cyazypyr (HGW86), cyenopyrafen, flupyrazofos, cyflumetofen, amidoflumet, imicyafos, bistrifluron, and pyrifluquinazon.

The present invention furthermore relates to agrochemical compositions comprising a mixture of at least one compound of the formula I used according to the invention and compounds according to the invention (component 1) and at least one further active substance useful for plant protection, e. g. selected from the groups A) to I) (component 2), in particular one further fungicide, e. g. one or more fungicide from the groups A) to F), as described above, and if desired one suitable solvent or solid carrier. Those mixtures are of particular interest, since many of them at the same application rate show higher efficiencies against harmful fungi. Furthermore, combating harmful fungi with a mixture of compounds of the formula I used according to the invention and compounds according to the invention and at least one fungicide from groups A) to F), as described above, is more efficient than combating those fungi with individual compounds of the formula I used according to the invention and compounds according to the invention or individual fungicides from groups A) to F). By applying compounds of the formula I used according to the invention and compounds according to the invention together with at least one active substance from groups A) to I) a synergistic effect can be obtained, i.e. more then simple addition of the individual effects is obtained (synergistic mixtures).

According to this invention, applying the compounds of the formula I used according to the invention and compounds according to the invention together with at least one further active substance is to be understood to denote, that at least one compound of formula I and at least one further active substance occur simultaneously at the site of action (i.e. the harmful fungi to be controlled or their habitats such as infected plants, plant propagation materials, particularly seeds, surfaces, materials or the soil as well as plants, plant propagation materials, particularly seeds, soil, surfaces, materials or rooms to be protected from fungal attack) in a fungicidally effective amount. This can be obtained by applying the compounds of the formula I used according to the invention and compounds according to the invention and at least one further active substance simultaneously, either jointly (e. g. as tank-mix) or sperately, or in succession, wherein the time interval between the individual applications is selected to ensure that the active substance applied first still occurs at the site of action in a sufficient amount at the time of application of the further active substance(s). The order of application is not essential for working of the present invention.

In binary mixtures, i.e. compositions according to the invention comprising one (component 1) and one further active substance (component 2), e. g. one active substance from groups A) to I), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:3 to 3:1.

In ternary mixtures, i.e. compositions according to the invention comprising one compound of the formula I used according to the invention and compounds according to the invention (component 1) and a first further active substance (component 2) and a second further active substance (component 3), e. g. two active substances from groups A) to I), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

The components can be used individually or already partially or completely mixed with one another to prepare the composition according to the invention. It is also possible for them to be packaged and used further as combination composition such as a kit of parts.

In one embodiment of the invention, the kits may include one or more, including all, components that may be used to prepare a subject agrochemical composition. E. g., kits may include one or more fungicide component(s) and/or an adjuvant component and/or a insecticide component and/or a growth regulator component and/or a herbicde. One or more of the components may already be combined together or pre-formulated. In those embodiments where more than two components are provided in a kit, the components may already be combined together and as such are packaged in a single container such as a vial, bottle, can, pouch, bag or canister. In other embodiments, two or more components of a kit may be packaged separately, i. e., not pre-formulated. As such, kits may include one or more separate containers such as vials, cans, bottles, pouches, bags or canisters, each container containing a separate component for an agrochemical composition. In both forms, a component of the kit may be applied separately from or together with the further components or as a component of a combination composition according to the invention for preparing the composition according to the invention.

The user applies the composition according to the invention usually from a predosage device, a knapsack sprayer, a spray tank or a spray plane. Here, the agrochemical composition is made up with water and/or buffer to the desired application concentration, it being possible, if appropriate, to add further auxiliaries, and the ready-to-use spray liquor or the agrochemical composition according to the invention is thus obtained. Usually, 50 to 500 liters of the ready-to-use spray liquor are applied per hectare of agricultural useful area, preferably 100 to 400 liters.

According to one embodiment, individual components of the composition according to the invention such as parts of a kit or parts of a binary or ternary mixture may be mixed by the user himself in a spray tank and further auxiliaries may be added, if appropriate (tank mix).

In a further embodiment, either individual components of the composition according to the invention or partially premixed components, e. g. components comprising compounds of the formula I used according to the invention and compounds according to the invention and/or active substances from the groups A) to I), may be mixed by the user in a spray tank and further auxiliaries and additives may be added, if appropriate (tank mix).

In a further embodiment, either individual components of the composition according to the invention or partially premixed components, e. g. components comprising compounds of the formula I used according to the invention and compounds according to the invention and/or active substances from the groups A) to I), can be applied jointly (e..g. after tankmix) or consecutively.

Preference is also given to mixtures comprising a compound of the formula I used according to the invention and compounds according to the invention (component 1) and at least one active substance selected from the strobilurines of group A) (component 2) and particularly selected from azoxystrobin, dimoxystrobin, fluoxastrobin, kresoxim-methyl, orysastrobin, picoxystrobin, pyraclostrobin and trifloxystrobin.

Preference is also given to mixtures comprising a compound of the formula I used according to the invention and compounds according to the invention (component 1) and at least one active substance selected from the carboxamides of group B) (component 2) and particularly selected from bixafen, boscalid, fluopyram, fluxapyroxad, isopyrazam, penflufen, penthiopyrad, sedaxane, metalaxyl, mefenoxam, ofurace, dimethomorph, flumorph, fluopicolid (picobenzamid), zoxamide, carpropamid and mandipropamid. Preference is given to mixtures comprising a compound of formula I (component 1) and at least one active substance selected from the azoles of group C) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, cyazofamid, benomyl, carbendazim and ethaboxam.

Preference is also given to mixtures comprising a compound of the formula I used according to the invention and compounds according to the invention (component 1) and at least one active substance selected from the heterocyclic compounds of group D) (component 2) and particularly selected from fluazinam, cyprodinil, fenarimol, mepanipyrim, pyrimethanil, triforine, fludioxonil, dodemorph, fenpropimorph, tridemorph, fenpropidin, iprodione, vinclozolin, famoxadone, fenamidone, probenazole, proquinazid, acibenzolar-S-methyl, captafol, folpet, fenoxanil, quinoxyfen and ametoctradin.

Preference is also given to mixtures comprising a compound of the formula I used according to the invention and compounds according to the invention (component 1) and at least one active substance selected from the carbamates of group E) (component 2) and particularly selected from mancozeb, metiram, propineb, thiram, iprovalicarb, benthiavalicarb and propamocarb.

Preference is also given to mixtures comprising a compound of the formula I used according to the invention and compounds according to the invention (component 1) and at least one active substance selected from the fungicides given in group F) (component 2) and particularly selected from dithianon, fentin salts, such as fentin acetate, fosetyl, fosetyl-aluminium, H₃PO₃ and salts thereof, chlorthalonil, dichlofluanid, thiophanat-methyl, copper acetate, copper hydroxide, copper oxychloride, copper sulfate, sulfur, cymoxanil, metrafenone, spiroxamine and *N*-methyl-2-{1-[(5-methyl-3-trifluoromethyl-1H-pyrazol-1-yl)-acetyl]-piperidin-4-yl}-*N*-[(1R)-1,2,3,4-tetrahydronaphthalen-1-yl]-4-thiazolecarboxamide.

Preference is also given to mixtures comprising a compound of the formula I used according to the invention and compounds according to the invention (component 1) and at least one active substance selected from the antifungal biocontrol agents given in group F) (component 2) and particularly selected from *Bacillus subtilis* strain NRRL No. B-*21661, Bacillus pumilus* strain NRRL No. B-30087 and *Ulocladium oudemansii*.

Accordingly, the present invention furthermore relates to compositions comprising one compound of the formula I used according to the invention and compounds according to the invention (component 1) and one further active substance (component 2), which further active substance is selected from the column "Component 2" of the lines B-1 to B-346 of Table B.

A further embodiment relates to the compositions B-1 to B-346 listed in Table B, where a row of Table B corresponds in each case to a fungicidal composition comprising one of the in the present specification individualized compounds of formula I (component 1) and the respective further active substance from groups A) to I) (component 2) stated in the row in question. Preferably, the compositions described comprise the active substances in synergistically effective amounts.

**Table B: Composition comprising one indiviualized compound of the formula I used according to the invention and compounds according to the invention and one further active substance from groups A) to I)**

| **Mixture** | **Component 1** | **Component 2** |
|---|---|---|
| B-1 | one individualized compound I | Azoxystrobin |
| B-2 | one individualized compound I | Coumethoxystrobin |
| B-3 | one individualized compound I | Coumoxystrobin |
| B-4 | one individualized compound I | Dimoxystrobin |
| B-5 | one individualized compound I | Enestroburin |
| B-6 | one individualized compound I | Fluoxastrobin |
| B-7 | one individualized compound I | Kresoxim-methyl |
| B-8 | one individualized compound I | Metominostrobin |
| B-9 | one individualized compound I | Orysastrobin |
| B-10 | one individualized compound I | Picoxystrobin |
| B-11 | one individualized compound I | Pyraclostrobin |
| B-12 | one individualized compound I | Pyrametostrobin |
| B-13 | one individualized compound I | Pyraoxystrobin |
| B-14 | one individualized compound I | Pyribencarb |
| B-15 | one individualized compound I | Trifloxystrobin |
| B-16 | one individualized compound of the formula I | 2-[2-(2,5-dimethyl-phenoxymethyl)-phenyl]-3-methoxy-acrylic acid methyl ester |
| B-17 | one individualized compound of the formula I | 2-(2-(3-(2,6-dichlorophenyl)-1-methyl-allylideneaminooxymethyl)-phenyl)-2-methoxyimino-N-methyl-acetamide |
| B-18 | one individualized compound of the formula I | Benalaxyl |
| B-19 | one individualized compound of the formula I | Benalaxyl-M |
| B-20 | one individualized compound I | Benodanil |
| B-21 | one individualized compound I | Bixafen |
| B-22 | one individualized compound I | Boscalid |
| B-23 | one individualized compound I | Carboxin |
| B-24 | one individualized compound I | Fenfuram |
| B-25 | one individualized compound I | Fenhexamid |
| B-26 | one individualized compound I | Flutolanil |
| B-27 | one individualized compound I | Fluxapyroxad |
| B-28 | one individualized compound I | Furametpyr |
| B-29 | one individualized compound I | Isopyrazam |
| B-30 | one individualized compound I | Isotianil |
| B-31 | one individualized compound I | Kiralaxyl |
| B-32 | one individualized compound I | Mepronil |
| B-33 | one individualized compound I | Metalaxyl |
| B-34 | one individualized compound I | Metalaxyl-M |
| B-35 | one individualized compound I | Ofurace |
| B-36 | one individualized compound I | Oxadixyl |
| B-37 | one individualized compound I | Oxycarboxin |
| B-38 | one individualized compound I | Penflufen |
| B-39 | one individualized compound I | Penthiopyrad |
| B-40 | one individualized compound I | Sedaxane |
| B-41 | one individualized compound I | Tecloftalam |
| B-42 | one individualized compound I | Thifluzamide |
| B-43 | one individualized compound I | Tiadinil |
| B-44 | one individualized compound I | 2-Amino-4-methyl-thiazole-5-carboxylic acid anilide |
| B-45 | one individualized compound I | N-(4'-trifluoromethylthiobiphenyl-2-yl)-3-difluoromethyl-1-methyl-1H-pyrazole-4-carboxamide |
| B-46 | one individualized compound I | N-(2-(1,3,3-trimethyl-butyl)-phenyl)-1,3-dimethyl-5-fluoro-1 H-pyrazole-4-carboxamide |
| B-47 | one individualized compound I | Dimethomorph |
| B-48 | one individualized compound I | Flumorph |
| B-49 | one individualized compound I | Pyrimorph |
| B-50 | one individualized compound I | Flumetover |
| B-51 | one individualized compound I | Fluopicolide |
| B-52 | one individualized compound I | Fluopyram |
| B-53 | one individualized compound I | Zoxamide |
| B-54 | one individualized compound I | Carpropamid |
| B-55 | one individualized compound I | Diclocymet |
| B-56 | one individualized compound I | Mandipropamid |
| B-57 | one individualized compound I | Oxytetracyclin |
| B-58 | one individualized compound I | Silthiofam |
| B-59 | one individualized compound I | N-(6-methoxy-pyridin-3-yl) cyclopropane-carboxylic acid amide |
| B-60 | one individualized compound I | Azaconazole |
| B-61 | one individualized compound I | Bitertanol |
| B-62 | one individualized compound I | Bromuconazole |
| B-63 | one individualized compound I | Cyproconazole |
| B-64 | one individualized compound I | Difenoconazole |
| B-65 | one individualized compound I | Diniconazole |
| B-66 | one individualized compound I | Diniconazole-M |
| B-67 | one individualized compound I | Epoxiconazole |
| B-68 | one individualized compound I | Fenbuconazole |
| B-69 | one individualized compound I | Fluquinconazole |
| B-70 | one individualized compound I | Flusilazole |
| B-71 | one individualized compound I | Flutriafol |
| B-72 | one individualized compound I | Hexaconazol |
| B-73 | one individualized compound I | Imibenconazole |
| B-74 | one individualized compound I | Ipconazole |
| B-75 | one individualized compound I | Metconazole |
| B-76 | one individualized compound I | Myclobutanil |
| B-77 | one individualized compound I | Oxpoconazol |
| B-78 | one individualized compound I | Paclobutrazol |
| B-79 | one individualized compound I | Penconazole |
| B-80 | one individualized compound I | Propiconazole |
| B-81 | one individualized compound I | Prothioconazole |
| B-82 | one individualized compound I | Simeconazole |
| B-83 | one individualized compound I | Tebuconazole |
| B-84 | one individualized compound I | Tetraconazole |
| B-85 | one individualized compound I | Triadimefon |
| B-86 | one individualized compound I | Triadimenol |
| B-87 | one individualized compound I | Triticonazole |
| B-88 | one individualized compound I | Uniconazole |
| B-89 | one individualized compound I | Cyazofamid |
| B-90 | one individualized compound I | Imazalil |
| B-91 | one individualized compound I | Imazalil-sulfate |
| B-92 | one individualized compound I | Pefurazoate |
| B-93 | one individualized compound I | Prochloraz |
| B-94 | one individualized compound I | Triflumizole |
| B-95 | one individualized compound I | Benomyl |
| B-96 | one individualized compound I | Carbendazim |
| B-97 | one individualized compound I | Fuberidazole |
| B-98 | one individualized compound I | Thiabendazole |
| B-99 | one individualized compound I | Ethaboxam |
| B-100 | one individualized compound I | Etridiazole |
| B-101 | one individualized compound I | Hymexazole |
| B-102 | one individualized compound I | 2-(4-Chloro-phenyl)-N-[4-(3,4-dimethoxyphenyl)-isoxazol-5-yl]-2-prop-2-ynyloxy-acetamide |
| B-103 | one individualized compound I | Fluazinam |
| B-104 | one individualized compound I | Pyrifenox |
| B-105 | one individualized compound I | 3-[5-(4-Chloro-phenyl)-2,3-dimethyl-isoxazolidin-3-yl]-pyridine |
| B-106 | one individualized compound I | 3-[5-(4-Methyl-phenyl)-2,3-dimethyl-isoxazolidin-3-yl]-pyridine |
| B-107 | one individualized compound I | Bupirimate |
| B-108 | one individualized compound I | Cyprodinil |
| B-109 | one individualized compound I | Diflumetorim |
| B-110 | one individualized compound I | Fenarimol |
| B-111 | one individualized compound I | Ferimzone |
| B-112 | one individualized compound I | Mepanipyrim |
| B-113 | one individualized compound I | Nitrapyrin |
| B-114 | one individualized compound I | Nuarimol |
| B-115 | one individualized compound I | Pyrimethanil |
| B-116 | one individualized compound I | Triforine |
| B-117 | one individualized compound I | Fenpiclonil |
| B-118 | one individualized compound I | Fludioxonil |
| B-119 | one individualized compound I | Aldimorph |
| B-120 | one individualized compound I | Dodemorph |
| B-121 | one individualized compound I | Dodemorph-acetate |
| B-122 | one individualized compound I | Fenpropimorph |
| B-123 | one individualized compound I | Tridemorph |
| B-124 | one individualized compound I | Fenpropidin |
| B-125 | one individualized compound I | Fluoroimid |
| B-126 | one individualized compound I | Iprodione |
| B-127 | one individualized compound I | Procymidone |
| B-128 | one individualized compound I | Vinclozolin |
| B-129 | one individualized compound I | Famoxadone |
| B-130 | one individualized compound I | Fenamidone |
| B-131 | one individualized compound I | Flutianil |
| B-132 | one individualized compound I | Octhilinone |
| B-133 | one individualized compound I | Probenazole |
| B-134 | one individualized compound I | 5-Amino-2-iso-propyl-4-ortho-tolyl-2,3-di-hydro-pyrazole-1-carbothioic acid S-allyl ester |
| B-135 | one individualized compound I | Acibenzolar-S-methyl |
| B-136 | one individualized compound I | Ametoctradin |
| B-137 | one individualized compound I | Amisulbrom |
| B-138 | one individualized compound I | Anilazin |
| B-139 | one individualized compound I | Blasticidin-S |
| B-140 | one individualized compound I | Captafol |
| B-141 | one individualized compound I | Captan |
| B-142 | one individualized compound I | Chinomethionat |
| B-143 | one individualized compound I | Dazomet |
| B-144 | one individualized compound I | Debacarb |
| B-145 | one individualized compound I | Diclomezine |
| B-146 | one individualized compound I | Difenzoquat, |
| B-147 | one individualized compound I | Difenzoquat-methylsulfate |
| B-148 | one individualized compound I | Fenoxanil |
| B-149 | one individualized compound I | Folpet |
| B-150 | one individualized compound I | Oxolinsäure |
| B-151 | one individualized compound I | Piperalin |
| B-152 | one individualized compound I | Proquinazid |
| B-153 | one individualized compound I | Pyroquilon |
| B-154 | one individualized compound I | Quinoxyfen |
| B-155 | one individualized compound I | Triazoxid |
| B-156 | one individualized compound I | Tricyclazole |
| B-157 | one individualized compound I | 2-Butoxy-6-iodo-3-propyl-chromen-4-one |
| B-158 | one individualized compound I | 5-Chloro-1-(4,6-dimethoxy-pyrimidin-2-yl)-2-methyl-1 H-benzoimidazole |
| B-159 | one individualized compound I | 5-Chloro-7-(4-methyl-pi perid in-1-yl)-6-(2,4,6-trifluoro-phenyl)-[1,2,4]tri-azolo[1,5-a]pyrimidine |
| B-160 | one individualized compound I | Ferbam |
| B-161 | one individualized compound I | Mancozeb |
| B-162 | one individualized compound I | Maneb |
| B-163 | one individualized compound I | Metam |
| B-164 | one individualized compound I | Methasulphocarb |
| B-165 | one individualized compound I | Metiram |
| B-166 | one individualized compound I | Propineb |
| B-167 | one individualized compound I | Thiram |
| B-168 | one individualized compound I | Zineb |
| B-169 | one individualized compound I | Ziram |
| B-170 | one individualized compound I | Diethofencarb |
| B-171 | one individualized compound I | Benthiavalicarb |
| B-172 | one individualized compound I | Iprovalicarb |
| B-173 | one individualized compound I | Propamocarb |
| B-174 | one individualized compound I | Propamocarb hydrochlorid |
| B-175 | one individualized compound I | Valifenalate |
| B-176 | one individualized compound I | N-(1-(1-(4-cyanophenyl)ethanesulfonyl)-but-2-yl) carbamic acid-(4-fluorophenyl) ester |
| B-177 | one individualized compound I | Dodine |
| B-178 | one individualized compound I | Dodine free base |
| B-179 | one individualized compound I | Guazatine |
| B-180 | one individualized compound I | Guazatine-acetate |
| B-181 | one individualized compound I | Iminoctadine |
| B-182 | one individualized compound I | Iminoctadine-triacetate |
| B-183 | one individualized compound I | Iminoctadine-tris(albesilate) |
| B-184 | one individualized compound I | Kasugamycin |
| B-185 | one individualized compound I | Kasugamycin-hydrochloride-hydrate |
| B-186 | one individualized compound I | Polyoxine |
| B-187 | one individualized compound I | Streptomycin |
| B-188 | one individualized compound I | Validamycin A |
| B-189 | one individualized compound I | Binapacryl |
| B-190 | one individualized compound I | Dicloran |
| B-191 | one individualized compound I | Dinobuton |
| B-192 | one individualized compound I | Dinocap |
| B-193 | one individualized compound I | Nitrothal-isopropyl |
| B-194 | one individualized compound I | Tecnazen |
| B-195 | one individualized compound I | Fentin salts |
| B-196 | one individualized compound I | Dithianon |
| B-197 | one individualized compound I | Isoprothiolane |
| B-198 | one individualized compound I | Edifenphos |
| B-199 | one individualized compound I | Fosetyl, Fosetyl-aluminium |
| B-200 | one individualized compound I | Iprobenfos |
| B-201 | one individualized compound I | Phosphorous acid (H₃PO₃) and derivatives |
| B-202 | one individualized compound I | Pyrazophos |
| B-203 | one individualized compound I | Tolclofos-methyl |
| B-204 | one individualized compound I | Chlorothalonil |
| B-205 | one individualized compound I | Dichlofluanid |
| B-206 | one individualized compound I | Dichlorophen |
| B-207 | one individualized compound I | Flusulfamide |
| B-208 | one individualized compound I | Hexachlorbenzene |
| B-209 | one individualized compound I | Pencycuron |
| B-210 | one individualized compound I | Pentachlorophenol and salts |
| B-211 | one individualized compound I | Phthalide |
| B-212 | one individualized compound I | Quintozene |
| B-213 | one individualized compound I | Thiophanate Methyl |
| B-214 | one individualized compound I | Tolylfluanid |
| B-215 | one individualized compound I | N-(4-chloro-2-nitro-phenyl)-N-ethyl-4-methyl-benzenesulfonamide |
| B-216 | one individualized compound I | Bordeau⁶ mixture |
| B-217 | one individualized compound I | Copper acetate |
| B-218 | one individualized compound I | Copper hydroxide |
| B-219 | one individualized compound I | Copper oxychloride |
| B-220 | one individualized compound I | basic Copper sulfate |
| B-221 | one individualized compound I | Sulfur |
| B-222 | one individualized compound I | Biphenyl |
| B-223 | one individualized compound I | Bronopol |
| B-224 | one individualized compound I | Cyflufenamid |
| B-225 | one individualized compound I | Cymoxanil |
| B-226 | one individualized compound I | Diphenylamin |
| B-227 | one individualized compound I | Metrafenone |
| B-228 | one individualized compound I | Pyriofenone |
| B-229 | one individualized compound I | Mildiomycin |
| B-230 | one individualized compound I | Oxin-copper |
| B-231 | one individualized compound I | Prohexadione calcium |
| B-232 | one individualized compound I | Spiroxamine |
| B-233 | one individualized compound I | Tebufloquin |
| B-234 | one individualized compound I | Tolylfluanid |
| B-235 | one individualized compound I | N-(Cyclopropylmethoxyimino-(6-difluoro-methoxy-2,3-difluoro-phenyl)-methyl)-2-phenyl acetamide |
| B-236 | one individualized compound I | N'-(4-(4-chloro-3-trifluoromethyl-phenoxy)-2,5-dimethyl-phenyl)-N-ethyl-N methyl formamidine |
| B-237 | one individualized compound I | N'-(4-(4-fluoro-3-trifluoromethyl-phenoxy) 2,5-dimethyl-phenyl)-N-ethyl-N-methyl formamidine |
| B-238 | one individualized compound I | N'-(2-methyl-5-trifluoromethyl-4-(3-tri-methylsilanyl-propoxy)-phenyl)-N-ethyl-N methyl formamidine |
| B-239 | one individualized compound I | N'-(5-d ifl uoromethyl-2-methyl-4-(3-tri-methylsilanyl-propoxy)-phenyl)-N-ethyl-N methyl formamidine |
| B-240 | one individualized compound I | 2-{1-[2-(5-Methyl-3-trifluoromethyl-pyrazole-1-yl)-acetyl]-piperidin-4-yl}-thiazole-4-carboxylic acid methyl-(1,2,3,4-tetrahydro-naphthalen-1-yl)-amide |
| B-241 | one individualized compound I | 2-{1-[2-(5-Methyl-3-trifluoromethyl-pyrazole-1-yl)-acetyl]-piperidin-4-yl}-thiazole-4-carboxylic acid methyl-(R)-1,2,3,4-tetrahydro-naphthalen-1-yl-amide |
| B-242 | one individualized compound I | Methoxy-acetic acid 6-tert-butyl-8-fluoro-2,3-dimethyl-quinolin-4-yl ester |
| B-243 | one individualized compound I | *N*-Methyl-2-{1-[(5-methyl-3-trifluoro-methyl-1 H-pyrazol-1-yl)-acetyl]-piperidin-4-yl}-*N*-[(1R)-1,2,3,4-tetrahydro-naphthalen-1-yl]-4-thiazolecarboxamide |
| B-244 | one individualized compound I | *Bacillus subtilis* NRRL No. B-21661 |
| B-245 | one individualized compound I | *Bacillus pumilus* NRRL No. B-30087 |
| B-246 | one individualized compound I | *Ulocladium oudemansii* |
| B-247 | one individualized compound I | Carbaryl |
| B-248 | one individualized compound I | Carbofuran |
| B-249 | one individualized compound I | Carbosulfan |
| B-250 | one individualized compound I | Methomylthiodicarb |
| B-251 | one individualized compound I | Bifenthrin |
| B-252 | one individualized compound I | Cyfluthrin |
| B-253 | one individualized compound I | Cypermethrin |
| B-254 | one individualized compound I | alpha-Cypermethrin |
| B-255 | one individualized compound I | zeta-Cypermethrin |
| B-256 | one individualized compound I | Deltamethrin |
| B-257 | one individualized compound I | Esfenvalerate |
| B-258 | one individualized compound I | Lambda-cyhalothrin |
| B-259 | one individualized compound I | Permethrin |
| B-260 | one individualized compound I | Tefluthrin |
| B-261 | one individualized compound I | Diflubenzuron |
| B-262 | one individualized compound I | Flufenoxuron |
| B-263 | one individualized compound I | Lufenuron |
| B-264 | one individualized compound I | Teflubenzuron |
| B-265 | one individualized compound I | Spirotetramate |
| B-266 | one individualized compound I | Clothianidin |
| B-267 | one individualized compound I | Dinotefuran |
| B-268 | one individualized compound I | Imidacloprid |
| B-269 | one individualized compound I | Thiamethoxam |
| B-270 | one individualized compound I | Acetamiprid |
| B-271 | one individualized compound I | Thiacloprid |
| B-272 | one individualized compound I | Endosulfan |
| B-273 | one individualized compound I | Fipronil |
| B-274 | one individualized compound I | Abamectin |
| B-275 | one individualized compound I | Emamectin |
| B-276 | one individualized compound I | Spinosad |
| B-277 | one individualized compound I | Spinetoram |
| B-278 | one individualized compound I | Hydramethylnon |
| B-279 | one individualized compound I | Chlorfenapyr |
| B-280 | one individualized compound I | Fenbutatin oxide |
| B-281 | one individualized compound I | Indoxacarb |
| B-282 | one individualized compound I | Metaflumizone |
| B-283 | one individualized compound I | Flonicamid |
| B-284 | one individualized compound I | Lubendiamide |
| B-285 | one individualized compound I | Chlorantraniliprole |
| B-286 | one individualized compound I | Cyazypyr (HGW86) |
| B-287 | one individualized compound I | Cyflumetofen |
| B-288 | one individualized compound I | Acetochlor |
| B-289 | one individualized compound I | Dimethenamid |
| B-290 | one individualized compound I | metolachlor |
| B-291 | one individualized compound I | Metazachlor |
| B-292 | one individualized compound I | Glyphosate |
| B-293 | one individualized compound I | Glufosinate |
| B-294 | one individualized compound I | Sulfosate |
| B-295 | one individualized compound I | Clodinafop |
| B-296 | one individualized compound I | Fenoxaprop |
| B-297 | one individualized compound I | Fluazifop |
| B-298 | one individualized compound I | Haloxyfop |
| B-299 | one individualized compound I | Paraquat |
| B-300 | one individualized compound I | Phenmedipham |
| B-301 | one individualized compound I | Clethodim |
| B-302 | one individualized compound I | Cycloxydim |
| B-303 | one individualized compound I | Profoxydim |
| B-304 | one individualized compound I | Sethoxydim |
| B-305 | one individualized compound I | Tepraloxydim |
| B-306 | one individualized compound I | Pendimethalin |
| B-307 | one individualized compound I | Prodiamine |
| B-308 | one individualized compound I | Trifluralin |
| B-309 | one individualized compound I | Acifluorfen |
| B-310 | one individualized compound I | Bromoxynil |
| B-311 | one individualized compound I | Imazamethabenz |
| B-312 | one individualized compound I | Imazamox |
| B-313 | one individualized compound I | Imazapic |
| B-314 | one individualized compound I | Imazapyr |
| B-315 | one individualized compound I | Imazaquin |
| B-316 | one individualized compound I | Imazethapyr |
| B-317 | one individualized compound I | 2,4-Dichlorophenoxyacetic acid (2,4-D) |
| B-318 | one individualized compound I | Chloridazon |
| B-319 | one individualized compound I | Clopyralid |
| B-320 | one individualized compound I | Fluroxypyr |
| B-321 | one individualized compound I | Picloram |
| B-322 | one individualized compound I | Picolinafen |
| B-323 | one individualized compound I | Bensulfuron |
| B-324 | one individualized compound I | Chlorimuron-ethyl |
| B-325 | one individualized compound I | Cyclosulfamuron |
| B-326 | one individualized compound I | lodosulfuron |
| B-327 | one individualized compound I | Mesosulfuron |
| B-328 | one individualized compound I | Metsulfuron-methyl |
| B-329 | one individualized compound I | Nicosulfuron |
| B-330 | one individualized compound I | Rimsulfuron |
| B-331 | one individualized compound I | Triflusulfuron |
| B-332 | one individualized compound I | Atrazine |
| B-333 | one individualized compound I | Hexazinone |
| B-334 | one individualized compound I | Diuron |
| B-335 | one individualized compound I | Florasulam |
| B-336 | one individualized compound I | Pyroxasulfone |
| B-337 | one individualized compound I | Bentazone |
| B-338 | one individualized compound I | Cinidon-ethlyl |
| B-339 | one individualized compound I | Cinmethylin |
| B-340 | one individualized compound I | Dicamba |
| B-341 | one individualized compound I | Diflufenzopyr |
| B-342 | one individualized compound I | Quinclorac |
| B-343 | one individualized compound I | Quinmerac |
| B-344 | one individualized compound I | Mesotrione |
| B-345 | one individualized compound I | Saflufenacil |
| B-346 | one individualized compound I | Topramezone |

The active substances referred to as component 2, their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624).

The mixtures of active substances can be prepared as compositions comprising besides the active ingridients at least one inert ingredient by usual means, e. g. by the means given for the compositions of compounds of the formula I used according to the invention and compounds according to the invention.

Concerning usual ingredients of such compositions reference is made to the explanations given for the compositions containing compounds of the formula I used according to the invention and compounds according to the invention.

The mixtures of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes ). In addition, it is refered to the explanations regarding the fungicidal activity of the compounds and the compositions containing compounds of the formula I used according to the invention and compounds according to the invention , respectively.

## Claims

1. Use of pyrazolopyridine compounds of the formula I 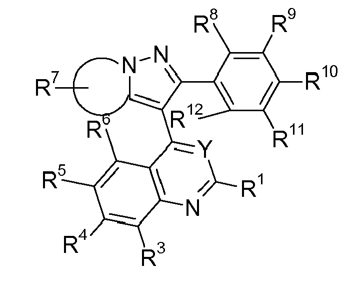 in which 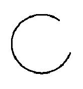
is C₅-C₆-cycloalkyl;
Y is N or C-X,
R¹ to R¹², X independently of one another are
H, halogen, cyano, nitro, N₃; or
C₁-C₁₀-alkyl, C₁-C₁₀-haloalkyl, C₂-C₁₀-alkenyl, C₂-C₁₀-alkynyl, C₃-C₁₀₋cycloalkyl, C₃-C₁₀-halocycloalkyl, C₃-C₁₀-cycloalkenyl; or
phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or
NA¹A² where
A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl, wherein
the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members; or
independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or
OA³ where
A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or
a radical of the formula -C(=O)A⁴, -OC(=O)A⁴, -NA⁴C(=O)A⁴, -N=OA⁴ where
A⁴ is hydrogen, C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy; or
a radical of the formula -S(O)ₙA⁵, - OS(O)ₙA⁵, -NA⁵S(O)ₙA⁵ where
n is 0, 1,2
A⁵ is C₁-C₁₀-alkyl, C₁-C₁₀-haloalkyl, C₃-C₁₀-cycloalkyl, C₃-C₁₀-halocycloalkyl, NH₂, mono-(C₁-C₁₀-alkyl)amino, di-(C₁-C₁₀-alkyl)amino, phenyl, benzyl, naphthyl or a saturated, partially unsaturated or aromatic 5-, 6-, 7-, 8-, 9- or 10- membered heterocycle, where the heterocycle contains 1, 2, 3 or 4 heteroatoms selected from the group consisting of O, N and S as ring members and may furthermore contain one or two CO, SO or SO₂ groups as ring members, wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy;
and/or of an agriculturally acceptable salt thereof for controlling phytopathogenic fungi.

2. The use according to claim 1 where
R¹ is H.

3. The use according to claim 1 where
R¹ is phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members,
wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

4. The use according to claim 1 where
R¹ is NA¹A² where
A¹ and A² independently of one another are hydrogen, C₁-C₁₀-alkyl, C₁-C₁₀-haloalkyl, C₃-C₁₀-cycloalkyl, C₃-C₁₀-halocycloalkyl, C₁-C₁₀-alkoxy-C₁-C₁₀-alkyl, amino-C₁-C₁₀-alkyl,
wherein
the amino group is substituted by B¹ and B² which are independently of one another hydrogen, C₁-C₁₀-alkyl, C₁-C₁₀-haloalkyl, C₃-C₁₀-cycloalkyl, C₃-C₁₀-halocycloalkyl or B¹ and B² together with the N atom to which these radicals are attached may also form a five-, six-, seven-, eight-, nine- or ten-membered saturated or partially unsaturated ring which, in addition to carbon atoms, may contain one, two or three heteroatoms from the group consisting of O, N and S as ring members;
or
independently of one another are phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members,
wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

5. The use according to claim 1 where
R¹ is OA³ where
A³ is hydrogen, C₁-C₁₀-alkyl, C₁-C₁₀-haloalkyl, C₃-C₁₀-cycloalkyl, C₃-C₁₀-halocycloalkyl, phenyl, benzyl, naphthyl or a saturated, partially unsaturated or aromatic 5-, 6-, 7-, 8-, 9- or 10-membered heterocycle, where the heterocycle contains 1, 2, 3 or 4 heteroatoms selected from the group consisting of O, N and S as ring members and may furthermore contain one or two CO, SO or SO₂ groups as ring members,
wherein
the above mentioned groups may carry one, two, three or four identical or different substituents selected from the group consisting of halogen, hydroxyl, cyano, nitro, NH₂, C₁-C₁₀-alkyl, C₁-C₁₀-haloalkyl, C₁-C₁₀-alkoxy, C₁-C₁₀-haloalkoxy.

6. The use according to claim 1 where
R³ to R⁷ independently of one another are
H, halogen, cyano, nitro, N₃; or
C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalkyl, C₃-C₁₀-cycloalkenyl.

7. The use according to claim 1 where
R⁸ to R¹² independently of one another are
H, halogen, cyano, nitro, N₃; or
C₁-C₁₀-alkyl, C₁-C₁₀-haloalkyl, C₂-C₁₀-alkenyl, C₂-C₁₀-alkynyl, C₃-C₁₀-cycloalkyl, C₃-C₁₀-halocycloalkyl, C₃-C₁₀-cycloalkenyl.

8. A pyrazolopyridine compound of the formula I according to the definition in claim 1, with the proviso, that if 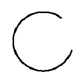
is C₅-cycloalkyl;
R⁸ to R¹² can not be simultaneously H; and
R⁸ is not F, methoxy;
R⁹ is not Cl, F, C₁-haloalkyl, OA³ where A³ is CH₃; C₁-haloalkoxy;
R¹⁰ is not Cl, F, C₁-haloalkyl;
R¹¹ is not Cl, F, C₁-haloalkyl, OA³ where A³ is CH₃; C₁-haloalkoxy; and
R¹² is not F, methoxy.

9. A pyrazolopyridine compound of the formula I according to the definition in claim 1, with the proviso, that if 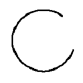
is C₆-cycloalkyl;
R⁸ to R¹² can not be simultaneously H; and
R⁸ is not F;
R⁹ is not Cl, F, C₁-haloalkyl, C₁-haloalkoxy;
R¹⁰ is not Cl, F, C₁-haloalkyl;
R¹¹ is not Cl, F, C₁-haloalkyl, C₁-haloalkoxy; and
R¹² is not F.

10. A composition for controlling phytopathogenic fungi comprising at least one compound of the formula I according to any of claims 1 to 7 and 8 to 9 and/or an salt thereof and at least one solid or liquid carrier.

11. The composition according to claim 10 furthermore comprising at least one further fungicidally, insecticidally and/or herbicidally active compound.

12. Seed, comprising at least one compound of the formula I according to any of claims 1 to 7 and 8 to 9 and/or an agriculturally acceptable salt thereof in an amount of from 1 to 1000 g per 100 kg.

13. A method for controlling phytopathogenic fungi wherein the fungi or the materials, plants, the soil or seed to be protected from fungal attack are treated with an effective amount of a compound of the formula I according to any of claims 1 to 7 and 8 to 9 and/or an agriculturally acceptable salt thereof.
